# EUROPEAN PATENT APPLICATION

(11) **EP 3 561 029 A1**
(43) Date of publication of application: **30.10.2019**
(21) Application number: 18169757.4
(22) Date of filing: 27.04.2018
(51) Int. Cl.: C11B 9/00, C07C 69/02, C07C 43/04

(54) **ESTERS AND ETHERS OF 3-METHYL-PENTAN-DIOL AND UNSATURATED DERIVATES THEREOF AND THEIR USE AS AROMA CHEMICAL**

(71) Applicant: BASF SE, 67056 Ludwigshafen am Rhein (DE)
(72) Inventor: Bru Roig, Miriam, 67056 Ludwigshafen (DE); Danz, Manuel, 67056 Ludwigshafen (DE); Pelzer, Ralf, 68623 Lampertheim (DE); Garlichs, Florian, 68623 Lampertheim (DE)
(74) Representative: Reitstötter Kinzebach

(57) **Abstract**

The present invention relates to the use of esters of 3-methyl-pentan-diol and unsaturated derivates thereof and ethers of 3-methyl-pentan-diol and unsaturated derivates thereof as aroma chemicals, to aroma chemical compositions comprising at least one ester of 3-methyl-pentan-diol and unsaturated derivates thereof and/or at least one ether of 3-methyl-pentan-diol and unsaturated derivates thereof and to a method for preparing a fragranced composition in particular a fragranced ready-to-use composition, which comprises incorporating at least one ester of 3-methyl-pentan-diol and unsaturated derivates thereof and/or at least one ether 3-methyl-pentan-diol and unsaturated derivates thereof into a composition, in particular into a ready-to-use composition. The present invention further relates to specific ethers 3-methyl-pentan-diol and unsaturated derivates thereof and specific esters 3-methyl-pentan-diol and unsaturated derivates thereof and a method for their preparation.

## Description

The present invention relates to the use of esters of 3-methyl-pentan-diol and unsaturated derivates thereof and ethers of 3-methyl-pentan-diol and unsaturated derivates thereof as aroma chemicals, to aroma chemical compositions comprising at least one ester of 3-methyl-pentan-diol and unsaturated derivates thereof and/or at least one ether of 3-methyl-pentan-diol and unsaturated derivates thereof and to a method for preparing a fragranced composition in particular a fragranced ready-to-use composition, which comprises incorporating at least one ester of 3-methyl-pentan-diol and unsaturated derivates thereof and/or at least one ether 3-methyl-pentan-diol and unsaturated derivates thereof into a composition, in particular into a ready-to-use composition. The present invention further relates to specific ethers 3-methyl-pentan-diol and unsaturated derivates thereof and specific esters 3-methyl-pentan-diol and unsaturated derivates thereof and a method for their preparation.

### BACKGROUND OF THE INVENTION

Aroma chemicals, especially fragrances, are of great interest especially in the field of cosmetics and cleaning and laundry compositions. Most fragrances of natural origin are expensive, often limited in their available amount and, on account of fluctuations in environmental conditions, are also subject to variations in their content, purity etc. To circumvent these undesirable factors, it is therefore of great interest, to create synthetic substances, which have organoleptic properties that resemble those of more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should, however, also have additional positive secondary properties, such as e.g. an efficient preparation method, the possibility of providing better sensory profiles as a result of synergistic effects with other fragrances, a higher stability under certain application conditions, a higher extendability, a better staying power, etc.

However, since even small changes in chemical structure bring about massive changes in the sensory properties such as odor and also taste, the targeted search for substances with certain sensory properties such as a certain odor is extremely difficult.

The search for new fragrances and flavorings is therefore in most cases difficult and laborious and it is uncertain whether a substance with the desired odor and/or taste will even actually be found.

EP 0908455 describes an odorant compositions which contains macrocycles, such as 15- to 17-memered compounds and processes for manufacturing the same. In particular 3-methyl-1,5-pentandiol diacetate [CAS No. 40065-27-8] is used as recatant for the preaprartion of 3-methyl-5-oxopentanol acetate. However, 3-methyl-1,5-pentandiol diacetate is not mentioned as aroma chemical.

The following compounds are known:
- compound (5-acetoxy-3-methylene-pentyl) acetate (CAS No 40760-37-0),
- compound [(E)-5-acetoxy-3-methyl-pent-3-enyl] acetate (CAS No 1262763-96-1),
- compound [(Z)-5-acetoxy-3-methyl-pent-3-enyl] acetate (CAS No 1262764-00-0),
- compound (5-acetoxy-3-methyl-pentyl) acetate (CAS No. 40065-27-8, EP 0908455),
- compound [(E)-5-formyloxy-3-methyl-pent-3-enyl] formate (CAS No. 53799-56-7),
- compound [(Z)-5-formyloxy-3-methyl-pent-3-enyl] formate (CAS No. 53799-55-6),
- compound (3-methyl-5-propanoyloxy-pentyl) propanoate (CAS No. 1438983-26-6),
- compound [5-(2,2-dimethylpropanoyloxy)-3-methyl-pentyl] 2,2-dimethylpropanoate (CAS No. 762268-77-9),
- compound 1,5-dimethoxy-3-methylene-pentane (CAS No. 79437-43-7),
- compound (E)-1,5-dimethoxy-3-methyl-pent-2-ene (CAS No. 123351-71-3),
- compound (Z)-1,5-dimethoxy-3-methyl-pent-2-ene (CAS No. 123351-70-2),
- compound 1,5-dimethoxy-3-methyl-pentane (CAS No. 4457-73-2),
- compound (Z)-1,5-diethoxy-3-methyl-pent-2-ene (CAS No. 1086266-95-6),
- compound 1,5-diethoxy-3-methyl-pentane (CAS No. 99868-13-0),
- compound (E)-1,5-diisopropoxy-3-methyl-pent-2-ene (CAS No. 116097-01-9),
- compound (Z)-1,5-diisopropoxy-3-methyl-pent-2-ene (CAS No. 116114-80-8),
- compound 1,5-dibutoxy-3-methylene-pentane (CAS No. 79437-42-6),
- compound (E)-1,5-dibutoxy-3-methyl-pent-2-ene (CAS No. 121997-62-4),
- compound (Z)-1,5-dibutoxy-3-methyl-pent-2-en (CAS No. 121997-61-3).

The following mixtures are known:
- mixture [(E)-5-acetoxy-3-methyl-pent-3-enyl] acetate and [(Z)-5-acetoxy-3-methyl-pent-3-enyl] acetate (CAS No. 18273-38-6),
- mixture of [(E)-3-methyl-5-propanoyloxy-pent-3-enyl] propanoate and [(Z)-3-methyl-5-propanoyloxy-pent-3-enyl] propanoate (CAS No. 38169-71-0),
- mixtures of (E)-1,5-dimethoxy-3-methyl-pent-2-ene and (Z)-1,5-dimethoxy-3-methyl-pent-2-ene (CAS No. 54284-82-1),
- mixtures of (Z)-1,5-diethoxy-3-methyl-pent-2-ene and (E)-1,5-diethoxy-3-methyl-pent-2-ene (CAS No. 42173-34-2),
- mixtures of (E)-3-methyl-1,5-dipropoxy-pent-2-ene and (Z)-3-methyl-1,5-dipropoxy-pent-2-ene (CAS No. 54284-83-2),
- mixture of (E)-1,5-dibutoxy-3-methyl-pent-2-ene and (Z)-1,5-dibutoxy-3-methyl-pent-2-en (CAS No 79437-47-1).

The use of esters and ethers derived from 3-methyl-pentan-diol and unsaturated derivates thereof as aroma chemicals has so far not been described in the prior art.

### SUMMARY OF THE INVENTION

It is an object of the present invention to provide substances exhibiting pleasant organoleptical properties, which can be advantageously used as aroma chemicals. It is a further object of the present invention to provide substances, which can be used as an aroma chemical in ready-to-use compositions. In particular, odor-intensive substances having a pleasant odor are sought. Furthermore, these aroma chemicals should be combinable with other aroma chemicals, allowing the creation of novel advantageous sensory profiles. In addition, these aroma chemicals should be obtainable from redily available starting materials, allowing their fast and economic manifacturing, and should not raise of toxicological concerns.

It was surprisingly found that these and further objects are achieved by esters and ethers derived from 3-methyl-pentan-diol and unsaturated derivatives thereof.

Accordingly, a first aspect of the present invention relates to the use of a compound of the general formula (I) 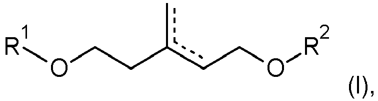 wherein
one of the symbols 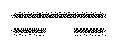 is a single bond and the other is a double bond or a single bond,
R¹ is hydrogen, C₁-C₄-alkyl or -(C=O)-R³,
R² is hydrogen, C₁-C₄-alkyl or -(C=O)-R⁴,
R³ is hydrogen or C₁-C₄-alkyl,
R⁴ is hydrogen or C₁-C₄-alkyl,
a stereoisomer thereof or a mixture of stereoisomers thereof, as an aroma chemical, with the proviso that if R¹ is hydrogen, R² is different from hydrogen.

In Particular, the present invention relates to the use of a compound of the general formula (I.a) or (I.b) 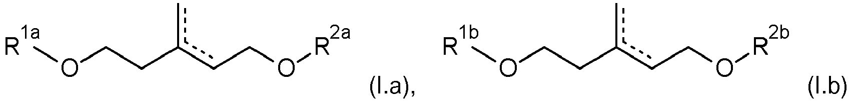 wherein
one of the symbols 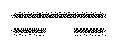 is a single bond and the other is a double bond or a single bond,
R^{1a} is hydrogen, C₁-C₄-alkyl or -(C=O)-R³,
R^{2a} is hydrogen or C₁-C₄-alkyl,
R^{1b} is hydrogen or -(C=O)-R³,
R^{2a} is hydrogen or -(C=O)-R⁴,
R³ is hydrogen, C₁-C₄-alkyl,
R⁴ is hydrogen or C₁-C₄-alkyl,
a stereoisomer thereof, or a mixture of stereoisomers thereof, as an aroma chemical, with the proviso that if R^{1a} or is R^{1b} hydrogen, R^{2a} or R^{2b} is different from hydrogen.

The present invention further relates to an aroma chemical compositions comprising at least one compound of formula (I), (I.a) or (I.b), a stereoisomer thereof or a mixture of stereoisomers thereof, as defined above, and at least one further compound selected from the group consisting of aroma chemicals different from compounds (I), (I.a) or (I.b) and non-aroma chemical carriers.

It was further found that the compounds of the general formula (I), (I.a) or (I.b) generally exhibit a pleasant and characteristic odor and can be used to produce fragranced ready-to-use compositions. In addition, they can advantageously be combined with other aroma chemicals different from compounds (I), (I.a) or (I.b) to create new scent profiles.

Therefore, the present invention further relates to a method of preparing a fragranced composition, in partivular a fragranced ready-to-use composition, comprising incorporating at least one compound of formula (I), (I.a) or (I.b), a stereoisomer thereof or a mixture of stereoisomers thereof, into a composition, in particular into a ready-to-use composition, and to the use of a compound of formula (I) (I.a) or (I.b), a stereoisomer thereof or a mixture of stereoisomers thereof, as defined above, for modifying the scent character of a fragranced ready-to-use composition, in particular a fragranced ready-to-use composition.

Amongst the group of compounds of formula (I), the ether compounds of formula (I.a) or the ester compounds of (I.b) some of the ether compounds and the ester compounds of formula (I) have not been described in the art.

Therefore, the present invention also relates to novel compounds of the general formula (I) 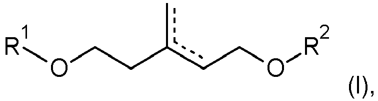 wherein
one of the symbols 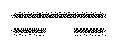 is a single bond and the other is a double bond or a single bond,
R¹ is hydrogen, C₁-C₄-alkyl or -(C=O)-R³,
R² is hydrogen, C₁-C₄-alkyl or -(C=O)-R⁴,
R³ is hydrogen, C₁-C₄-alkyl,
R⁵ is hydrogen, C₁-C₄-alkyl,
a stereoisomer thereof or a mixture of stereoisomers thereof, with the proviso that if R¹ is hydrogen R² is different from hydrogen,
except for the following compounds
   - (5-acetoxy-3-methylene-pentyl) acetate,
   - [(E)-5-acetoxy-3-methyl-pent-3-enyl] acetate,
   - [(Z)-5-acetoxy-3-methyl-pent-3-enyl] acetate,
   - (5-acetoxy-3-methyl-pentyl) acetate,
   - [(E)-5-formyloxy-3-methyl-pent-3-enyl] formate,
   - [(Z)-5-formyloxy-3-methyl-pent-3-enyl] formate,
   - (3-methyl-5-propanoyloxy-pentyl) propanoate,
   - [5-(2,2-dimethylpropanoyloxy)-3-methyl-pentyl] 2,2-dimethylpropanoate,
   - 1,5-dimethoxy-3-methylene-pentane,
   - (E)-1,5-dimethoxy-3-methyl-pent-2-ene,
   - (Z)-1,5-dimethoxy-3-methyl-pent-2-ene,
   - 1,5-dimethoxy-3-methyl-pentane,
   - (Z)-1,5-diethoxy-3-methyl-pent-2-ene,
   - 1,5-diethoxy-3-methyl-pentane,
   - (E)-1,5-diisopropoxy-3-methyl-pent-2-ene,
   - (Z)-1,5-diisopropoxy-3-methyl-pent-2-ene,
   - 1,5-dibutoxy-3-methylene-pentane,
   - (E)-1,5-dibutoxy-3-methyl-pent-2-ene,
   - (Z)-1,5-dibutoxy-3-methyl-pent-2-ene
   - mixture of [(E)-5-acetoxy-3-methyl-pent-3-enyl] acetate and [(Z)-5-acetoxy-3-methyl-pent-3-enyl] acetate,
   - mixture of [(E)-3-methyl-5-propanoyloxy-pent-3-enyl] propanoate and [(Z)-3-methyl-5-propanoyloxy-pent-3-enyl] propanoate,
   - mixture of (E)-1,5-dimethoxy-3-methyl-pent-2-ene and (Z)-1,5-dimethoxy-3-methyl-pent-2-ene,
   - mixture of (Z)-1,5-diethoxy-3-methyl-pent-2-ene and (E)-1,5-diethoxy-3-methyl-pent-2-ene,
   - mixture of (E)-3-methyl-1,5-dipropoxy-pent-2-ene and (Z)-3-methyl-1,5-dipropoxy-pent-2-ene and
   - mixture of (E)-1,5-dibutoxy-3-methyl-pent-2-ene and (Z)-1,5-dibutoxy-3-methyl-pent-2-en.

Therefore, the present invention also relates to novel compounds of the general formula (I.a) 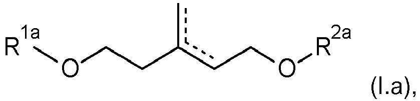 wherein
one of the symbols 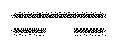 is a single bond and the other is a double bond or a single bond,
R^{1a} is hydrogen, C₁-C₄-alkyl, or -(C=O)-R³,
R^{2a} is hydrogen or C₁-C₄-alkyl,
R³ is hydrogen or C₁-C₄-alkyl,
a stereoisomer thereof or a mixture of stereoisomers thereof, with the proviso, if R^{1a} is hydrogen R^{2a} is different from hydrogen.

Furthermore, the present invention relates to novel compounds of the general formula (I.b) 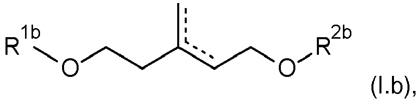 wherein
one of the symbols 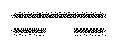 is a single bond and the other is a double bond or a single bond,
R^{1b} is hydrogen or -(C=O)-R³,
R^{2a} is hydrogen or -(C=O)-R⁴,
R³ is hydrogen or C₁-C₄-alkyl,
R⁴ is hydrogen or C₁-C₄-alkyl,
a stereoisomer thereof or a mixture of stereoisomers thereof, with the proviso that if R^{1b} is hydrogen R^{2b} is different from hydrogen.

The compounds of formula (I), (I.a) or (I.b), their stereoisomers or a mixture of stereoisomers, possess advantageous organoleptic properties, in particular a pleasant odor. Therefore, they can be favorably used as an aroma chemical for example in perfume compositions, cosmetic compositions, body care compositions, product for oral and dental hygiene, hygiene articles, cleaning compositions, textile detergent compositions, dishwashing compositions, scent dispensers, food, food supplements, pharmaceutical compositions, crop protection compositions and other ready-to-use compositions.

By virtue of their physical properties, the compounds of formula (I), (I.a) or (I.b), their stereoisomers or a mixture of stereoisomers, have particularly good, virtually universal solvent properties for other fragrances and other customary ingredients in fragranced ready-to-use compositions such as, in particular, perfume compositions. Therefore, the compounds of formula (I), (I.a) or (I.b), their stereoisomers or a mixture of stereoisomers, are favorably combinable with other aroma chemicals, allowing, in particular, the creation of perfume compositions having novel advantageous sensory profiles.

Furthermore, the compounds of formula (I), (I.a) or (I.b), their stereoisomers or a mixture of stereoisomers thereof, can be produced in good yields and purities by a one-step or a two-step synthesis, respectively, starting from readily available and cheap 3-methyl-pentan-diol and/or unsaturated derivates thereof. Thus, the compounds of formula (I), (I.a) or (I.b), their stereoisomers or a mixture of stereoisomers thereof, can be produced in large scales and in a simple and cost-efficient manner.

### DETAILED DESCRIPTION OF THE INVENTION

In the context of the present invention, the expression "C₁-C₄-alkyl" refers to methyl, ethyl, n-propyl, isopropyl, n-butyl, isobutyl and tert.-butyl. Preferably, the expression "C₁-C₄-alkyl" refers to methyl, ethyl, n-propyl and isopropyl, in particular to methyl and ethyl.

The term "aroma chemical" denotes a substance, which is used to obtain a sensory impression, and encompasses its use in "aroma chemical compositions" and/or "fragranced ready-to-use compositions". Accordingly, the term "aroma chemical composition", as used herein, refers to a composition, which predominately induces an odor impression. Likewise, the term "fragranced ready-to-use composition", as used herein, refers to a ready-to-use composition, which predominately induces an odor impression. The term "odor-intensive substances" refers to substances or aroma chemicals exhibiting intense odor impressions. Intense odor impressions are to be understood as meaning those properties of aroma chemicals, which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

"Advantageous sensory properties", "advantageous organoleptic properties" or "pleasant odor" are hedonistic expressions, which describe the niceness and conciseness of an odor impression conveyed by an aroma chemical. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "red berries": the odor would then be concisely of "red berries". If this red berries odor were very pleasant because the odor is reminiscent, for example, of sweet, fully ripe red berries, the odor would be termed "nice" and/or "sweet". However, the odor of a typically tart red berries can also be concise. If both reactions arise upon smelling the substance, in the example thus a nice and concise red berries odor, then this substance has particularly advantageous sensory properties.

The term "stereoisomers" denots isomers, which have the the same molecular formula and sequence of bonded atoms, but differ in the three-dimensional orientations of their atoms in space.

Firstly, the term "stereoisomers" refers to the E/Z isomers of the unsaturated compounds of formula (I), (I.a) or (I.b). The E-Z configuration describes the absolute stereochemistry of double bonds in organic chemistry. Each substituent on a double bond is assigned a priority. If the two groups of higher priority are on opposite sides of the double bond, the bond is assigned the configuration E. If the two groups of higher priority are on the same side of the double bond, the bond is assigned the configuration Z.

If, in the following, Z and E stereoisomers of the compounds (I), (I.a) or (I.b) are in question, only one of the forms is shown in each case. For the purposes of illustration only, the isomers of [5-formyloxy-3-methyl-pent-3-enyl] formate are shown below:

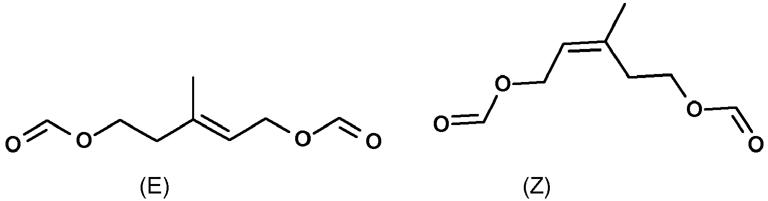

Secondly, the term "stereoisomer" refers also to optical isomers of the saturated compounds of formula (I), (I.a) or (I.b). It refers to all enantiomers in pure form and also racemic and optically active mixtures of the enantiomers of these compounds. Optical stereoisomer is a stereoisomer that has the opposite configuration at a stereocenter.

If, in the following, optical isomers of the saturated compounds (I), (I.a) or (I.b) are in question, only one of the enantiomeric forms is shown in each case. For the purposes of illustration only, the isomers of (5-hydroxy-3-methyl-pentyl) formate are shown below:

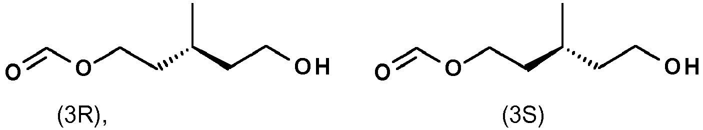

Depending on the presence and arrangement of the double bound, the compounds of the formula (I) can exist as individual exo-isomers (I.1) (exo), E-isomers (I.2) (E), Z-isomers (I.3) (Z) or as saturated-Isomer (I.4) (sat): 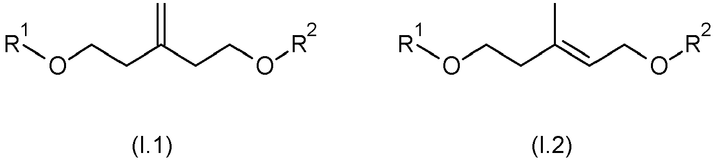 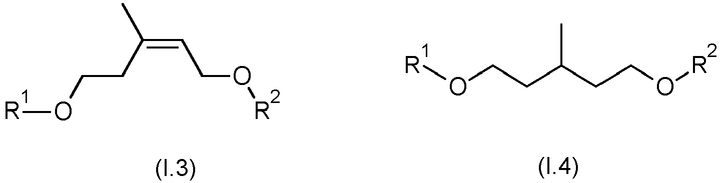 or as exo/E/Z/sat isomer mixtures or as exo/E/Z isomer mixtures.

The present invention thus relates to the use of the exo isomers, i.e. (I.1), the use of the E-isomers, i.e. (I.2), the use of the Z-isomers, i.e. (I.3), the use of the saturated isomers, i.e. (I.4) and also the use of exo/Z/E/sat isomer mixtures thereof, i.e. (I.1, I.2, I.3, I.4). Thus, if not stated otherwise, the expressions "compounds I", "compounds of the general formula I" and the like, as used herein, refers to the pure exo isomers, the pure E isomers, the pure Z isomer, the pure saturated isomer as well as to exo/E/Z/sat isomer mixtures thereof, in which the isomers are present in equal quantities or contain one of the isomers in excess or one or more isomers are absent.

The pure exo-isomers, the pure E-isomers, the pure Z-isomer and the pure saturated isomers as well as the exo/E/Z/sat isomer mixtures of the compounds (I) have all advantageous organoleptic properties. Thus, exo isomers, the pure E-isomer, the pure Z-isomer and the pure saturated isomers as well as the exo/E/Z/sat isomer mixtures of the compounds (I) are equally suitable for use as aroma chemicals.

On the other hand, usually the basic scent profile of the ether compounds of the general formula (I) differs from the scent profile of the ester compounds of the general formula (I).

Therefore a first embodiment of the present invention relates to the use of an ether compound of the general formula (I), where in formula (I)
- R¹: is C₁-C₄-alkyl and
- R²: is hydrogen or C₁-C₄-alkyl.

The compounds (I) of this first embodiment, encompass the mono-ethers as well as the di-ethers of 3-methyl-pentan-diol and unsaturated derivates thereof. Typically, the mono-ethers of 3-methyl-pentan-diol and unsaturated derivates thereof have all advantageous organoleptic properties. Thus, mono-ethers as well as the di-ethers of 3-methyl-pentan-diol and unsaturated derivates thereof are equally suitable for use as aroma chemicals.

Therefore, the di-ether compounds, i.e. the compounds of the general formula (I), where in formula (I) R¹ and R², independently of one another, are selected from C₁-C₄-alkyl, are preferred for use.

Furthermore, the synthesis of the ether compounds of the general formula (I), where the substituents attached to the oxygen atoms are identical, is generally simpler than the synthesis of the ether compounds of the general formula (I) having different substituents on the oxygen atoms.

Accordingly, further preferred for use are ether compounds of the general formula (I), wherein the radicals R¹ and R² are identical.

More preferred for use are ether compounds of the general formula (I), wherein R¹ and R² are C₁-C₃-alkyl.

Even more preferred for use are ether compounds of the general formula (I), wherein R¹ and R² are identical and selected from the group consisting of methyl, ethyl, n-propyl, isopropyl and n-butyl.

Particularly preferred for use are ether compounds of the general formula (I), wherein R¹ and R² are methyl, ethyl or n-butyl.

Especially preferred for use are ether compounds of the general formula (I), wherein R¹ and R² are ethyl.

Especially preferred for use are
- 1,5-diethoxy-3-methyl-pentane,
- 1,5-diethoxy-3-methylene-pentane,
- (E)-1,5-diethoxy-3-methyl-pent-2-ene,
- (Z)-1,5-diethoxy-3-methyl-pent-2-ene,
- 1,5-dimethoxy-3-methyl-pentane,
- 1,5-dimethoxy-3-methylene-pentane,
- (E)-1,5-dimethoxy-3-methyl-pent-2-ene,
- (Z)-1,5-dimethoxy-3-methyl-pent-2-ene,
- 1,5-dibutoxy-3-methyl-pentane,
- 1,5-dibutoxy-3-methylene-pentane,
- (E)-1,5-dibutoxy-3-methyl-pent-2-ene,
- (Z)-1,5-dibutoxy-3-methyl-pent-2-ene and
- mixtures thereof.

In another embodiment a composition comprising a mixture of at least two compounds selected of a compound of formulae (I.1), (I.2), (I.3) and (I.4) 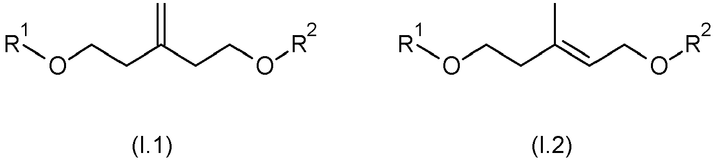 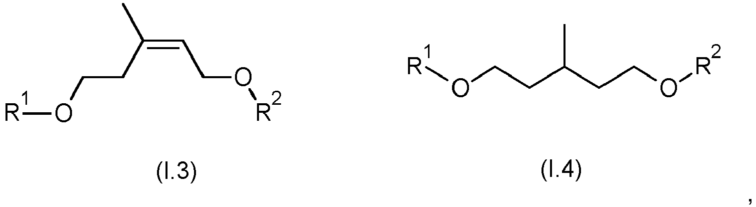 wherein R¹ and R² has one of the meanings as defined above and below, comprising each of the compounds in an amount of 1 - 99 % by weight, with the proviso that weight of the compounds of formulae (I.1), (I.2), (I.3) and (I.4) in said mixture adds up to 100%.

Preferred for use are compositions comprising a mixture of at least two compounds selected from the compounds of formulae (I.1), (I.2) and (I.3), comprising each of the compounds in an amount of 1 - 99 % by weight, with the proviso that weight of the compounds of formulae (1.1), (I.2), (I.3) and (I.4) in said mix-ture adds up to 100%.

More preferred for use are compositions comprising a mixture of at least two compounds selected from the compounds of formulae (I.1), (I.2) and (I.3), comprising each of the compounds in an amount of 1 - 99 % by weight, with the proviso that weight of the compounds of formulae (I.1), (I.2) and(I.3) in said mixture adds up to 100%, wherein the ratio of (I.1):(I.2):(I.3) is in the range of 90:5:5 to 10:45:45, preferably 80:10:10 to 20:40:40, especially 55:22.5:22.5 to 45:27.5:27.5, more especially is 50:25:25.

A second embodiment of the present invention relates to the use of an ester compound of the general formula (I), where in formula (I)
R¹ is -(C=O)-R³,
R² is hydrogen, -(C=O)-R⁴ or C₁-C₄-alkyl
R³ and R⁴ are as defined above.

The compounds (I) of this second embodiment, encompass the mono-esters as well as the di-esters and mono-ester-mono-ether of 3-methyl-pentan-diol and unsaturated derivates thereof. Typically, the mono-esters of 3-methyl-pentan-diol and unsaturated derivates thereof and the mono-ester-mono-ether of 3-methyl-pentan-diol and unsaturated derivates thereof have all advantageous organoleptic properties. Thus, mono-esters as well as the di-esters of 3-methyl-pentan-diol and unsaturated derivates thereof are equally suitable for use as aroma chemicals.

Therefore, the di-ester compounds, i.e. the compounds of the general formula (I), where in formula (I)
R¹ is -(C=O)-R³,
R² is -(C=O)-R⁴ and
R³ and R⁴ are as defined above,
are preferred for use.

Again, the synthesis of the ester compounds of the general formula (I), where the substituents attached to the oxygen atoms are identical, is generally simpler than the synthesis of the ester compounds of the general formula (I) having different substituents on the oxygen atoms.

Accordingly, further preferred for use are ester compounds of the general formula (I), wherein
R¹ is -(C=O)-R³,
R² is -(C=O)-R⁴, and
R³ and R⁴ are as defined above,
and where the radicals R³ and R⁴ are identical.

Further preferred for use are ester compounds of the general formula (I), wherein R³ and R⁴ are selected from the group consisting of hydrogen and C₁-C₃-alkyl.

Even more preferred for use are ester compounds of the general formula (I), wherein R³ and R⁴ are identical and selected from the group consisting of hydrogen and C₁-C₃-alkyl.

Particularly preferred for use are ester compounds of the general formula (I), wherein R³ and R⁴ are selected from the group consisting of hydrogen, methyl or ethyl.

Especially preferred for use are
- (3-formyloxy-2-methyl-propyl) formate,
- 2-(formyloxymethyl)allyl formate,
- [(E)-3-formyloxy-2-methyl-allyl] formate,
- [(Z)-3-formyloxy-2-methyl-allyl] formate,
- (3-acetoxy-2-methyl-propyl) acetate,
- 2-(acetoxymethyl)allyl acetate,
- [(E)-3-acetoxy-2-methyl-allyl] acetate,
- [(Z)-3-acetoxy-2-methyl-allyl] acetate,
- (2-methyl-3-propanoyloxy-propyl) propanoate,
- 2-(propanoyloxymethyl)allyl propanoate,
- [(E)-2-methyl-3-propanoyloxy-allyl] propanoate,
- [(Z)-2-methyl-3-propanoyloxy-allyl] propanoate, and
- mixtures thereof.

In another embodiment the invention relates to the use of a composition comprising a mixture of at least two compounds selected of a compound of formulae (I.1), (I.2), (I.3) and (I.4) 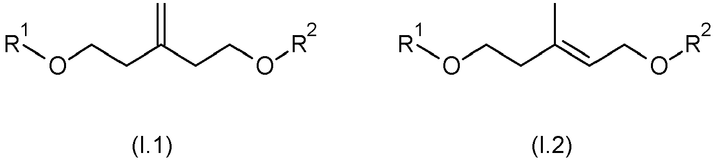 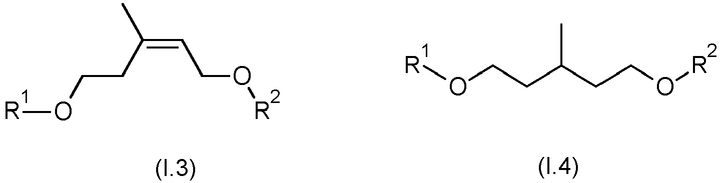 wherein R¹ and R² has one of the meanings as defined above and below, comprising each of the compounds in an amount of 1 - 99 % by weight, with the proviso that weight of the compounds of formulae (I.1), (I.2), (I.3) and (I.4) in said mixture adds up to 100%.

Preferred for use are compositions comprising a mixture of at least two compounds selected from the compounds of formulae (I.1), (I.2) and (I.3), comprising each of the compounds in an amount of 1 - 99 % by weight, with the proviso that weight of the compounds of formulae (1.1), (I.2), (I.3) and (I.4) in said mix-ture adds up to 100%.

More preferred for use are compositions comprising a mixture of at least two compounds selected from the compounds of formulae (I.1), (I.2) and (I.3), comprising each of the compounds in an amount of 1 - 99 % by weight, with the proviso that weight of the compounds of formulae (I.1), (I.2) and(I.3) in said mixture adds up to 100%, wherein the ratio of (I.1 ):(I.2):(I.3) is in the range of 90:5:5 to 10:45:45, preferably 80:10:10 to 20:40:40, especially 55:22.5:22.5 to 45:27.5:27.5, more especially is 50:25:25.

In a particular embodiment of the present invention, the ether and ester compounds of the general formula (I) are essentially present in the form of its exo isomer.

In another particular embodiment of the present invention, the ether and ester compounds of the general formula (I) are essentially present in the form of its E-isomers.

In another particular embodiment of the present invention, the ether and ester compounds of the general formula (I) are essentially present in the form of its Z-isomers.

In another particular embodiment of the present invention, the ether and ester compounds of the general formula (I) are essentially present in the form of its sat isomers.

In this connection, the term "essentially" means that the exo, E, Z or the sat isomer of the particular compound is present in an amount of at least 90% by weight, preferably in an amount of at least 95% by weight, in particular in an amount of at least 98% by weight, based on the total amount of the exo, E, Z and the sat isomers.

Typically, the compounds (I) are produced as exo/E/Z/sat isomer mixtures. The separation of the exo, E, Z and the sat isomers can be difficult and laborious or even cannot be achieved completely with reasonable effort.

Thus, a preferred embodiment of the present invention relates to the use of a compound of the general formula (I), as defined above and below, where the compound of the general formula (I) is present in the form of an exo/E/Z/sat isomer mixture, in particular in form of an exo/E/Z isomer mixture.

In one emodiment, in these mixtures, the four isomers can be present in equal amounts or almost equal amounts, e.g. in a exo/E/Z/sat isomer ratio of 25:25:25:25 or 31:23:23:23 or 23:31:23:23 or 23:23:31:23 or 23:23:23:31, or one of the isomers, i.e. either the exo, E, Z or sat isomer, can be present in excess, e.g. one of the isomers can be present in exo/E/Z/sat isomer ratio of 91:3:3:3, 3:91:3:3, 3:3:91:3 or:3:3:3:91. In a preferred embodiment the ratio of exo-isomer: E-isomer: Z-isomer: sat-isomer is from 48:25:25:2 to 49:25:25:1.

In another emodiment in these mixtures, the three isomers, exo/E/Z isomers can be present in equal amounts 33.3:33.3:33.3 or 40:30:30, or one of the isomers, i.e. either the exo, E or Z isomer, can be present in excess, e.g. one of the isomers can be present in a exo/E/Z or E/exo/Z or E/Z/exo or Z/exo/E or Z/E/exo isomer ratio of 90:9:1, 90:5:5, 80:10:10, 60:20:20, 50:25:25, 40:30:30, preferably the isomer ratio is from 75:12.5:12.5 to 45:27.5:27.5.

A preferred embodiment of the present invention relates to the use of a composition comprising a mixture of at least two compounds selected from the compounds of formulae (I.1), (I.2) and (I.3),
- (I.1) is in an amount of 25 to 75 weight %, preferably, 30 to 60 weight %, especially 35 to 55 weight %, based on the total weight of (I.1), (I.2) and (I.3),
- (I.2) is in an amount of 15 to 35 weight %, preferably, 18 to 30 weight %, especially 20 to 28 weight %, based on the total weight of (I.1), (I.2) and (I.3),
- (I.3) is in an amount of 15 to 35 weight %, preferably, 18 to 30 weight %, especially 20 to 28 weight %, based on the total weight of (I.1), (I.2) and (I.3),
with the proviso, that the weight of compound (1.1), (I.2) and (I.3) is add up to 100 %. A special embodiment ist the use of a composition comprising a mixture of at least two ether compounds of general formulae (I.1), (I.2), and (I.3) comprising each of the compound in said in an amount of 1 - 99 % by weight, with the proviso that weight of compound of formulae (1.1), (I.2), and (I.3) adds up to 100%, wherein the ratio of (I.1):(I.2):(I.3) is in the range of 90:5:5 to 10:45:45, preferably 80:10:10 to 20:40:40, especially 55:22.5:22.5 to 45:27.5:27.5, more especially is 50:25:25.

A preferred embodiment of the present invention relates to the use of a composition comprising a mixture of at least two compounds selected from the compounds of formulae (I.1), (I.2) and (I.3),
- (I.1) is in an amount of 25 to 75 weight %, preferably, 30 to 60 weight %, especially 35 to 55 weight %, based on the total weight of (I.1), (I.2) and (I.3),
- (I.2) is in an amount of 15 to 35 weight %, preferably, 18 to 30 weight %, especially 20 to 28 weight %, based on the total weight of (I.1), (I.2) and (I.3),
- (I.3) is in an amount of 15 to 35 weight %, preferably, 18 to 30 weight %, especially 20 to 28 weight %, based on the total weight of (I.1), (I.2) and (I.3),
with the proviso, that the weight of compound (1.1), (I.2) and (I.3) is add up to 100 %.

A special embodiment is the use of a composition comprising a mixture of at least two ester compounds of general formulae (I.1), (I.2), and (I.3) comprising each of the compound in said in an amount of 1 - 99 % by weight, with the proviso that weight of compound of formulae (1.1), (I.2), and (I.3) adds up to 100%, wherein the ratio of (I.1):(I.2):(I.3) is in the range of 90:5:5 to 10:45:45, preferably 80:10:10 to 20:40:40, especially 55:22.5:22.5 to 45:27.5:27.5, more especially is 50:25:25.

Furthermore, the compounds (I), (I.a) or (I.b) can be used as aroma chemicals over a wide range of purity, as long as the impurities do not have a significant detrimental effect on the scent of the compounds (I), (I.a) or (I.b). For the use as aroma chemicals, according to the invention, the purity of the compounds (I), (I.a) or (I.b) is therefore not specifically limited. Preferably, the compounds (I), (I.a) or (I.b) have a purity of at least 70%, in particular of at least 90% and especially of at least 95%.

Due to the method of their preparation starting from 3-methyl-pentan-diol and/or unsaturated derivates thereof, the di-ether compounds of the general formula (I) or (I.a) may comprise minor amounts of mono-ether compounds (I-OH) and the di-ester compounds of the general formula (I) or (I.b) may comprise minor amount of mono-ester compounds (II-OH) 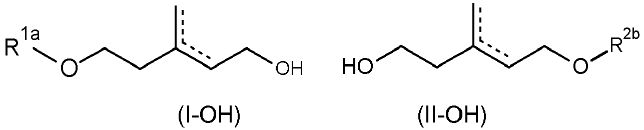 wherein R^{1a} and R^{2b} have one of the meaning given above.

Preferably, the amounts of mono-substituted compounds (I-OH) or (II-OH), which can be comprised in the di-ether and the di-ester compounds of the general formula (I) are less than 25% by weight, more preferably less than 10% by weight, even more preferably less than 5% by weight, in particular less than 1% by weight, based on the total amount of the compound (I).

In a particularly preferred embodiment of the present invention the di-ether and di-ester compounds of the general formula (I) do not comprise mono-substituted compounds (I-OH) or (II-OH), respectively.

Likewise, the mono-ether and the mono-ester compounds of the general formula (I) may comprise minor amounts of the corresponding di-substituted compounds. Preferably, the amounts of di-substituted compounds of the general formula (I), which can be comprised in the mono-ether and the mono-ester compounds of the general formula (I) are less than 25% by weight, more preferably less than 10% by weight, even more preferably less than 5% by weight, in particular less than 1 % by weight, based on the total amount of the compound (I).

Furthermore, the di-ether compounds of the general formula (I), wherein the radicals R¹ and R² are different, as well as the di-ester compounds of the general formula (I), wherein the radicals R³ and R⁴ are different, hereinafter referred to as unsymmetrically substituted compounds, may comprise minor amounts of the corresponding symmetrically substituted compounds, i.e. di-ether compounds of the general formula (I), wherein the radicals R¹ and R² are identical, and di-esters compounds of the general formula (I), wherein the radicals R³ and R⁴ are identical. Preferably, the amounts of symmetrically substituted compounds, which can be comprised in the unsymmetrically substituted compounds of the general formula (I) are less than 25% by weight, more preferably less than 10% by weight, even more preferably less than 5% by weight, in particular less than 1% by weight, based on the total amount of the compound (I).

The aforesaid preferred embodiments can be combined with one another as desired.

Accordingly, in a particular preferred embodiment, the present invention relates to the use of a compound of the general formula (I), which has a purity of at least 95% and/or is present in the form of a exo/E/Z isomer mixture as mentioned above.

The mono-ether compounds of the general formula (I) can efficiently be prepared by alkylating compound (II) or mixtures thereof 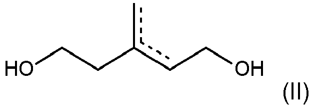 using the alkylation reagent R¹-X, wherein R¹ has one of the meanings given above and X represents a leaving group, selected from halogen, such as Cl, Br, I, and sulfonates, such as tosylate, mesylate, triflate or nonaflate, typically in the presence of a base.

Suitable bases are typically selected from inorganic bases and organic bases.

Suitable inorganic bases that can be used in this alkylation reaction are for example alkali metal carbonates, e.g. Li₂CO₃, Na₂CO₃, K₂CO₃ or Cs₂CO₃, alkali metal hydroxides, e.g. LiOH, NaOH or KOH, and hydride donors, e.g. NaH, LiAlH₄ or NaBH₄.

Suitable organic bases that can be used in this alkylation reaction are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The alkylation reaction is performed under conventional alkylation reaction conditions that are well known to the skilled person.

Typically, compound (II) or mixtures thereof are reacted with sub-equimolar or almost equimolar amounts, e.g. 0.7, 0.8, 0.9, or 0.95 equivalents, of the alkylation reagent R¹-X to give the mono-substituted compound (I-OH), which is further subjected to a purification step in order to remove unwanted by-products or impurities, such as residual starting material or the corresponding di-substituted compounds.

Generally, the purification step is performed by using common purification methods, such as crystallization, distillation or chromatographic methods, e.g. column chromatography or high performance liquid chromatography.

The preparation of di-ether compounds of the general formula (I), where the radicals R¹ and R² are identical, is typically performed by reacting of a compound (II) or mixtures thereof with at least two equivalents, e.g. 2.0, 2.1, 2.5 or 3.0 equivalents, of the alkylation reagent R¹-X or R²-X, respectively, wherein the radicals R¹, R² and X have one of the meanings given above, and the obtained raw product is subsequently subjected to a purification step, as defined above. In case where only minor amounts of impurities and by-products are present in the raw-product mixture the purification step can also be performed via a simple extractive workup.

The preparation of di-ether compounds of the general formula (I), where the radicals R¹ and R² are different, is generally performed by first reacting a compound (II) or mixtures thereof with sub-equimolar or almost equimolar amounts, e.g. 0.7, 0.8, 0.9, or 0.95 equivalents, of the alkylation reagent R¹-X to give the mono-substituted compound (I-OH), which after a purification step is further reacted with the second alkylation reagent R²-X.

Generally, the ester compounds of the general formula (I) can efficiently be prepared by reacting a compound (II) or mixtures thereof with the carboxylic acid R³-COOH, with the carboxylic acid R⁴-COOH, wherein R³ and R⁴ have one of the meanings given above, or an acid anhydride thereof, or an acid halogenide of the formulae R³-(C=O)X' or R⁴⁻(C=O)X or a mixture of the carboxylic acid R³-COOH and/or R⁴-COOH with an acid anhydride thereof and acid halogenide. The reaction is typically performed in the presence of an esterification catalyst or a base.

Suitable esterification catalysts that can be applied in this reaction are well known to the skilled person. Suitable esterification catalysts are for example metal based catalysts, e.g. iron, cadmium, cobalt, lead, zinc, antimony, magnesium, titanium and tin catalysts in the form of metals, metal oxides or metal salts, such as metal alcoxylates; mineral acids, such as sulfuric acid, hydrochloric acid or phosphoric acid; or organic sulfonic acids, such as methane sulfonic acid or para-toluene sulfonic acid.

Suitable bases are for example organic bases, as defined above.

Alternatively, the ester compounds of the general formula (I) can be prepared by reacting a compound (II) or mixtures thereof with an acid halogenide of the formulae R³-(C=O)X' or R⁴-(C=O)X', wherein R³ and R⁴ have one of the meanings given above and X' is halogen, such as Cl, Br or I, in the presence of an organic base.

Suitable organic bases are as defined above.

The individual reaction conditions for the preparation of the ester compounds of the general formula (I) are well known to the skilled person.

For the preparation of the mono-ester compounds of the general formula (I), compound (II) or mixtures thereof are typically reacted with sub-equimolar or almost equimolar amounts, e.g. 0.7, 0.8, 0.9, or 0.95 equivalents, of the carboxylic acid R³-COOH or an anhydride thereof or acid halogenide thereof or a mixture of said carboxylic acid with an anhydride thereof and/or acid halogenide. Alternatively, compound (II) or mixtures thereof are typically reacted with the same amounts of the acid halogenide R³-(C=O)X' in the presence of an organic base. The obtained raw product is subsequently subjected to a purification step, as defined above.

For the preparation of di-ester compounds of the general formula (I), where the radicals R³ and R⁴ are identical, compound (II) or mixtures thereof is typically reacted with at least two equivalents, e.g. 2.0, 2.1, 2.5 or 3.0 equivalents, of the carboxylic acid R³-COOH or R⁴-COOH, respectively, an anhydride thereof or a mixture of said carboxylic acid with an anhydride thereof. Alternatively, compound (II) or mixtures thereof are typically reacted with at least two equivalents of the acid halogenide R³-(C=O)X' or R⁴-(C=O)X', respectively, in the presence of an organic base. The obtained raw product is subsequently subjected to a purification step, as defined above. In case where only minor amounts of impurities and by-products are present in the raw-product mixture the purification step can also be performed via a simple extractive workup.

The preparation of di-ester compounds of the general formula (I), where the radicals R³ and R⁴ are different, is generally performed by first reacting of compound (II) or mixtures thereof with sub-equimolar or almost equimolar amounts, e.g. 0.7, 0.8, 0.9, or 0.95 equivalents, of the carboxylic acid R³-COOH, or an anhydride thereof, to give the mono-substituted compound (II-OH), which after a purification step is further reacted with a second carboxylic acid R⁴-COOH, or an anhydride thereof.

Alternatively, the preparation of compounds (I), where the radicals R³ and R⁴ are different, can also be achieved by first reacting of compound (II) or mixtures thereof with sub-equimolar or almost equimolar amounts, e.g. 0.7, 0.8, 0.9, or 0.95 equivalents, of the acid halogenide R³-(C=O)X' in the presence of an organic base to give the mono-substituted compound (II-OH), which after a purification step is further reacted with a second acid halogenide R⁴-(C=O)X'.

The starting material, compound (II) or mixtures thereof, is a diol building block that is frequently used as a monomer for the synthesis of polymeric materials. Thus, compound (II) or mixtures thereof are readily available from commercial sources. Alternatively, compound (II) or mixtures thereof can also by synthesized in large quantities using processes that are well described in the art, e.g. via the dimerization of dimethylketene.

### Compositions:

The compounds of formula (I), the stereoisomers thereof or a mixture of stereoisomers thereof can be used in a wide range of compositions, such as ready-to-use compositions. The olfactory properties, the substance properties (such as solubility in customary solvents and compatibility with further customary constituents of such compositions), as well as the toxicological acceptability of the compounds of formula (I), underline their particular suitability for the stated use purposes and compositions.

Furthermore, the compounds of the general formula (I) exhibit advantageous secondary properties.

For example, the compounds of the general formula (I) can provide better sensory profiles as a result of synergistic effects with other fragrances, which means that they can provide a booster effect for other fragrances. They are therefore suitable as boosters for other fragrances.

Booster effect means that the substances enhance and intensify, in perfumery formulations, the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione® (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compounds of the general formula (I), the stereoisomers thereof or a mixture of stereoisomers thereof are generally used in an amount of 0.1 - 20% by weight, preferably in an amount of 0.5 to 5% by weight, in particular in an amount of from 0.6 to 3% by weight, based on the total weight of the fragrance mixture.

Furthermore, the compounds of the general formula (I), the stereoisomers thereof or a mixture of stereoisomers thereof can have further positive effects on the composition itself, where they are applied in. For example, the compounds of the general formula (I), the stereoisomers thereof or a mixture of stereoisomers thereof can enhance the overall performance of the composition, such as the stability, e.g. the formulation stability, the extendability or the staying power of the composition.

Accordingly, a further embodiment of the invention therefore relates to the use of a compound of the general formula (I), a stereoisomer thereof or a mixture of stereoisomers thereof, as an aroma chemical in a ready-to-use composition.

The term "ready-to-use composition", as used herein, refers to composition, which is intended to be applied or used on its own by the final user.

Generally, the ready-to-use composition, in which the aroma chemicals of the present invention, i.e. the compounds of the general formula (I), as defined above, can be applied, are fragranced ready-to-use compositions.

Fragranced ready-to-use composition, in which the aroma chemicals of the present invention, i.e. the compounds of the general formula (I), as defined above, can be applied, are for example compositions used in personal care, compositions used in home care, compositions used in industrial applications as well as compositions used in other applications, such as pharmaceutical compositions or crop protection compositions.

Preferably, the ready-to-use compositions are selected from perfume composition, cosmetic composition, body care composition, product for oral and dental hygiene, hygiene article, cleaning composition, textile detergent composition, dishwashing compositions, scent dispensers, food, food supplement, pharmaceutical composition and crop protection composition.

Perfume compositions can be selected from fine fragrances, air fresheners in liquid form, gel-like form or a form applied to a solid carrier, aerosol sprays, scented cleaners, perfumed candles and oils, such as lamp oils or oils for massage.

Exemples for fine fragrances are perfume extracts, Eau de Parfums, Eau de Toilettes, Eau de Colognes, Eau de Solide and Extrait Parfum and the like.

Body care compositions can be selected from aftershaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, aftershave creams and lotions and tanning creams and lotions, powders, hydrogels, hair care products, such as e.g. hairsprays, hair gels, setting hair lotions, hair conditioners, hair shampoo and permanent and semi-permanent hair colorants, hair shaping compositions, such as cold waves and hair smoothing compositions, hair tonics, hair creams and hair lotions, deodorants and antiperspirants, such as e.g. underarm sprays, roll-ons, deodorant sticks and deodorant creams, products of decorative cosmetics, such as e.g. eye-liners eye-shadows, nail varnishes, make-ups, lipsticks and mascara.

Products for oral and dental hygiene can be selected from toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces can be selected from perfumed acidic, alkaline and neutral cleaners, such as e.g. floor cleaners, window cleaners, bath and sanitary cleaners, scouring milk, solid and liquid toilet cleaners, powder and foam carpet cleaners, dishwashing detergents for hand and machine dishwashing, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers and facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions, which are intended for use in the diagnosis, cure, mitigation, treatment, or prevention of disease as well as articles (other than food) intended to affect the structure or any function of the body of man or other animals.

Crop protection compositions comprise compositions, which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, anti-inflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant sub-stances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, polyols, surfactants, electrolytes, organic solvents or silicone derivatives.

As mentioned above, it was further found that the compounds of the general formula (I) generally exhibit a pleasant and characteristic odor and can be used to produce fragranced ready-to-use compositions and/or they can advantageously be combined with other aroma chemicals different from compounds (I) to create new scent profiles.

Accordingly, a specific embodiment of the present invention relates to the use of a compound of formula (I), a stereoisomer thereof or a mixture of stereoisomers thereof for modifying the scent character of a fragranced ready-to-use composition.

The present invention further relates to aroma chemical compositions comprising at least one compound of formula (I), a stereoisomer thereof or a mixture of stereoisomers thereof, as defined above, and at least one further compound selected from the group consisting of further aroma chemicals different from compounds (I) and non-aroma chemical carriers.

This includes aroma chemical compositions comprising at least one compound of formula (I), a stereoisomer thereof or a mixture of stereoisomers thereof, as defined above, and at least one further aroma chemical different from compounds (I).

Further included are aroma chemical compositions comprising at least one compound of formula (I), a stereoisomer thereof or a mixture of stereoisomers thereof, as defined above, and at least one non-aroma chemical carrier.

Also included are aroma chemical compositions comprising at least one compound of formulae (I), a stereoisomer thereof or a mixture of stereoisomers thereof, as defined above, at least one further aroma chemical different from compounds (I) and at least one non-aroma chemical carrier.

Preferably, the aroma chemical compositions comprise at least one compound of formulae (I), a stereoisomer thereof or a mixture of stereoisomers thereof, as defined above, at least one further aroma chemical different from compounds (I) and at least one non-aroma chemical carrier.

The further aroma chemical different from compounds (I) can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60% by weight) (Hedione⁹, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lilial²), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide¹), 15-cyclopentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol(Florol⁹), 2-ethyl-4-(2,2,3-trimethyl-3-cyclopenten-1-yl)-2-buten-1-ol(Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)-propanal (Helional³), 3-(4-ethylphenyl)-2,2-dimethylpropanal (Florazon¹), 7-methyl-2H-1,5-benzodioxepin-3(4H)-one (Calone), 3,3,5-trimethylcyclohexyl acetate (preferably with a content of cis isomers of 70% by weight) or more and 2,5,5-trimethyl-1,2,3,4,4a,5,6,7-octahydronaphthalen-2-ol (Ambrinol S¹).

Where trade names are given above, these refer to the following sources:
¹ trade name of Symrise GmbH, Germany;
² trade name of Givaudan AG, Switzerland;
³ trade name of International Flavors & Fragrances Inc., USA;
⁵ trade name of Danisco Seillans S.A., France;
⁹ trade name of Firmenich S.A., Switzerland;
¹⁰ trade name of PFW Aroma Chemicals B.V., the Netherlands.

Further aroma chemicals with which the compounds of formula (I), the stereoisomers thereof or a mixture of stereoisomers thereof, as defined above, can be combined e.g. to give a composition according to the invention can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
taliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial,
neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano-naphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
cyclic and macrocyclic ketones such as e.g. 4-tert-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclohexadecen-1-one; 7-cyclohexadecen-1-one; (7/8)-cyclohexadecen-1-one; 9-cycloheptadecen-1-one; cyclopentadecanone; cyclohexadecanone;
cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1 ,2,3,4,5,6,7,8-octahydro-2-naphthalenyl methyl ketone; methyl 2,6,10-trimethyl-2,5,9-cyclododecatrienyl ketone; tert-butyl (2,4-dimethyl-3-cyclohexen-1-yl) ketone;
esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
esters of cycloaliphatic alcohols such as e.g. 1-cyclohexylethyl crotonate;
esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl-2-methyl-1,3-dioxolane-2-acetate;
araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
aromatic and araliphatic aldehydes such as e.g. benzaldehyde; phenylacetaldehyde; 3-phenylpropanal; hydratropaaldehyde; 4-methylbenzaldehyde; 4-methylphenyl-acetaldehyde; 3-(4-ethylphenyl)-2,2-dimethylpropanal; 2-methyl-3-(4-isopropylphenyl)-propanal; 2-methyl-3-(4-tert-butylphenyl)propanal; 2-methyl-3-(4-isobutylphenyl)-propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
aromatic and araliphatic ketones such as e.g. acetophenone; 4-methylacetophenone; 4-methoxyacetophenone; 4-tert-butyl-2,6-dimethylacetophenone; 4-phenyl-2-butanone; 4-(4-hydroxyphenyl)-2-butanone; 1-(2-naphthalenyl)ethanone; 2-benzofuranyl-ethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropyl-pyrazine; 2-isobutyl-3-methoxypyrazine;
phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl) phenol; p-cresyl phenylacetate;
heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

The at least one non-aroma chemical carrier can be a compound, a mixture of compounds or other additives, which have no or no noteworthy sensory properties. The non-aroma chemical carrier serves for the dilution and/or the fixing of the aroma chemical(s), i.e. the compounds of formula (I) and optionally one or more further aroma chemical different from compounds (I), as defined above, comprised in the aroma chemical composition.

Suitable carrier materials can be liquid or oil-like carrier materials as well as wax-like or solid carrier materials.

Suitable liquid or oil-like carrier materials are selected, for example, from water, alcohols, such as methanol or ethanol, aliphatic diols and polyols with a melting temperature below 20°C, such as ethylene glycol, glycerol, diglycerol, propylene glycol or dipropylene glycol and 1,2-butylene glycol, cyclic siloxanes, such as hexamethylcyclotrisiloxane or decamethylcyclopentasiloxane, diethylene glycol monoethyl ether, diethyl phthalate, isopropyl myristate, triethyl citrate, benzyl benzoate, vegetable oils, such as fractionated coconut oil or esters of fatty alcohols with melting temperatures below 20°C, such as tetradecyl acetate or tetradecyl lactate, esters of glycerol with melting temperatures below 20°C, and alkyl esters of fatty acids with melting temperatures below 20°C, such as isopropyl myristate.

Suitable wax-like or solid carrier materials are selected, for example, from fatty alcohols with melting temperatures below 20°C, such as myristyl alcohol, stearyl alcohol or cetyl alcohol, polyols with melting temperatures above 20°C, fatty acid esters with fatty alcohols which have a melting temperature of above 20°C, such as lanolin, beeswax, carnauba wax, candelilla wax or Japan wax, waxes produced from petroleum, such as hard paraffin, water-insoluble porous minerals, such as silica gel, silicates, for example talc, microporous crystalline aluminosilicates (zeolites), clay minerals, for example bentonite, or phosphates, for example sodium tripolyphosphate, paper, cardboard, wood, textile composite or nonwoven materials made of natural and/or synthetic fibers.

Suitable carrier materials are also selected, for example, from water-soluble polymers, such as polyacrylic acid esters or quaternized polyvinylpyrrolidones, or water-alcohol-soluble polymers, such as specific thermoplastic polyesters and polyamides. The polymeric carrier material can be present in various forms, e.g. in the form of a gel, a paste, solid particles, such as microcapsules, or brittle coatings.

Preferably, the aroma chemical composition is selected from fragranced ready-to-use compositions, as defined above.

Generally, the total amount of the at least one compound of formula (I), a stereoisomer thereof or a mixture of stereoisomers thereof in the aroma chemical compositions according to the present invention are typically adapted to the particular intended use or the intended application and can, thus, vary over a wide range. As a rule, the customary standard commercial use amounts for scents are used.

Accordingly, the total amount of the at least one compound of formula (I), a stereoisomer thereof or a mixture of stereoisomers thereof in the composition is in the range of from 0.001 to 99.9% by weight, preferably in the range of from 0.01 to 90% by weight, more preferably in the range of from 0.05 to 80 % by weight, even more preferably in the range of from 0.1 to 60 % by weight, in particular in the range of from 0.1 to 40 % by weight, based on the total weight of the composition.

In one embodiment of the invention, the total amount of the at least one compound of formula (I), a stereoisomer thereof or a mixture of stereoisomers thereof in the composition is in the range of from 0.001 to 5 weight%, preferably from 0.01 to 2 weight%, based on the total weight of the composition.

A further embodiment of the invention is directed to a composition comprising at least one compound of formula (I), a stereoisomer thereof or a mixture of stereoisomers thereof and at least one component selected from the group consisting of surfactants, emollients and solvents.

One embodiment of the invention is directed to a composition comprising at least one compound of formula (I), a stereoisomer thereof or a mixture of stereoisomers thereof and at least one solvent.

In the context of the present invention, a "solvent" serves for the dilution of the compound of formula (I), a stereoisomer thereof or a mixture of stereoisomers thereof, as defined above. Some solvents have fixing properties at the same time.

The one or more solvent(s) can be present in the composition from 0.01 to 99% by weight based on the composition. In a preferred embodiment of the invention, the composition comprise 0.1 to 90 % by weight, preferably 0.5 to 80 % by weight of solvent(s) based on the composition. The amount of solvent(s) can be chosen depending on the composition. In one embodiment of the invention, the composition comprises 0.05 to 10 % by weight, preferably 0.1 to 5 % by weight, more preferably 0.2 to 3 % by weight based on the composition. In one embodiment of the invention, the composition comprises 20 to 70 % by weight, preferably 25 to 50 % by weight of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compounds of formula (I), the stereoisomers thereof or a mixture of stereoisomers thereof are suitable for use in surfactant-containing compositions. According to their characteristic scent profiles, the compounds of formula (I), the stereoisomers thereof or a mixture of stereoisomers thereof can especially be used for the perfuming of surfactant-containing compositions such as, for example, cleaners (in particular laundry cleaners and all-purpose cleaners).

One embodiment of the invention is therefore directed to a composition comprising at least one compound of formula (I), a stereoisomer thereof or a mixture of stereoisomers thereof and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C8 to C18 alkyl or acyl group, contain at least one free amino group and at least one -COOH- or -SO₃H-group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C₁₂ to C₈ alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart® series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising at least one compound of formula (I), a stereoisomer thereof or a mixture of stereoisomers thereof and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15% by weight based on the composition.

The oil components may be selected, for example, from Guerbet alcohols based on fatty alcohols con taining 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl ole- ate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol@ CC), Guerbet carbonates based on fatty alcohols containing 6 to 18 and preferably 8 to 10 carbon atoms, esters of benzoic acid with linear and/or branched C₆ to C₂₂-alcohols (for example Finsolv® TN), linear or branched, symmetrical or nonsymmetrical dialkyl ethers containing 6 to 22 carbon atoms per alkyl group such as, for example, dicaprylyl ether (Cetiol® OE), ring opening products of epoxidized fatty acid esters with polyols and hydrocarbons or mixtures thereof.

### Method of preparation:

A further embodiment of the present invention relates to a method of preparing a fragranced ready-to-use composition, comprising incorporating at least one compound of formula (I), a stereoisomer thereof or a mixture of stereoisomers thereof, as defined above, into a ready-to-use composition.

This method comprises the incorporation of at least one compound of formula (I), a stereoisomer thereof or a mixture of stereoisomers thereof, to an ready-to-use composition, which has no or no notable sensory properties, on order to provide a specific odor and/or a specific flavor to this ready-to-use composition. In addition, this method also comprises the modification of the odor and/or the flavor of an ready-to-use composition, which already has notable sensory properties, by incorporating at least one compound of formula (I), a stereoisomer thereof or a mixture of stereoisomers thereof, into said ready-to-use composition.

Preferred ready-to-use compositions are those mentioned above.

The total amount of the at least one compound of formula (I), a stereoisomer thereof or a mixture of stereoisomers thereof, that is incorporated in the ready-to-use compositions highly depend on the intended use or the intended application and can, thus, vary over a wide range. Typical amounts of the at least one compound of formulae (I), a stereoisomer thereof or a mixture of stereoisomers thereof that is incorporated in the ready-to-use compositions are those as defined above for the compositions.

The compounds of formula (I), the stereoisomers thereof or a mixture of stereoisomers thereof used according to the invention, the compositions obtainable by the above method of the invention, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the compounds of formula (I), the stereoisomers thereof or a mixture of stereoisomers thereof, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compounds of formula (I), the stereoisomers thereof or a mixture of stereoisomers thereof with a suitable wax-like substance and by extrusion with subsequent solidification, optionally in a suitable solvent, e.g. isopropanol.

A further embodiment of the present invention relates to a ether compound of the general formula (I.a) 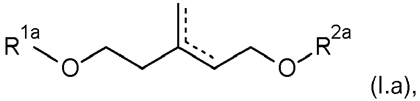 wherein
one of the symbols 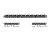 is a single bond and the other is a double bond or a single bond, wherein R^{1a}, and R^{2a} has one of the meanings as defined above a stereoisomer or a mixture of stereoisomers.

A preferred embodiment of the present invention relates to the di-ether compounds of the general formula (I.a), i.e. to compounds of the general formula (I.a), wherein R^{1a} and R^{2a}, independently of one another, are selected from C₁-C₄-alkyl.

Further preferred are di-ether compounds of the general formula (I.a), wherein the radicals R^{1a} and R^{2a} are identical.

More preferred are compounds of the general formula (I.a), wherein the radicals R^{1a} and R^{2a} are selected from the group consisting of methyl, ethyl, n-propyl,ispopropyl and n-butyl.

Even more preferred are compounds of the general formula (I.a), wherein the radicals R^{1a} and R^{2a} are identical and selected from the group consisting of methyl and ethyl.

Particularly preferred compounds (I.a) are
- 1,5-diethoxy-3-methylene-pentane,
- (E)-1,5-diethoxy-3-methyl-pent-2-ene,
- 3-methyl-1,5-dipropoxy-pentane,
- 3-methylene-1,5-dipropoxy-pentane,
- (E)-3-methyl-1,5-dipropoxy-pent-2-ene,
- (Z)-3-methyl-1,5-dipropoxy-pent-2-ene,
- 1,5-diisopropoxy-3-methyl-pentane,
- 1,5-diisopropoxy-3-methylene-pentane
- 1,5-dibutoxy-3-methyl-pentane,
- 1,5-ditert-butoxy-3-methyl-pentane,
- 1,5-ditert-butoxy-3-methylene-pentane
- (E)-1,5-ditert-butoxy-3-methyl-pent-2-ene,
- (Z)-1,5-ditert-butoxy-3-methyl-pent-2-ene.

The compounds of the general formula (I.a) can be present in the form of a exo/E/Z/sat isomer mixture, or can be essentially present in the form of their exo isomers, their E-isomer, their Z isomer or their sat isomers, respectively.

Regarding the expressions "exo/E/Z/sat isomer mixture" and "essentially present", reference is made to the statements given above for the compounds of the general formula (I).

Preferably, the compounds of the general formula (I.a) are present in the form of a exo/E/Z/sat isomer mixture, especially in the form of a exo/E/Z isomer mixture.

The compounds of the general formula (I.a) can be prepared as described for the compounds of the general formula (I).

A further embodiment of the present invention relates to a compound of the general formula (I.b) 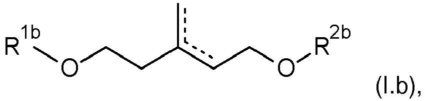 wherein
one of the symbols 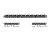 is a single bond and the other is a double bond or a single bond, wherein R^{1b} and R^{2b} has one of the meanings as defined above, a stereoisomer thereof, or a mixture of stereoisomers thereof.

A preferred embodiment of the present invention relates to the di-ester compounds of the general formula (I.b), i.e. to compounds of the general formula (I.b), wherein R^{2b} is-(C=O)-R⁴.

Further preferred are compounds of the general formula (I.b), where R³ and R⁴ are selected from the group consisting of hydrogen and C₁-C₃-alkyl, in particular from the group consisting of hydrogen, methyl and ethyl.

Examples of preferred compounds of the general formula (I.b) are
- (5-formyloxy-3-methylene-pentyl) formate,
- (5-formyloxy-3-methyl-pentyl) formate,
- (3-methylene-5-propanoyloxy-pentyl) propanoate,
- [(E)-3-methyl-5-propanoyloxy-pent-3-enyl] propanoate,
- [(Z)-3-methyl-5-propanoyloxy-pent-3-enyl] propanoate,
- [5-(2,2-dimethylpropanoyloxy)-3-methylene-pentyl] 2,2-dimethylpropanoate,
- [(E)-5-(2,2-dimethylpropanoyloxy)-3-methyl-pent-3-enyl] 2,2-dimethylpropanoate,
- [(Z)-5-(2,2-dimethylpropanoyloxy)-3-methyl-pent-3-enyl] 2,2-dimethylpropanoate,

The compounds of the general formula (I.b) can be present in the form of a exo/E/Z/sat isomer mixture, or can be essentially present in the form of their exo isomers, their E-isomer, their Z isomer or their sat isomers, respectively.

Regarding the expressions "exo/E/Z/sat isomer mixture" and "essentially present", reference is made to the statements given above for the compounds of the general formula (I).

Preferably, the compounds of the general formula (I.b) are present in the form of a exo/E/Z/sat isomer mixture, especially in the form of a exo/E/Z isomer mixture.

The compounds of the general formula (I.b) can be prepared as described for the ester compounds of the general formula (I).

Another prederred embodiment of the invention are composition comprising a mixture of at least two compounds selected from the compounds of formulae (I.1), (I.2), (I.3) and (I.4) 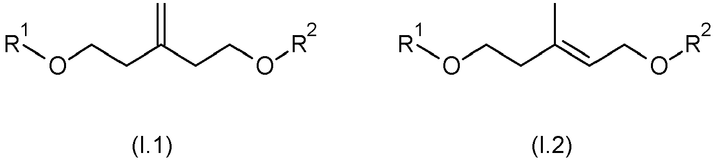 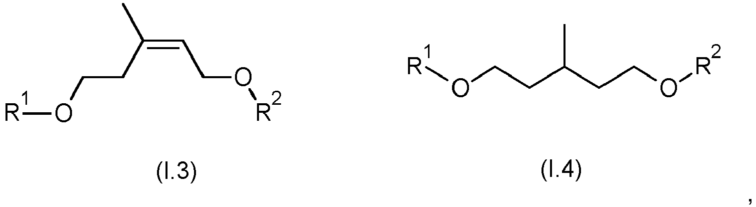 wherein R¹ and R² has one of the meanings as defined above and below, comprising each of the compounds in an amount of 1 - 99 % by weight, with the proviso that weight of the compounds of formulae (I.1), (I.2), (I.3) and (I.4) in said mixture adds up to 100%.

In a preferred embodiment the invention relates to a compostion comprising a mixture of at least two compounds selected from the compounds of formulae (I.1), (I.2) and (I.3), comprising each of the compounds in an amount of 1 - 99 % by weight, with the proviso that weight of the compounds of formulae (I.1), (I.2) and (I.3) in said mixture adds up to 100%.

Preferrably, the composition comprising a mixture of at least two compounds selected from the compounds of formulae (I.1), (I.2) and (I.3), comprising each of the compounds in an amount of 1 - 99 % by weight, with the proviso that weight of the compounds of formulae (1.1), (I.2) and (I.3) in said mixture adds up to 100%, and wherein the ratio of (I.1 ):(I.2):(I.3) is in the range of 90:5:5 to 10:45:45, preferably 80:10:10 to 20:40:40, especially 55:22.5:22.5 to 45:27.5:27.5, more escpecially is 50:25:25.

In particular the composition comprising a mixtures of at least two compounds selected from the compounds of formulae (I.1), (I.2) and (I.3) wherein
R¹ and R² are C₁-C₄-alkyl.

In particular the composition comprising a mixtures of at least two compounds selected from the compounds of formulae (I.1), (I.2) and (I.3) wherein
R¹ is -(C=O)-R³,
R² -(C=O)-R⁴, R³ and
R⁴ are C₁-C₄-alkyl.

A preferred embodiment of the present invention relates to a composition comprising a mixture of at least two compounds selected from the compounds of formulae (I.1), (I.2) and (I.3),
- (I.1) is in an amount of 25 to 75 weight %, preferably, 30 to 60 weight %, especially 35 to 55 weight %, based on the total weight of (I.1), (I.2) and (I.3),
- (I.2) is in an amount of 15 to 35 weight %, preferably, 18 to 30 weight %, especially 20 to 28 weight %, based on the total weight of (I.1), (I.2) and (I.3),
- (I.3) is in an amount of 15 to 35 weight %, preferably, 18 to 30 weight %, especially 20 to 28 weight %, based on the total weight of (I.1), (I.2) and (I.3),
with the proviso, that the weight of compound (1.1), (I.2) and (I.3) is add up to 100 %.

A special embodiment a composition comprising a mixture of at least two ether compounds of general formulae (I.1), (I.2), and (I.3) comprising each of the compound in said in an amount of 1 - 99 % by weight, with the proviso that weight of compound of formulae (I.1), (I.2), and (I.3) adds up to 100%, wherein the ratio of (I.1):(I.2):(I.3) is in the range of 90:5:5 to 10:45:45, preferably 80:10:10 to 20:40:40, especially 55:22.5:22.5 to 45:27.5:27.5, more especially is 50:25:25.

A further special embodiment a composition comprising a mixture of at least two ester compounds of general formulae (I.1), (I.2), and (I.3) comprising each of the compound in said in an amount of 1 - 99 % by weight, with the proviso that weight of compound of formulae (I.1), (I.2), and (I.3) adds up to 100%, wherein the ratio of (I.1):(I.2):(I.3) is in the range of 90:5:5 to 10:45:45, preferably 80:10:10 to 20:40:40, especially 55:22.5:22.5 to 45:27.5:27.5, more especially is 50:25:25.

A further embodiemnt relates to a method for preparing the compound of formula (I), a stereoisomer thereof or a mixture of stereoisomers as defined above comprising the steps
(i) reacting of 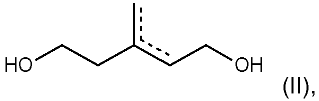 with 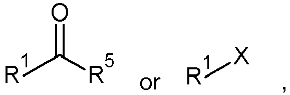 wherein
   one of the symbols 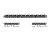 is a single bond and the other is a double bond or a single bond,
   R¹ have one of the meanings given above,
   R² have one of the meanings given above,
   R⁵ is X or -O-C(=O)-R²,
   X represents a leaving group,
   to obtain a raw-product mixture,
(ii) optionally subjecting the obtained raw-product mixture in step (i) to a purification step.

Accordingly, the present invention also relates to a method for preparing the compound of formula (I.a), a stereoisomer thereof or a mixture of stereoisomers thereof or as defined above, comprising
(i) reacting compound (II) or mixtures thereof 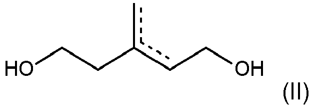 with the alkylation reagent R^{1a}-X and optionally with the alkylation reagent R^{2a}-X, wherein R^{1a} and R^{2a} have one of the meanings given above and X represents a leaving group, selected from halogen, such as Cl, Br, I, and sulfonates, such as tosylate, mesylate, triflate or nonaflate, in the presence of a base to obtain a raw-product mixture, and
(ii) subjecting the raw-product mixture obtained in step (i) to a purification step.

The alkylation reaction in step (i) is performed under conventional alkylation reaction conditions that are well known to the skilled person.

Suitable bases that can be used in the alkylation reaction are as defined above.

For the preparation of the mono-ether compounds of the general formula (I.a), compound (II) or mixtures thereof is typically reacted with sub-equimolar or almost equimolar amounts, e.g. 0.7, 0.8, 0.9, or 0.95 equivalents, of the alkylation reagent R^{1a}-X to give the mono-substituted compound (I.a-OH), 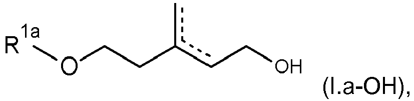
wherein R^{1a} has one of the meanings given above,
which is further subjected to a purification step, as defined above, in order to remove unwanted by-products or impurities, such as residual starting material or the corresponding di-substituted compounds.

The preparation of di-ether compounds of the general formula (I.a), where the radicals R^{1a} and R^{2a} are identical, is typically performed by reacting compound (II) or mixtures thereof with at least two equivalents, e.g. 2.0, 2.1, 2.5 or 3.0 equivalents, of the alkylation reagent R^{1a}-X or R^{2a}-X, respectively, wherein the radicals R^{1a}, R^{2a} and X have one of the meanings given above, and the obtained raw product is subsequently subjected to a purification step, as defined above.

The preparation of di-ether compounds of the general formula (I.a), where the radicals R^{1a} and R^{2a} are different, is generally performed by first reacting compound (II) or mixtures thereof with sub-equimolar or almost equimolar amounts, e.g. 0.7, 0.8, 0.9, or 0.95 equivalents, of the alkylation reagent R¹-X to give the mono-substituted compound (I.-a-OH), which after a purification step is further reacted with the second alkylation reagent R^{2a}-X.

Generally, the starting material compound (II) or mixtures thereof is applied as exo/E/Z/sat isomer mixture, in particular exo/E/Z isomer. Consequently, the compounds (I.a) are typically obtained as a mixture of exo/E/Z/sat isomers, in particular exo/E/Z isomer.

In step (ii) of the method for preparing the compound of formula (I.a), the raw-product mixture obtained in step (i) is subjected to a purification step, in order to remove unwanted by-products or impurities, such as residual mono-substituted compound (I.a-OH) and, if desired, to separate the exo/E/Z isomers.

Generally the purification in step (ii) can be performed by using common purification methods, such as crystallization, distillation or chromatographic methods, e.g. column chromatography or high performance liquid chromatography. In case where only minor amounts of impurities and by-products are present in the raw-product mixture the purification step can also be performed via a simple extractive workup. If desired the cis/trans isomers can be separated by fractional crystallization, column chromatography or high performance liquid chromatography.

Accordingly, the present invention also relates to a method for preparing the compound of formula (I.b), a stereoisomer thereof, or a mixture of stereoisomers, as defined above, comprising
(i) reacting compound (II) or mixtures thereof 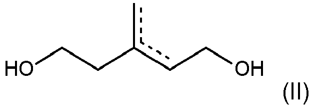 with the 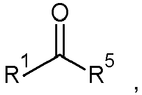 wherein R⁵ is X or -O-C(=O)-R², X represents a leaving group, selected from halogen, such as Cl, Br, or I, R¹ and R² have one of the meanings as defined above, in particular,
   reacting compound (II) or mixtures thereof with carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture, and
(ii) subjecting the raw-product mixture obtained in step (i) to a purification step.

The individual reaction conditions for the preparation of the ester compounds of the general formula (I.b) in step i) are well known to the skilled person.

For the preparation of the mono-ether compounds of the general formula (I.b), compound (II) or mixtures thereof is typically reacted with sub-equimolar or almost equimolar amounts, e.g. 0.7, 0.8, 0.9, or 0.95 equivalents, of the alkylation reagent R^{1b}-X to give the mono-substituted compound (I.b-OH), 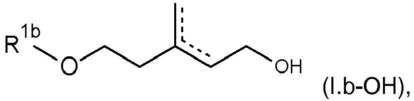
wherein R^{1b} has one of the meanings given above,
which is further subjected to a purification step, as defined above, in order to remove unwanted by-products or impurities, such as residual starting material or the corresponding di-substituted compounds.

The preparation of di-ester compounds of the general formula (I.b), where the radicals R^{1b} and R^{2b} are identical, is typically performed by reacting compound (II) or mixtures thereof with at least two equivalents, e.g. 2.0, 2.1, 2.5 or 3.0 equivalents, of a carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture, and the obtained raw product is subsequently subjected to a purification step, as defined above.

The preparation of di-ester compounds of the general formula (I.b), where the radicals R^{1b} and R^{2b} are different, is generally performed by first reacting compound (II) or mixtures thereof with sub-equimolar or almost equimolar amounts, e.g. 0.7, 0.8, 0.9, or 0.95 equivalents, carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture to give the mono-substituted compound (I.-b-OH), which after a purification step is further reacted with the second carboxylic acid R³-COOH or an anhydride thereof, or with the acid halogenide R³-(C=O)X' in the presence of an organic base and optionally with the carboxylic acid R⁴-COOH or an anhydride thereof, or with the acid halogenide R⁴-(C=O)X', wherein R³ and R⁴ have one of the meanings given above and X' represents a halogen, such as Cl, Br or I, to obtain a raw-product mixture.

Generally, the starting material compound (II) or mixtures thereof is applied as exo/E/Z isomer mixture. Consequently, the compounds I.b are typically obtained as a mixture of exo/E/Z isomers.

In step (ii) of the method for preparing the compound of formula (I.b), the raw-product mixture obtained in step (i) is subjected to a purification step, in order to remove unwanted by-products or impurities, such as residual mono-substituted compound (I.b-OH) and, if desired, to separate the exo/E/Z isomers.

Generally the purification in step (ii) can be performed by using common purification methods, such as crystallization, distillation or chromatographic methods, e.g. column chromatography or high performance liquid chromatography. In case where only minor amounts of impurities and by-products are present in the raw-product mixture the purification step can also be performed via a simple extractive workup. If desired the exo/Z/E isomers can be separated by fractional crystallization, column chromatography or high performance liquid chromatography.

The present invention is now illustrated in further detail by the following examples, without imposing any limitation thereto.

### EXAMPLES

### Abbreviations:

DMAP: 4-(dimethylamino)-pyridine
MTBE: 2-methoxy-2-methylpropan
RT: room temperature
THF: tetrahydrofuran
mixture of 3-methylenepentane-1,5-diol, (E)-3-methylpent-2-ene-1,5-diol and (Z)-3-methylpent-2-ene-1,5-diol (available as a side stream from the citral production)

### Analytics:

The purity of the products was determined by Gas Chromatography area-%:
GC-system: Agilent 7890 B;
GC-Column: DB-WAX (30 m (Length), 0.32 mm (ID), 0,25 micrometer (film));
Temperature program: 85°C to 230°C at 5°/min

**Odor description:**

| Compound | Odor description |
|---|---|
| 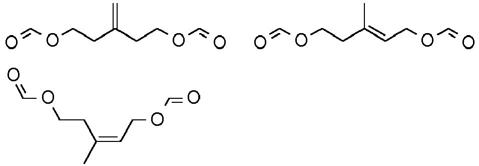 | Technical, mushroom |
| ratio exo:E:Z 50:25:25 | |
| 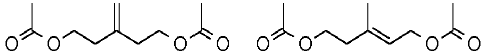 | Sweet, powdery, terpenic, fruity |
| 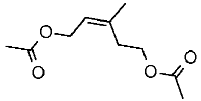 | |
| ratio exo:E:Z 50:25:25 | |
| 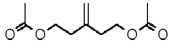 | Fruity, sweet, rubber, musty, dusty |
| 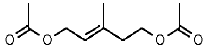 | Fruity, woody, sweet |
| 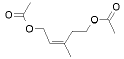 | Weak |
| 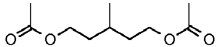 | Sweet, fruity |
| 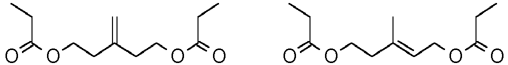 | Sweet, red berries |
| 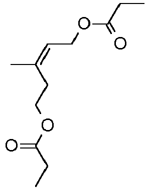 | |
| ratio exo:E:Z 50:25:25 | |
| 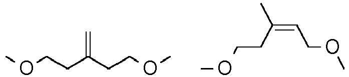 | Herbal, algae, green, watery, fruity |
| 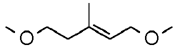 | |
| ratio exo:E:Z 50:25:25 | |
| 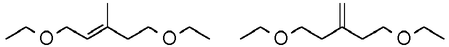 | Green, herbal, raw potato, green banana |
| 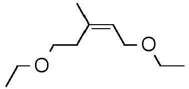 | |
| ratio exo:E:Z 50:25:25 | |
| 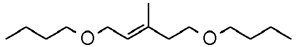 | Cedar, citrus, warm, herbal |
| 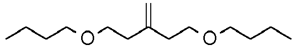 | |
| 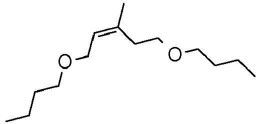 | |
| 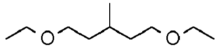 | Green, banana, apple (ripe) |

### Example 1:

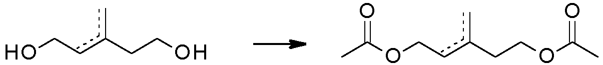

| - **Compound** | **- MW** | **- Mass/Volume** | **- Moles** |
|---|---|---|---|
| mixture of 3-methylenepentane-1,5-diol, (E)-3-methylpent-2-ene-1,5-diol and (Z)-3-methylpent-2-ene-1,5-diol, molar ratio exo:E:Z is 50:25:25 | - 116.16 | - 20g | - 0.172 |
| - acetic anhydride | - 102.09 | - 39.5g | - 0.387 |
| - N,N'-dimethylaminopyridine | - 122.17 | - 0.631g | - 0.005 |
| - Tetrahydrofuran | - | - 150mL | - |

DMAP was added to the THF solution of the diol at RT. The mixture was set to reflux (53°C) and acetic anhydride (2.25 eq) was slowly added at this temperature. After 1 h, 99% diol conversion was observed by GC. The reaction was cooled down to room temperature and slowly quenched with 100 mL of water. 100 mL of ethyl acetate were then added. The organic phase was separated and washed with NaHCO₃ and brine solution. The organic extracts were combined and dried with sodium sulfate. After evaporating the solvent at reduced pressure, 37.1 g of crude product that contained ca. 90% of the diacetate according to the GC (area %) were obtained. The product was purified by distillation. The mixture of the corresponding di-acetates was obtained with a purity of 93.2% (GC area %). The NMR confirmed that the composition of the mixture was 50% of the 3-Methylene di-acetate and 50% of a 1:1 E/Z mixture of the 2-pentene derivatives.

| | | |
|---|---|---|
| 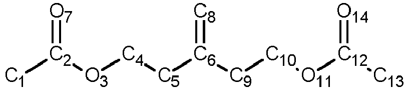 | Atom number | ¹³C shifts measured (multiplicity) |
| | C1/ C13 | 20.95 (q) |
| | C2/ C12 | 170.01 (s) |
| | C4/ C10 | 62.59 (t) |
| | C5/ C9 | 35.01 (t) |
| | C6 | 141.54 (s) |
| | C8 | 113.53 (t) |

| | | |
|---|---|---|
| 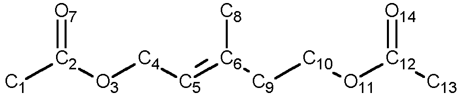 | Atom number | ¹³C shifts measured (multiplicity) |
| | C1/C13 | 21.02/ 20.95 (q) |
| | C2/ C12 | 170.99 (s) |
| | C4 | 62.28 (t) |
| | C5 | 120.91 (d) |
| | C6 | 137.90 (s) |
| | C8 | 16.51 (q) |
| | C9 | 38.38 (t) |
| | C10 | 61.06 (t) |

| | | |
|---|---|---|
| 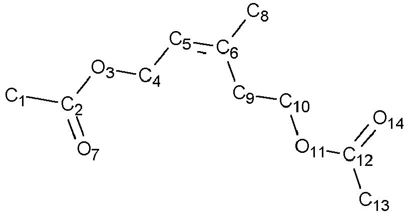 | Atom number | ¹³C shifts measured (multiplicity) |
| | C1/ C13 | 21.02/ 20.95 (q) |
| | C2/ C12 | 171.01/ 170.99 (s) |
| | C4 | 62.36 (t) |
| | C5 | 121.86 (d) |
| | C6 | 138.07 (s) |
| | C8 | 23.63 (q) |
| | C9 | 31.35 (t) |
| | C10 | 60.85 (t) |

### Example 2:

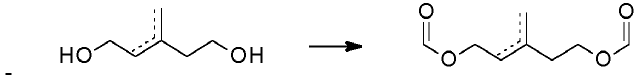

| - **Compound** | **- MW** | **- Mass/Volume** | **- Moles** |
|---|---|---|---|
| mixture of 3-methylenepentane-1,5-diol, (E)-3-methylpent-2-ene-1,5-diol and (Z)-3-methylpent-2-ene-1,5-diol, | - 116.16 | - 20g | - 0.172 |
| | | | |
| molar ratio exo:E:Z is 50:25:25 | | | |
| - formic acid | - 46.03 | - 55.48g | - 1.205 |
| - acetic anhydride | - 102.09 | - 105.47g | - 1.033 |
| - N,N'-dimethylaminopyridine | - 122.17 | - 0.421g | - 0,02 |
| - tetrahydrofuran | - | - 100mL | - |

At 0°C formic acid was slowly added to acetic anhydride. The mixture was stirred 1.5 h at 55°C. The mixture was then cooled down to 0°C and 50 mL THF were added, at this temperature a solution of the alcohol in 50 mL THF was slowly added. After the addition DMAP was added to the mixture. The reaction was stirred at room temperature for 4h and full conversion was observed by GC. 100 mL toluene were added to the mixture and the organic phase was washed 3 times with 30 mL of water. The organic phase was dried with sodium sulfate and the solvent was removed in the rotatory evaporator to give 19.6 g of crude product that contained ca 90% of the diformate according to the GC (area %). The product was purified by distillation. The mixture of the corresponding di-formates was obtained with a purity of 98.1% (GC area %). The NMR confirmed that the composition of the mixture was 50% of the 3-Methylene di-formiate and 50% of a 1:1 E/Z mixture of the 2-pentene derivatives.

| | | |
|---|---|---|
| 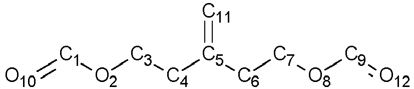 | Atom number | ¹³C shifts measured (multiplicity) |
| | C1/ C9 | 160.95 (d) |
| | C3/ C7 | 61.91 (t) |
| | C4/ C6 | 34.81 (t) |
| | C5 | 140.87 (s) |
| | C11 | 114.04 (t) |

| | | |
|---|---|---|
| | Atom number | ¹³C shifts measured (multiplicity) |
| | C1/ C9 | 160.97/ 160.89 (s) |
| 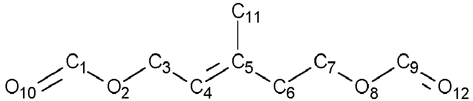 | C3 | 60.33/ 60.10 (t) |
| | C4 | 121.57/ 121.00 (s) |
| E:Z Mixture 1:1 | C5 | 138.45 (s) |
| | C6 | 38.19/ 31.21 (t) |
| | C7 | 61.64/ 61.59 (d) |
| | C11 | 23.44/ 16.43 (q) |

### Example 3:

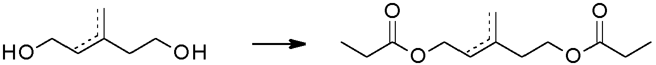

| - **Compound** | **- MW** | **- Mass/Volume** | - **Moles** |
|---|---|---|---|
| - mixture of 3-methylenepentane-1,5-diol, (E)-3-methylpent-2-ene-1,5-diol and (Z)-3-methylpent-2-ene-1,5-diol, - molar ratio exo:E:Z is 50:25:25 | - 144.21 | - 20g | - 0.172 |
| - Propionyl chloride | - 92.52 | - 39.83g | - 0.430 |
| - N,N'-dimethylaminopyridine | - 122.17 | - 0.300g | - 0.01 |
| - Triethylamine | - 101.19 | - 43.56g | - 0.430 |
| - Hexan | - | - 100mL | - |

A solution of the alcohol, triethylamine and DMAP in hexane was cooled down to 0°C. At this temperature, propionyl chloride was slowly added. The reaction was stirred at room temperature for 1h, 50 mL of hexane were added and the reaction was stirred 4 h at reflux (67°C). After this time full conversion was observed by GC. The reaction was cooled down to room temperature and slowly quenched with 100 mL of water and 100 mL of ethyl acetate were added, the organic phase was washed with NaHCO₃ and brine solution. The organic extracts were combined and dried with sodium sulfate. After evaporating the solvent at reduced pressure, 38.4 g of crude product that contained ca 90% of the dipropionate according to the GC (area %). The product was purified by distillation. The mixture of the corresponding di-propionates was obtained with a purity of 97.8% (GC area %). The NMR confirmed that the composition of the mixture was 50% of the 3-Methylene di-propionate and 50% of a 1:1 E/Z mixture of the 2-pentene derivatives.

| | | |
|---|---|---|
| 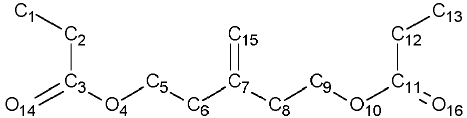 | Atom number | ¹³C shifts measured (multiplicity) |
| | C1/ C13 | 9.13 (q) |
| | C2/ C12 | 27.56 (t) |
| | C3/ C11 | 174.41 (s) |
| | C5/ C9 | 62.43 (t) |
| | C6/ C8 | 35.07 (t) |
| | C7 | 141.61 (s) |
| | C15 | 113.54 (t) |

| | Atom number | ¹³C shifts measured (multiplicity) |
|---|---|---|
| 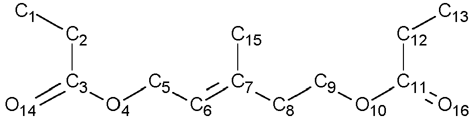 | C1/ C13 | 9.14/ 9.08 (q) |
| | C2/ C12 | 27.56 (t) |
| | C3/ C11 | 174.41 (s) |
| | C5/ C9 | 62.20/ 62.19/ 60.95/ 60.77(t) |
| E:Z Mixture 1:1 | C6 | 121.93/ 121.04 (d) |
| | C7 | 138.01/ 137.83 (s) |
| | C8 | 38.46/ 31.40 (t) |
| | C15 | 23.66/ 16.50 (q) |

### Example 4:

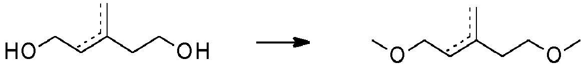

| - **Compound** | **- MW** | **- Mass/Volume** | - **Moles** |
|---|---|---|---|
| - mixture of 3-methylenepentane-1,5-diol, (E)-3-methylpent-2-ene-1,5-diol and (Z)-3-methylpent-2-ene-1,5-diol, - molar ratio exo:E:Z is 50:25:25 | - 116.16 | - 15g | - 0.129 |
| - Methyl iodide | - 141.94 | - 42.16g | - 0.297 |
| - Sodium hydride | - 23.99 | - 7.12g | - 0.297 |
| - Tetrahydrofuran | - | - 105mL | - |

To a dispersion of sodium hydride (2.3 eq) in 75 mL of THF a solution of the citral s.s. diol mixture in 30 mL of THF was slowly added at 0°C. The mixture was stirred 30 min at 0°C. After this time 2.3 eq of methyl iodide were slowly added at RT. After the addition, the mixture was stirred at 40°C for 4 h. After this time the reaction mixture was cooled down to 0°C and the addition of 0.75 eq of NaH followed by 0.75 eq of methyl iodide was repeated. The mixture was then stirred for 20h at 40°C. At this point, the reaction was slowly quenched with 50 mL of water. The organic phase was extracted with 3 times 50 mL MTBE. The organic extracts were combined and washed with 50 mL of NH3 solution and with 50mL brine solution. The organic extracts were combined and dried with sodium sulfate. After evaporating the solvent at reduced pressure 20 g of crude product were obtained with 98% of the diether according to the GC (area %). The product was purified by distillation. The mixture of the corresponding di-methyl ethers was obtained with a purity > 99% (GC area %). The NMR confirmed that the composition of the mixture was 50% of the exo-di-Methyl ether and 50% of a 1:1 E/Z mixture of the 2-pentene derivative.

| | | |
|---|---|---|
| 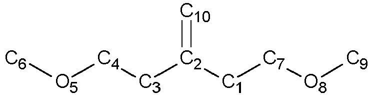 | Atom number | ¹³C shifts measured (multiplicity) |
| | C1/ C3 | 36.11 (t) |
| | C4/ C7 | 71.08 (t) |
| | C6/ C9 | 58.56 (q) |
| | C2 | 143.52 (s) |
| | C10 | 112.07 (t) |

| | | |
|---|---|---|
| 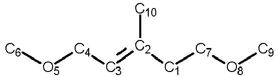 | Atom number | ¹³C shifts measured (multiplicity) |
| | C1 | 39.40/ 32.49 (t) |
| | C2 | 137.27/ 137.23 (s) |
| E:Z Mixture 1:1 | C3 | 123.47/ 122.42 (d) |
| | C4/C7 | 71.14/ 71.06/ 68.83/ 68.65 (t) |
| | C6/C9 | 57.91/ 57.87 (q) |
| | C10 | 23.82/ 16.61 (q) |

### Example 5:

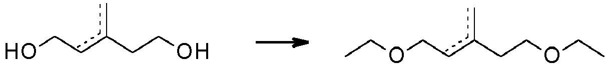

| - **Compound** | **- MW** | **- Mass/Volume** | - **Moles** |
|---|---|---|---|
| - mixture of 3-methylenepentane-1,5-diol, (E)-3-methylpent-2-ene-1,5-diol and (Z)-3-methylpent-2-ene-1,5-diol, - molar ratio exo:E:Z is 50:25:25 | - 116.16 | - 15g | - 0.129 |
| - Ethyl iodide | - 155.97 | - 46.32g | - 0.297 |
| - Sodium hydride | - 23.99 | - 7.12g | - 0.297 |
| - Tetrahydrofuran | - | - 105mL | - |

To a dispersion of sodium hydride (2.3 eq) in 75 mL of THF a solution of the citral s.s. diol mixture in 30 mL of THF was slowly added at 0°C. The mixture was stirred 30 min at 0°C. After this time 2.3 eq of ethyl iodide were slowly added at RT. After the addition, the mixture was stirred at 40°C for 4 h. After this time the reaction mixture was cooled down to 0°C and the addition of 0.75 eq of NaH followed by 0.75 eq of ethyl iodide was repeated. The mixture was then stirred for 20 h at 40°C. At this point, the reaction was slowly quenched with 50 mL of water. The organic phase was extracted with 3 times 50 mL MTBE. The organic extracts were combined and washed with 50mL of NH₃ solution and with 50mL brine solution. The organic extracts were combined and dried with sodium sulfate. After evaporating the solvent at reduced pressure 24.6 g of crude product were obtained with 93% of the diether according to the GC (area %). The product was purified by distillation. The mixture of the corresponding di-ethyl ethers was obtained with a purity > 96% (GC area %). The NMR confirmed that the composition of the mixture was 50% of the exo-diethyl ether and 50% of a 1:1 E/Z mixture of the 2-pentene derivative.

| | | |
|---|---|---|
| 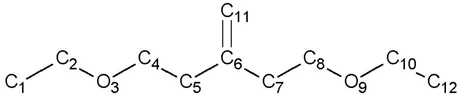 | Atom number | ¹³C shifts measured (multiplicity) |
| | C1/ C12 | 15.20 (q) |
| | C2/ C10 | 66.16 (t) |
| | C4/ C8 | 69.19 (t) |
| | C5/ C7 | 36.46 (t) |
| | C6 | 143.72 (s) |
| | C11 | 111.84 (t) |

| | | |
|---|---|---|
| 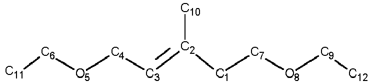 | Atom number | ¹³C shifts measured (multiplicity) |
| | C1 | 39.65/ 32.80(t) |
| | C2 | 136.87/ 136.79 (s) |
| | C3 | 123.81/ 122.79 (d) |
| E:Z Mixture 1:1 | C4/C7 | 69.53/ 69.30/ 67.01/ 66.85 (t) |
| | C6/C9 | 65.52 (t) |
| | C10 | 24.01/ 16.74 (q) |
| | C11/C12 | 15.24 (q) |

### Example 6:

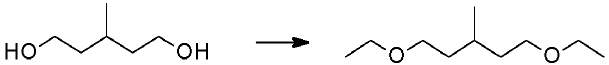

| - **Compound** | **- MW** | **- Mass/Volume** | - **Moles** |
|---|---|---|---|
| - 3-Methyl-1,5-pentandiol | - 118.17 | - 10g | - 0.085 |
| - Ethyl iodide | - 155.97 | - 30.35g | - 0.195 |
| - Sodium hydride | - 23.99 | - 4.67g | - 0.195 |
| - Tetrahydrofuran | - | - 105mL | - |

To a dispersion of sodium hydride (2.3 eq) in 75 mL of THF a solution of 3-methyl-1,5-pentandiol in 30 mL of THF was slowly added at 0°C. The mixture was stirred 30 min at 0°C. After this time 2.3 eq of ethyl iodide were slowly added at RT. After the addition, the mixture was stirred at 40°C for 4 h. After this time the reaction mixture was cooled down to 0°C and the addition of 0.75 eq of NaH followed by 0.75 eq of ethyl iodide was repeated. The mixture was then stirred for 20 h at 40°C. At this point, the reaction was slowly quenched with 50 mL of water. The organic phase was extracted with 3 times 50 mL MTBE. The organic extracts were combined and washed with 50 mL of NH₃ solution and with 50 mL brine solution. The organic extracts were combined and dried with sodium sulfate. After evaporating the solvent at reduced pressure 15.7 g of crude product were obtained with 92% of the diether according to the GC (area %). The product was purified by distillation. The corresponding di-ethyl ether was obtained with a purity > 97% (GC area %).

| | | |
|---|---|---|
| 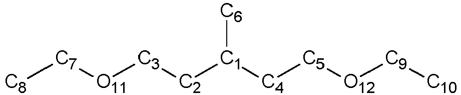 | Atom number | ¹³C shifts measured (multiplicity) |
| | C1 | 27.21 (d) |
| | C2/ C4 | 36.89 (t) |
| | C3/ C5 | 68.82 (t) |
| | C6 | 19.75 (q) |
| | C7/ C9 | 66.11 (t) |
| | C8/ C10 | 15.27 (q) |

### Example 7:

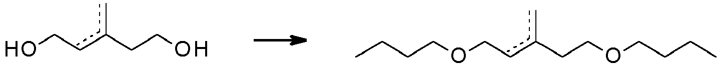

| - **Compound** | **- MW** | **- Mass/Volume** | **- Moles** |
|---|---|---|---|
| - mixture of 3-methylenepentane-1,5-diol, (E)-3-methylpent-2-ene- | - 116.16 | - 15g | - 0.129 |
| 1,5-diol and (Z)-3-methylpent-2-ene-1,5-diol, - molar ratio exo:E:Z is 50:25:25 | | | |
| - 1-lodo-butan | - 184.02 | - 56.65g | - 0.297 |
| - Sodium hydride | - 23.99 | - 7.12g | - 0.297 |
| - Tetrahydrofuran | - | - 105mL | - |

To a dispersion of sodium hydride (2.3 eq) in 75 mL of THF a solution of the citral s.s. diol mixture in 30 mL of THF was slowly added at 0°C. The mixture was stirred 30 min at 0°C. After this time 2.3 eq of ethyl iodide were slowly added at RT. After the addition, the mixture was stirred at 50°C for 4 h. After this time the reaction mixture was cooled down to 0°C and the addition of 0.75 eq of NaH followed by 0.75 eq of 1-iodo-butan was repeated. The mixture was then stirred for 90 h at 50°C. At this point, the reaction was slowly quenched with 50 mL of water. The organic phase was extracted with 3 times 50 mL MTBE. The organic extracts were combined and washed with 50 mL of NH₃ solution and with 50 mL brine solution. The organic extracts were combined and dried with sodium sulfate. After evaporating the solvent at reduced pressure 26.2 g of crude product were obtained with 37% of the di-n-butyl ether according to the GC (area %), being the rest the mono-ether derivatives. The product was purified by column chromatography. The mixture of the corresponding di-ethyl ethers was obtained with a purity > 95% (GC area %). The NMR confirmed that the composition of the mixture was 50% of the exo-di-n-butyl ether and 50% of a 1:1 E/Z mixture of the 2-pentene derivative.

| | | |
|---|---|---|
| 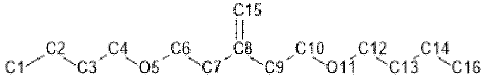 | Atom number | ¹³C shifts measured (multiplicity) |
| | C1/ C16 | 13.96 (q) |
| | C2/ C14 | 19.44 (t) |
| | C3/ C13 | 31.94 (t) |
| | C4/ C12 | 70.69 (t) |
| | C6/ C10 | 69.50 (t) |
| | C7/ C9 | 36.49 (t) |
| | C8 | 143.92 (s) |
| | C15 | 111.80 (t) |

| | | |
|---|---|---|
| 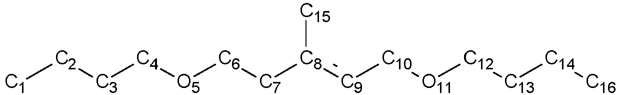 | Atom number | ¹³C shifts measured (multiplicity) |
| | C1/ C16 | 13.96 (q) |
| E:Z Mixture 1:1 | C2/ C14 | 19.45/ 19.41 (t) |
| | C3/ C13 | 32.75/ 31.95 (t) |
| | C4/ C12 | 70.14/ 70.05 (t) |
| | C6 | 69.40/ 69.33 (t) |
| | C7 | 39.58 (t) |
| | C8 | 136.98/ 136.89 (s) |
| | C9 | 123.72/ 122.74 (d) |
| | C10 | 67.17/ 67.02 (t) |
| | C15 | 16.77/ 24.03 (q) |

The compound of example 1 can be used as the essentially pure exo-isomer or as the essentially pure E-isomer or as the essentially pure Z-isomer, or as the essentially pure sat-isomer or as a mixture of the exo isomer, E-isomer, Z-Isomer and sat isomer, e.g. a 49:25:25:1 mixture, or as a mixture of the exo isomer, E-isomer, Z-Isomer e.g. a 50:25:25 mixture.

The compound of example 2 can be used as the essentially pure exo-isomer or as the essentially pure E-isomer or as the essentially pure Z-isomer, or as the essentially pure sat-isomer or as a mixture of the exo isomer, E-isomer, Z-Isomer and sat isomer, e.g. a 49:25:25:1 mixture, or as a mixture of the exo isomer, E-isomer, Z-Isomer e.g. a 50:25:25 mixture.

The compound of example 3 can be used as the essentially pure exo-isomer or as the essentially pure E-isomer or as the essentially pure Z-isomer, or as the essentially pure sat-isomer or as a mixture of the exo isomer, E-isomer, Z-Isomer and sat isomer, e.g. a 25:25:25:25 mixture, or as a mixture of the exo isomer, E-isomer, Z-Isomer e.g. a 50:25:25 mixture.

The compound of example 4 can be used as the essentially pure exo-isomer or as the essentially pure E-isomer or as the essentially pure Z-isomer, or as the essentially pure sat-isomer or as a mixture of the exo isomer, E-isomer, Z-Isomer and sat isomer, e.g. a 49:25:25:1 mixture, or as a mixture of the exo isomer, E-isomer, Z-Isomer e.g. a 50:25:25 mixture.

The compound of example 5 can be used as the essentially pure exo-isomer or as the essentially pure E-isomer or as the essentially pure Z-isomer, or as the essentially pure sat-isomer or as a mixture of the exo isomer, E-isomer, Z-Isomer and sat isomer, e.g. a 49:25:25:1 mixture, or as a mixture of the exo isomer, E-isomer, Z-Isomer e.g. a 50:25:25 mixture.

The compound of example 6 can be used as the essentially pure exo-isomer or as the essentially pure E-isomer or as the essentially pure Z-isomer, or as the essentially pure sat-isomer or as a mixture of the exo isome, E-isomer, Z-Isomer and sat isomer, e.g. a 49:25:25:1 mixture, or as a mixture of the exo isome, E-isomer, Z-Isomer e.g. a 50:25:25 mixture.

The compound of example 7 can be used as the essentially pure exo-isomer or as the essentially pure E-isomer or as the essentially pure Z-isomer, or as the essentially pure sat-isomer or as a mixture of the exo isome, E-isomer, Z-Isomer and sat isomer, e.g. a 49:25:25:1 mixture, or as a mixture of the exo isome, E-isomer, Z-Isomer e.g. a 50:25:25 mixture.

The following table 3 and 4 display typical perfume oil compositions (components are given as part by weight):

**Table 3: Perfume oil compositions 1A and 1B**

| | **1A** | **1B** |
|---|---|---|
| Benzoe Siam 20% | 711 | 711 |
| Rosewood Oil brasilian | 85 | 85 |
| Copaivabalm rect. | 9 | 9 |
| Linalyl-benzoate | 31 | 31 |
| 3-cis-Hexenyl-salicylate | 21 | 21 |
| Geranyl-acetate | 47 | 47 |
| Ethyl-benzoate | 12 | 12 |
| Cinnamyl-acetate | 2 | 2 |
| Benzyl-acetate | 71 | 71 |
| Methyl-anthranilate 10% | 5 | 5 |
| Bayoil St. Thomas 10% | 5 | 5 |
| Compound of example 1 | 0 | 20 |
| | 1000 | 1020 |

**Table 4: Perfume oil compositions 2A and 2B**

| | **2A** | **2B** |
|---|---|---|
| Ethyl Caproate | 1 | 1 |
| Ethyl Acetate | 1 | 1 |
| Iso Amyl Butyrate | 1 | 1 |
| Maltol or Veltol | 1 | 1 |
| Geranyl Butyrate | 2 | 2 |
| Ethyl Vanilline 10% DPG | 2 | 2 |
| Cis 3 Hexenyl Acetate | 3 | 3 |
| Allyl Caproate | 3 | 3 |
| Verdural B 10% DPG | 3 | 3 |
| Oxyphenylon | 3 | 3 |
| Hexyl Butyrate | 4 | 4 |
| Ethyl Decadienoate 10% DPG | 4 | 4 |
| DM.B.C. Butyrate | 4 | 4 |
| Ethyl Maltol or Veltol Plus | 4 | 4 |
| Cyclaprop | 5 | 5 |
| Iso Amyl Acetate | 5 | 5 |
| Cis 3 Hexenol 10% DPG | 6 | 6 |
| D.M.B.C. Acetate | 7 | 7 |
| Aldehyde C 16 100% | 8 | 8 |
| Geranyl Propionate | 8 | 8 |
| Ethyl 2 Methyl Butyrate | 8 | 8 |
| Decalactone Gamma | 10 | 10 |
| Orange Bresil Oil | 10 | 10 |
| Ethyl Aceto Acetate | 10 | 10 |
| Linalool | 15 | 15 |
| Benzyl Acetate | 15 | 15 |
| Aldehyde C 14 100% | 20 | 20 |
| Citronellol | 25 | 25 |
| Linalyl Acetate | 30 | 30 |
| Geranyl Acetate | 35 | 35 |
| Vertenex | 45 | 45 |
| Citronellyl Acetate | 50 | 50 |
| Verdox | 54 | 54 |
| Galaxolide 50 DEP | 100 | 100 |
| Hexyl Acetate | 190 | 190 |
| Mono Propylene Glycol | 300 | 300 |
| Compound of example 1 | 0 | 200 |
| | 1000 | 1200 |

Perfume oil composition 3 corresponds to perfume oil composition 1B, where the compound of example 1 is replaced by the same amount of the compound of example 2.

Perfume oil composition 4 corresponds to perfume oil composition 1B, where the compound of example 1 is replaced by the same amount of the compound of example 3.

Perfume oil composition 5 corresponds to perfume oil composition 1B, where the compound of example 1 is replaced by the same amount of the compound of example 4.

Perfume oil composition 6 corresponds to perfume oil composition 1B, where the compound of example 1 is replaced by the same amount of the compound of example 5.

Perfume oil composition 7 corresponds to perfume oil composition 1B, where the compound of example 1 is replaced by the same amount of the compound of example 6.

Perfume oil composition 8 corresponds to perfume oil composition 1B, where the compound of example 1 is replaced by the same amount of the compound of example 7.

Perfume oil composition 9 corresponds to perfume oil composition 2B, where the compound of example 1 is replaced by the same amount of the compound of example 2.

Perfume oil composition 10 corresponds to perfume oil composition 2B, where the compound of example 1 is replaced by the same amount of the compound of example 3.

Perfume oil composition 11 corresponds to perfume oil composition 2B, where the compound of example 1 is replaced by the same amount of the compound of example 4.

Perfume oil composition 12 corresponds to perfume oil composition 2B, where the compound of example 1 is replaced by the same amount of the compound of example 5.

Perfume oil composition 13 corresponds to perfume oil composition 2B, where the compound of example 1 is replaced by the same amount of the compound of example 6.

Perfume oil composition 14 corresponds to perfume oil composition 2B, where the compound of example 1 is replaced by the same amount of the compound of example 7.

The compounds of formula (I), in particular the compounds of examples 1, 2, 3, 4, 5, 6 and 7 either as the essentially pure exo, E-, Z- or sat-isomers or as a mixture thereof, may be used in a broad range of fragrance applications, e.g. in any field of fine and functional perfumery, such as perfumes, household products, laundry products, body care products and cosmetics. The compounds of formula (I), in particular the compounds of examples 1, 2, 3, 4, 5, 6 and 7, either as the essentially pure exo, E-, Z- or sat-isomers or as a mixture thereof, can be employed in widely varying amounts, depending upon the specific application and on the nature and quantity of other odorant ingredients. The proportion is typically from 0.001 to 20 weight per cent of the application. In one embodiment, the compounds of formula (I), in particular the compounds of examples 1, 2, 3, 4, 5, 6 and 7, either as the essentially pure exo, E-, Z- or sat-isomers or as a mixture thereof, are employed in a fabric softener in an amount of from 0.001 to 0.05 weight per cent. In another embodiment, the compounds of formula (I), in particular the compounds of examples 1, 2, 3, 4, 5, 6 and 7, either as the essentially pure exo, E-, Z- or sat-isomers isomers or as a mixture thereof, are used in fine perfumery in amounts of from 0.1 to 20 weight per cent, more preferably between 0.1 and 5 weight per cent. However, these values are given only by way of example, since the experienced perfumer may also achieve effects or may create novel accords with lower or higher concentrations.

The compounds of formula (I), in particular the compounds of examples 1, 2, 3, 4, 5, 6 and 7, either as the essentially pure exo, E-, Z- or sat-isomers or as a mixture thereof, may be employed into the fragrance application simply by directly mixing the fragrance composition with the fragrance application, or they may, in an earlier step be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of a compound of formula (I), in particular a compound of examples 1, 2, 3, 4, 5, 6 and 7, either as the essentially pure exo, E-, Z- or sat-isomers or as a mixture thereof, as a fragrance ingredient, either by directly admixing the compound of formula (I) to the application or by admixing a fragrance composition comprising a compound of the formula (I), in particular a compound of examples 1, 2, 3, 4, 5, 6 and 7, which may then be mixed to a fragrance application, using conventional techniques and methods.

As used herein, "fragrance application" means any product, such as fine perfumery, e.g. perfume and Eau de Toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odourant. This list of products is given by way of illustration and is not to be regarded as being in any way limiting.

The compounds of formula (I), in particular the compounds of examples 1, 2, 3, 4, 5, 6 and 7, either as the essentially pure exo, E-, Z- or sat-isomers or as a mixture thereof, may be used as part of the perfume in the above mentioned applications. The compounds of formula (I), in particular the compounds of examples 1, 2, 3, 4, 5, 6 and 7, either as the essentially pure exo, E-, Z- or sat-isomers or as a mixture thereof, may be used alone or as part of a perfume. The term "perfume" is used synonymously to "perfume oil" or "perfume (oil) composition".

In the following tables all amounts are given in weight-% (% b.w.). Conc. means concentration.

**Table 5: Deo pump spray 1**

| **Deo Pump Spray; PIT** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate | 3,9 |
| EUMULGlN® B 2 | Ceteareth-20 | 1.6 |
| CETIOL® OE | Dicaprylyl Ether | 5 |
| CETIOL® PGL | Hexyldecanol (and) Hexyldecyl Laurate | 2 |
| Irgasan DP 300 | Triclosan | 0.25 |
| HYDAGEN® DEO | Triethyl Citrate (and) BHT | 2.5 |
| Water, de ionized | Aqua | 19 |
| Eumulgin® HRE 60 | PEG-60 Hydrogenated Castor Oil | 1.5 |
| Perfume oil composition 1B | Perfume | 0.5 |
| Water, de ionized | Aqua | ad 100 |
| Preservative | | q.s. |
| Viscosity mPas | < 100 | |

Deo pump spray 2 corresponds to deo pump spray 1, where the perfume oil composition 1B is replaced by the same amount of the perfume oil composition 2B.
Deo pump spray 3 corresponds to deo pump spray 1, where the perfume oil composition 1B is replaced by the same amount of perfume oil composition 3.
Deo pump spray 4 corresponds to deo pump spray 1, where the perfume oil composition 1B is replaced by the same amount of the perfume oil composition 4.
Deo pump spray 5 corresponds to deo pump spray 1, where the perfume oil composition 1B is replaced by the same amount of the perfume oil composition 5.
Deo pump spray 6 corresponds to deo pump spray 1, where the perfume oil composition 1B is replaced by the same amount of the perfume oil composition 6.
Deo pump spray 7 corresponds to deo pump spray 1, where the perfume oil composition 1B is replaced by the same amount of the perfume oil composition 7.
Deo pump spray 8 corresponds to deo pump spray 1, where the perfume oil composition 1B is replaced by the same amount of the perfume oil composition 8.
Deo pump spray 9 corresponds to deo pump spray 1, where the perfume oil composition 1B is replaced by the same amount of the perfume oil composition 9.
Deo pump spray 10 corresponds to deo pump spray 1, where the perfume oil composition 1B is replaced by the same amount of the perfume oil composition 10.
Deo pump spray 11 corresponds to deo pump spray 1, where the perfume oil composition 1B is replaced by the same amount of the perfume oil composition 11.
Deo pump spray 12 corresponds to deo pump spray 1, where the perfume oil composition 1B is replaced by the same amount of the perfume oil composition 12.
Deo pump spray 13 corresponds to deo pump spray 1, where the perfume oil composition 1B is replaced by the same amount of the perfume oil composition 13.
Deo pump spray 14 corresponds to deo pump spray 1, where the perfume oil composition 1B is replaced by the same amount of the perfume oil composition 14.

**Table 6: Deo pump spray 15**

| **Deo Pump Spray** | | |
|---|---|---|
| **Component** | **INCI** | **amount %** |
| Eumulgin® HRE 60 | PEG-60 Hydrogenated Castor Oil | 2 |
| Farnesol | Deo ingredient | 0.2 |
| HYDAGEN® DCMF | Chitosan | 0.1 |
| glycolic acid (Fa. Merck) | glycolic acid | 0.04 |
| Water, de ionized | | ad 100 |
| Perfume oil composition 1B | Perfume | 0.5 |
| pH value | 4 | |

Deo pump spray 16 corresponds to deo pump spray 15, where the perfume oil composition 1 B is replaced by the same amount of the perfume oil composition 2B.
Deo pump spray 17 corresponds to deo pump spray 15, where the perfume oil composition 1 B is replaced by the same amount of the perfume oil composition 3.
Deo pump spray 18 corresponds to deo pump spray 15, where the perfume oil composition 1 B is replaced by the same amount of the perfume oil composition 4.
Deo pump spray 19 corresponds to deo pump spray 15, where the perfume oil composition 1 B is replaced by the same amount of the perfume oil composition 5.
Deo pump spray 20 corresponds to deo pump spray 15, where the perfume oil composition 1 B is replaced by the same amount of the perfume oil composition 6.
Deo pump spray 21 corresponds to deo pump spray 15, where the perfume oil composition 1 B is replaced by the same amount of the perfume oil composition 7.
Deo pump spray 22 corresponds to deo pump spray 15, where the perfume oil composition 1B is replaced by the same amount of the perfume oil composition 8.
Deo pump spray 23 corresponds to deo pump spray 15, where the perfume oil composition 1B is replaced by the same amount of the perfume oil composition 9.
Deo pump spray 24 corresponds to deo pump spray 15, where the perfume oil composition 1B is replaced by the same amount of the perfume oil composition 10.
Deo pump spray 25 corresponds to deo pump spray 15, where the perfume oil composition 1B is replaced by the same amount of the perfume oil composition 11.
Deo pump spray 26 corresponds to deo pump spray 15, where the perfume oil composition 1B is replaced by the same amount of the perfume oil composition 12.
Deo pump spray 27 corresponds to deo pump spray 15, where the perfume oil composition 1B is replaced by the same amount of the perfume oil composition 13.
Deo pump spray 28 corresponds to deo pump spray 15, where the perfume oil composition 1B is replaced by the same amount of the perfume oil composition 14.

**Table 7: Clean hair conditioner 1**

| **Clear Hair Conditioner** | | |
|---|---|---|
| **Component** | **INCI** | **amount %** |
| DEHYQUART® L 80 | Dicocoylethyl Hydroxyethylmonium Methosulfate (and) Propylene Glycol | 1.0 |
| Propylene Glycol | solvent | 10.0 |
| Eumulgin® HRE 60 | PEG-60 Hydrogenated Castor Oil | 1.0 |
| LAMESOFT® PO 65 | Coco-Glucoside (and) Glyceryl Oleate | 1.0 |
| GLUADIN® W 40 | Hydrolyzed Wheat Gluten Hydrolyzed Wheat Protein | 0.5 |
| Perfume oil composition 1B | Perfume | 0,02 |
| Preservative | | q.s |
| HYDAGEN® HCMF | Chitosan | 10.0 (sol.1%) |
| Water, deionized | | up to 100.0 |
| | | |
| pH-value | 3.5 - 4.0 | |

Clean hair conditioner 2 corresponds to clean hair conditioner 1, where the perfume oil composition 1 B is replaced by the same amount of the perfume oil composition 1B 2.
Clean hair conditioner 3 corresponds to clean hair conditioner 1, where the perfume oil composition 1 B is replaced by the same amount of the perfume oil composition 1B 3.
Clean hair conditioner 4 corresponds to clean hair conditioner 1, where the perfume oil composition 1B is replaced by the same amount of the perfume oil composition 1B 4.
Clean hair conditioner 5 corresponds to clean hair conditioner 1, where the perfume oil composition 1B is replaced by the same amount of the perfume oil composition 1B 5.
Clean hair conditioner 6 corresponds to clean hair conditioner 1, where the perfume oil composition 1B is replaced by the same amount of the perfume oil composition 1B 6.
Clean hair conditioner 7 corresponds to clean hair conditioner 1, where the perfume oil composition 1B is replaced by the same amount of the perfume oil composition 1B 7.
Clean hair conditioner 8 corresponds to clean hair conditioner 1, where the perfume oil composition 1B is replaced by the same amount of the perfume oil composition 8.
Clean hair conditioner 9 corresponds to clean hair conditioner 1, where the perfume oil composition 1B is replaced by the same amount of the perfume oil composition 9.
Clean hair conditioner 10 corresponds to clean hair conditioner 1, where the perfume oil composition 1B is replaced by the same amount of the perfume oil composition 10.
Clean hair conditioner 11 corresponds to clean hair conditioner 1, where the perfume oil composition 1B is replaced by the same amount of the perfume oil composition 11.
Clean hair conditioner 12 corresponds to clean hair conditioner 1, where the perfume oil composition 1B is replaced by the same amount of the perfume oil composition 12.
Clean hair conditioner 13 corresponds to clean hair conditioner 1, where the perfume oil composition 1B is replaced by the same amount of the perfume oil composition 13.
Clean hair conditioner 14 corresponds to clean hair conditioner 1, where the perfume oil composition 1B is replaced by the same amount of the perfume oil composition 14.

**Table 8: Face wash gel 1**

| **Face Wash Gel** | | |
|---|---|---|
| **Component** | **INCI** | **amount %** |
| PLANTAPON® 611 L | Sodium Laureth Sulfate (and) Lauryl Glucoside (and) Cocamidopropyl Betaine | 20 |
| PLANTAPON® ACG 35 | Disodium Cocoyl Glutamate | 1 |
| LAMESOFT® PO 65 | Coco-Glucoside (and) Glyceryl Oleate | 2 |
| NaCl | Sodium Chloride | 1.7 |
| PLANTACARE® PS 10 | Sodium Laureth Sulfate (and) Lauryl Polyglucose | 0.3 |
| Perfume oil composition 1B | Perfume | 0.1 |
| Water | Aqua | QS |
| Preservative | | QS |
| Dye | | QS |
| pH 6 to 7 | | |

Face wash gel 2 corresponds to face wash gel 1, where the perfume oil composition 1B is replaced by the same amount of the perfume oil composition 2B.
Face wash gel 3 corresponds to face wash gel 1, where the perfume oil composition 1B is replaced by the same amount of the perfume oil composition 3.
Face wash gel 4 corresponds to face wash gel 1, where the perfume oil composition 1B is replaced by the same amount of the perfume oil composition 4.
Face wash gel 5 corresponds to face wash gel 1, where the perfume oil composition 1B is replaced by the same amount of the perfume oil composition5.
Face wash gel 6 corresponds to face wash gel 1, where the perfume oil composition 1B is replaced by the same amount of the perfume oil composition 6.
Face wash gel 7 corresponds to face wash gel 1, where the perfume oil composition 1B is replaced by the same amount of perfume oil composition 7.
Face wash gel 8 corresponds to face wash gel 1, where the perfume oil composition 1B is replaced by the same amount of the perfume oil composition 8.
Face wash gel 9 corresponds to face wash gel 1, where the perfume oil composition 1B is replaced by the same amount of the perfume oil composition 9.
Face wash gel 10 corresponds to face wash gel 1, where the perfume oil composition 1B is replaced by the same amount of the perfume oil composition 10.
Face wash gel 11 corresponds to face wash gel 1, where the perfume oil composition 1B is replaced by the same amount of the perfume oil composition 11.
Face wash gel 12 corresponds to face wash gel 1, where the perfume oil composition 1B is replaced by the same amount of the perfume oil composition 12.
Face wash gel 13 corresponds to face wash gel 1, where the perfume oil composition 1B is replaced by the same amount of the perfume oil composition 13.
Face wash gel 14 corresponds to face wash gel 1, where the perfume oil composition 1B is replaced by the same amount of the perfume oil composition 14.

**Table 9: Foam bath concentrate 1**

| **Foam Bath Concentrate** | | |
|---|---|---|
| **Component** | **INCI** | **Amount %** |
| TEXAPON® K 14 S 70spec. | Sodium Myreth Sulfate | 25 |
| PLANTACARE® 2000 UP | Decyl Glucoside | 20 |
| DEHYTON® K | Cocamidopropyl Betaine | 20 |
| GLUADIN® WP | Hydrolyzed Wheat Gluten Hydrolyzed Wheat Protein | 1 |
| PLANTACARE® PS 10 | Sodium Laureth Sulfate (and) Lauryl Polyglucose | 5 |
| Water, de ionized | | ad 100 |
| Citric Acid, 50 % | | 0.5 |
| Perfume oil composition 1B | perfume | 2.0 |
| pH value | 5.5 | |

Foam bath concentrate 2 corresponds to foam bath concentrate 1, where the perfume oil composition 1B is replaced by the same amount of the perfume oil composition 2B.
Foam bath concentrate 3 corresponds to foam bath concentrate 1, where the perfume oil composition 1B is replaced by the same amount of the perfume oil composition 3.
Foam bath concentrate 4 corresponds to foam bath concentrate 1, where the perfume oil composition 1B is replaced by the same amount of the perfume oil composition 4.
Foam bath concentrate 5 corresponds to foam bath concentrate 1, where the perfume oil composition 1B is replaced by the same amount of the perfume oil composition 5.
Foam bath concentrate 6 corresponds to foam bath concentrate 1, where the perfume oil composition 1B is replaced by the same amount of the perfume oil composition 6.
Foam bath concentrate 7 corresponds to foam bath concentrate 1, where the perfume oil composition 1B is replaced by the same amount of the perfume oil composition 7.
Foam bath concentrate 8 corresponds to foam bath concentrate 1, where the perfume oil composition 1B is replaced by the same amount of the perfume oil composition 8.
Foam bath concentrate 9 corresponds to foam bath concentrate 1, where the perfume oil composition 1B is replaced by the same amount of the perfume oil composition 9.
Foam bath concentrate 10 corresponds to foam bath concentrate 1, where the perfume oil composition 1B is replaced by the same amount of the perfume oil composition 10.
Foam bath concentrate 11 corresponds to foam bath concentrate 1, where the perfume oil composition 1B is replaced by the same amount of the perfume oil composition 11.
Foam bath concentrate 12 corresponds to foam bath concentrate 1, where the perfume oil composition 1B is replaced by the same amount of the perfume oil composition 12.
Foam bath concentrate 13 corresponds to foam bath concentrate 1, where the perfume oil composition 1B is replaced by the same amount of the perfume oil composition 13.
Foam bath concentrate 14 corresponds to foam bath concentrate 1, where the perfume oil composition 1B is replaced by the same amount of the perfume oil composition 14.

**Table 10: Hair gel 1**

| **Hair Gel** | | |
|---|---|---|
| **Component** | **INCI** | **amount %** |
| HYDAGEN® HCMF | Chitosan | 0.5 |
| Glycolic Acid (Fa. Merck) | Solvent | 0.2 |
| Water | | ad 100 |
| Jaguar HP 105 (2%swelling) | Thickener | 65 |
| Eumulgin® HRE 60 | PEG-60 Hydrogenated Castor Oil | 1.5 |
| Perfume oil composition 1B | Perfume | 0.1 |
| Ethanol | Evaporation agent | 7 |
| pH-value | 5.8 | |
| Viscosity (mPas), Brook.RVF, 23°C, sp.7, 10rpm = 20.000 | | |

Hair gel 2 corresponds to hair gel 1, where the perfume oil composition 1B is replaced by the same amount of the perfume oil composition 2B.
Hair gel 3 corresponds to hair gel 1, where the perfume oil composition 1B is replaced by the same amount of the perfume oil composition 3.
Hair gel 4 corresponds to hair gel 1, where the perfume oil composition 1B is replaced by the same amount of the perfume oil composition 4.
Hair gel 5 corresponds to hair gel 1, where the perfume oil composition 1B is replaced by the same amount of the perfume oil composition 5.
Hair gel 6 corresponds to hair gel 1, where the perfume oil composition 1B is replaced by the same amount of the perfume oil composition 6.
Hair gel 7 corresponds to hair gel 1, where the perfume oil composition 1B is replaced by the same amount of the perfume oil composition 7.
Hair gel 8 corresponds to hair gel 1, where the perfume oil composition 1B is replaced by the same amount of the perfume oil composition 8.
Hair gel 9 corresponds to hair gel 1, where the perfume oil composition 1B is replaced by the same amount of the perfume oil composition 9.
Hair gel 10 corresponds to hair gel 1, where the perfume oil composition 1B is replaced by the same amount of the perfume oil composition 10.
Hair gel 11 corresponds to hair gel 1, where the perfume oil composition 1B is replaced by the same amount of the perfume oil composition 11.
Hair gel 12 corresponds to hair gel 1, where the perfume oil composition 1B is replaced by the same amount of the perfume oil composition 12.
Hair gel 13 corresponds to hair gel 1, where the perfume oil composition 1B is replaced by the same amount of the perfume oil composition 13.
Hair gel 14 corresponds to hair gel 1, where the perfume oil composition 1B is replaced by the same amount of the perfume oil composition 14.

**Table 11: Self foaming bodywash 1**

| **Self foaming bodywash** | | |
|---|---|---|
| **Component** | **INCI** | **amount %** |
| TEXAPON® SB 3 KC | Disodium Laureth Sulfosuccinate | 16.5 |
| DEHYTON® K | Cocamidopropyl Betaine | 6.5 |
| PLANTACARE® 818 UP | Coco Glucoside | 7.5 |
| TEXAPON® NSO | Sodium laureth sulfate | 14.2 |
| LAMESOFT® PO 65 | Coco-Glucoside (and) Glyceryl Oleate | 5 |
| Dow Corning 193 | Dimethicole Copolyol | 1 |
| PLANTACARE® PS 10 | Sodium Laureth Sulfate (and) Lauryl Polyglucose | 0.5 |
| Perfume oil composition | Perfume | 0.5 |
| Kathon CG | Methylchloroisothiazolinone (and) Methyl-isothiazolinone | 0.05 |
| Water, de-ionized | Aqua | ad 100 |
| Hexylen Glycol (Elf Atochem) | Humectant | 3 |
| UCARE Polymer JR 400 | Polyquaternium-10 | 0.5 |
| pH: 5.5 | | |
| Viscosity: > 100 mPas | | |

Self foaming bodywash 2 corresponds to self foaming bodywash, where the perfume oil composition 1B is replaced by the same amount of the perfume oil composition 2B.
Self foaming bodywash 3 corresponds to self foaming bodywash 1, where the perfume oil composition 1B is replaced by the same amount of the perfume oil composition 3.
Self foaming bodywash 4 corresponds to self foaming bodywash 1, where the perfume oil composition 1B is replaced by the same amount of the perfume oil composition 4.
Self foaming bodywash 5 corresponds to self foaming bodywash 1, where the perfume oil composition 1B is replaced by the same amount of the perfume oil composition 5.
Self foaming bodywash 6 corresponds to self foaming bodywash 1, where the perfume oil composition 1B is replaced by the same amount of the perfume oil composition 6.
Self foaming bodywash 7 corresponds to self foaming bodywash 1, where the perfume oil composition 1B is replaced by the same amount of the perfume oil composition 7.
Self foaming bodywash 8 corresponds to self foaming bodywash 1, where the perfume oil composition 1B is replaced by the same amount of the perfume oil composition 8.
Self foaming bodywash 9 corresponds to self foaming bodywash 1, where the perfume oil composition 1B is replaced by the same amount of the perfume oil composition 9.
Self foaming bodywash 10 corresponds to self foaming bodywash 1, where the perfume oil composition 1B is replaced by the same amount of the perfume oil composition 10.
Self foaming bodywash 11 corresponds to self foaming bodywash 1, where the perfume oil composition 1B is replaced by the same amount of the perfume oil composition 11.
Self foaming bodywash 12 corresponds to self foaming bodywash 1, where the perfume oil composition 1B is replaced by the same amount of the perfume oil composition 12.
Self foaming bodywash 13 corresponds to self foaming bodywash 1, where the perfume oil composition 1B is replaced by the same amount of the perfume oil composition 13.
Self foaming bodywash 14 corresponds to self foaming bodywash 1, where the perfume oil composition 1B is replaced by the same amount of the perfume oil composition 14.

**Table 12: Sprayable sun care emulsion 1**

| **Sprayable Sun Care Emulsion** | | |
|---|---|---|
| Component | INCI | amount |
| EMULGADE® SE-PF | Glyceryl Stearate (and) Ceteareth-20 (and) Ceteareth-12 (and) Cetearyl Alcohol (and) Cetyl Palmitate | 7.8 % |
| EUMULGIN® B 3 | Ceteareth-30 | 4.2 % |
| CETIOL® CC | Dicaprylyl Carbonate | 5.0 % |
| CETIOL® OE | Dicaprylyl Ether | 5.0 % |
| Neo Heliopan BB (Haarmann&Reimer) | | 4.5 % |
| Neo Heliopan AV (Haarmann&Reimer) | | 7.5 % |
| Neo Heliopan 357 (Haarmann& Reimer) | | 2.0 % |
| Water, de-ionized | | ad 100 |
| Perfume oil composition 1B | Perfume | 0,05 |
| Eumulgin® HRE 60 | PEG-60 Hydrogenated Castor Oil | 1.2 |
| pH | 5.5 | |
| Viscosity (mPas), RVF spindle 2, 20°C, 50 rpm < 100 | | |

Sprayable sun care emulsion 2 corresponds to sprayable sun care emulsion 1, where the perfume oil composition 1B is replaced by the same amount of the perfume oil composition 2B.
Sprayable sun care emulsion 3 corresponds to sprayable sun care emulsion 1, where the perfume oil composition 1B is replaced by the same amount of perfume oil composition 3.
Sprayable sun care emulsion 4 corresponds to sprayable sun care emulsion 1, where the perfume oil composition 1B is replaced by the same amount of the perfume oil composition 4.
Sprayable sun care emulsion 5 corresponds to sprayable sun care emulsion 1, where the perfume oil composition 1B is replaced by the same amount of the perfume oil composition 5.
Sprayable sun care emulsion 6 corresponds to sprayable sun care emulsion 1, where the perfume oil composition 1B is replaced by the same amount of the perfume oil composition 6.
Sprayable sun care emulsion 7 corresponds to sprayable sun care emulsion 1, where the perfume oil composition 1B is replaced by the same amount of the perfume oil composition 7.
Sprayable sun care emulsion 8 corresponds to sprayable sun care emulsion 1, where the perfume oil composition 1B is replaced by the same amount of the perfume oil composition 8.
Sprayable sun care emulsion 9 corresponds to sprayable sun care emulsion 1, where the perfume oil composition 1B is replaced by the same amount of the perfume oil composition 9.
Sprayable sun care emulsion 10 corresponds to sprayable sun care emulsion 1, where the perfume oil composition 1B is replaced by the same amount of the perfume oil composition 10.
Sprayable sun care emulsion 11 corresponds to sprayable sun care emulsion 1, where the perfume oil composition 1B is replaced by the same amount of the perfume oil composition 11.
Sprayable sun care emulsion 12 corresponds to sprayable sun care emulsion 1, where the perfume oil composition 1B is replaced by the same amount of the perfume oil composition 12.
Sprayable sun care emulsion 13 corresponds to sprayable sun care emulsion 1, where the perfume oil composition 1B is replaced by the same amount of the perfume oil composition 13.
Sprayable sun care emulsion 14 corresponds to sprayable sun care emulsion 1, where the perfume oil composition 1B is replaced by the same amount of the perfume oil composition 14.

**Table 13: Sprayable sun protection emulsion 1**

| **Sprayable Sun Protection Emulsion** | | |
|---|---|---|
| Component | INCI | amount % |
| EMULGADE® SE-PF | Glyceryl Stearate (and) Ceteareth-20 (and) Ceteareth-12 (and) Cetearyl Alcohol (and) Cetyl Palmitate Ceteareth-33 | 9.0 |
| Eumulgin B3 | | 6.0 |
| CETIOL® CC | Dicaprylyl Carbonate | 4.0 |
| Eusolex HMS | Homosalate | 7.0 |
| Parsol MCX (Givaudan Roure) | Ethylhexyl Methoxycinnamate | 7.5 |
| Parsol 1789 | Butyl Methoxydibenzoylmethane | 3.0 |
| Neo Heliopan OS | Ethylhexyl Salicylate | 5.0 |
| Preservative | | q.s. |
| Water, de-ionized | | ad 100 |
| perfume oil composition | Perfume | 0.2 |
| Eumulgin® HRE 60 | PEG-60 Hydrogenated Castor Oil | 1.2 |
| pH-value | 6.2 | |

Sprayable sun protection emulsion 2 corresponds to sprayable sun protection emulsion 1, where the perfume oil composition 1B is replaced by the same amount of the perfume oil composition 2B.
Sprayable sun protection emulsion 3 corresponds to sprayable sun protection emulsion 1, where the perfume oil composition 1B is replaced by the same amount of the perfume oil composition 3.
Sprayable sun protection emulsion 4 corresponds to sprayable sun protection emulsion 1, where the perfume oil composition 1B is replaced by the same amount of the perfume oil composition 4.
Sprayable sun protection emulsion 5 corresponds to sprayable sun protection emulsion 1, where the perfume oil composition 1B is replaced by the same amount of the perfume oil composition 5.
Sprayable sun protection emulsion 6 corresponds to sprayable sun protection emulsion 1, where the perfume oil composition 1B is replaced by the same amount of the perfume oil composition 6.
Sprayable sun protection emulsion 7 corresponds to sprayable sun protection emulsion 1, where the perfume oil composition 1B is replaced by the same amount of the perfume oil composition 7.
Sprayable sun protection emulsion 8 corresponds to sprayable sun protection emulsion 1, where the perfume oil composition 1B is replaced by the same amount of the perfume oil composition 8.
Sprayable sun protection emulsion 9 corresponds to sprayable sun protection emulsion 1, where the perfume oil composition 1B is replaced by the same amount of the perfume oil composition 9.
Sprayable sun protection emulsion 10 corresponds to sprayable sun protection emulsion 1, where the perfume oil composition 1B is replaced by the same amount of the perfume oil composition 10.
Sprayable sun protection emulsion 11 corresponds to sprayable sun protection emulsion 1, where the perfume oil composition 1B is replaced by the same amount of the perfume oil composition 11.
Sprayable sun protection emulsion 12 corresponds to sprayable sun protection emulsion 1, where the perfume oil composition 1B is replaced by the same amount of the perfume oil composition 12.
Sprayable sun protection emulsion 13 corresponds to sprayable sun protection emulsion 1, where the perfume oil composition 1B is replaced by the same amount of the perfume oil composition 13.
Sprayable sun protection emulsion 14 corresponds to sprayable sun protection emulsion 1, where the perfume oil composition 1B is replaced by the same amount of the perfume oil composition 14.

**Table 14: Emollient facial gel 1**

| **Emollient facial Gel** | | |
|---|---|---|
| **Component** | **INCI** | **amount %** |
| Water | | Ad 100 |
| HISPAGEL® 200 | Glycerin (and) Glyceryl Polyacrylate | 10 |
| CET-OE-Primasponge®BLUE | | 0.5 |
| PLANTACARE® PS 10 | Sodium Laureth Sulfate (and) Lauryl Polyglucose | 0.6 |
| Perfume oil composition 1B | | 0.1 |

Emollient facial gel 2 corresponds to emollient facial gel 1, where the perfume oil composition 1B is replaced by the same amount of the perfume oil composition 2B.
Emollient facial gel 3 corresponds to emollient facial gel 1, where the perfume oil composition 1B is replaced by the same amount of the perfume oil composition 3.
Emollient facial gel 4 corresponds to emollient facial gel 1, where the perfume oil composition 1B is replaced by the same amount of the perfume oil composition 4.
Emollient facial gel 5 corresponds to emollient facial gel 1, where the perfume oil composition 1B is replaced by the same amount of the perfume oil composition 5.
Emollient facial gel 6 corresponds to emollient facial gel 1, where the perfume oil composition 1B is replaced by the same amount of the perfume oil composition 6.
Emollient facial gel 7 corresponds to emollient facial gel 1, where the perfume oil composition 1B is replaced by the same amount of the perfume oil composition 7.
Emollient facial gel 8 corresponds to emollient facial gel 1, where the perfume oil composition 1B is replaced by the same amount of the perfume oil composition 8.
Emollient facial gel 9 corresponds to emollient facial gel 1, where the perfume oil composition 1B is replaced by the same amount of the perfume oil composition 9.
Emollient facial gel 10 corresponds to emollient facial gel 1, where the perfume oil composition 1B is replaced by the same amount of the perfume oil composition 10.
Emollient facial gel 11 corresponds to emollient facial gel 1, where the perfume oil composition 1B is replaced by the same amount of the perfume oil composition 11.
Emollient facial gel 12 corresponds to emollient facial gel 1, where the perfume oil composition 1B is replaced by the same amount of the perfume oil composition 12.
Emollient facial gel 13 corresponds to emollient facial gel 1, where the perfume oil composition 1B is replaced by the same amount of the perfume oil composition 13.
Emollient facial gel 14 corresponds to emollient facial gel 1, where the perfume oil composition 1B is replaced by the same amount of the perfume oil composition 14.

**Table 15: 2-phases oil foam bath 1**

| **2-Phases-Oilfoambath** | | |
|---|---|---|
| Component | INCI | amount % b.w. |
| PLANTACARE® PS 10 | Sodium Laureth Sulfate (and) Lauryl Polyglucose | 10 |
| Paraffin oil | (low viscosity) | 20 |
| TEXAPON® N 70 | Sodium Laureth sulfate | 13 |
| DEHYTON® PK 45 | Cocamidopropyl Betaine | 8 |
| Perfume oil composition 1B | Perfume | 2 |
| Glycerin (86%) | | 5 |
| Preservative | | q.s. |
| Water, de-ionized | | ad 100,0 |
| pH-value | 5,5 | |

2-phases oil foam bath 2 corresponds to 2-phases oil foam bath 1, where the perfume oil composition 1B is replaced by the same amount of the perfume oil composition 2B.
2-phases oil foam bath 3 corresponds to 2-phases oil foam bath 1, where the perfume oil composition 1B is replaced by the same amount of the perfume oil composition 3.
2-phases oil foam bath 4 corresponds to 2-phases oil foam bath 1, where the perfume oil composition 1B is replaced by the same amount of the perfume oil composition 4.
2-phases oil foam bath 5 corresponds to 2-phases oil foam bath 1, where the perfume oil composition 1B is replaced by the same amount of the perfume oil composition 5.
2-phases oil foam bath 6 corresponds to 2-phases oil foam bath 1, where the perfume oil composition 1B is replaced by the same amount of the perfume oil composition 6.
2-phases oil foam bath 7 corresponds to 2-phases oil foam bath 1, where the perfume oil composition 1B is replaced by the same amount of the perfume oil composition 7.
2-phases oil foam bath 8 corresponds to 2-phases oil foam bath 1, where the perfume oil composition 1B is replaced by the same amount of the perfume oil composition 8.
2-phases oil foam bath 9 corresponds to 2-phases oil foam bath 1, where the perfume oil composition 1B is replaced by the same amount of the perfume oil composition 9.
2-phases oil foam bath 10 corresponds to 2-phases oil foam bath 1, where the perfume oil composition 1B is replaced by the same amount of the perfume oil composition 10.
2-phases oil foam bath 11 corresponds to 2-phases oil foam bath 1, where the perfume oil composition 1B is replaced by the same amount of the perfume oil composition 11.
2-phases oil foam bath 12 corresponds to 2-phases oil foam bath 1, where the perfume oil composition 1B is replaced by the same amount of the perfume oil composition 12.
2-phases oil foam bath 13 corresponds to 2-phases oil foam bath 1, where the perfume oil composition 1B is replaced by the same amount of the perfume oil composition 13.
2-phases oil foam bath 14 corresponds to 2-phases oil foam bath 1, where the perfume oil composition 1B is replaced by the same amount of the perfume oil composition 14.

**Table 16: Shampoos 1, 2, 3 and 4**

| **Shampoo** | | | **1** | **2** | **3** | **4** |
|---|---|---|---|---|---|---|
| **Component** | **INCI** | **Conc.** | **% b.w.** | | | |
| Texapon N70 | Sodium Laureth Sulfate | 70% | 12.9 | 12.9 | 14.3 | 14.3 |
| Dehyton PK45 | Cocamidopropyl Betaine | 44 - 46% | 5.4 | 5.4 | 5.4 | 5.4 |
| Plantacare 818UP | Coco-Glucoside | 51 - 53% | 2.0 | 2.0 | --- | --- |
| PLANTACARE® PS 10 | Sodium Laureth Sulfate (and) Lauryl Polyglucose | 33% | 6.0 | 18.0 | 21.0 | 30.0 |
| Copherol 1250 C | Tocopheryl Acetate | 100% | 1.0 | --- | --- | --- |
| Cetiol J 600 | Oleyl Erucate | 100% | --- | 0,7 | --- | 1,0 |
| Almond Oil | Prunus Amygdalus Dulcis (Sweet Almond) Oil | 100% | --- | --- | 0.3 | --- |
| Jaguar C162 | Hydroxypropyl Guar Hydroxypropy-Itrimonium Chloride | 100% | 0.2 | 0.2 | --- | --- |
| Jaguar Excel | Guar Hydroxypropyltrimonium Chloride | 100% | --- | --- | 0.25 | 0.25 |
| Arlypon TT | PEG/PPG-120/10 Trimethylolpropane Trioleate (and) Laureth-2 | 40 - 50% 40 - 50% | 1.0 | 2.0 | 1.0 | 2.0 |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 70.7 | 49.0 | 56.95 | 46.25 |
| pH value | | | 5.0 | 4.9 | 5.2 | 5.1 |
| Viscosity [mPas] | | | 5600 | 3100 | 4600 | 2500 |

**Table 17: Shampoos 5, 6, 7 and 8**

| **Shampoo** | | | | **5** | **6** | **7** | **8** |
|---|---|---|---|---|---|---|---|
| **Component** | **INCI** | | **Conc.** | **% b.w.** | | | |
| Sulfopon 1216 | Sodium Coco- | | 90 - 95% | --- | --- | 12.8 | --- |
| G | Sulfate | | | | | | |
| Texapon ALS Benz | Ammonium Lauryl Sulfate | | 27 - 28% | --- | --- | --- | 32.0 |
| Plantacare 1200UP | Lauryl Glucoside | | 51-53% | 15.0 | --- | --- | --- |
| Plantacare 2000UP | Decyl Glucoside | | 51 - 55% | --- | - | - | 6.0 |
| Plantacare 818UP | Coco-Glucoside | | 51 - 53% | --- | 30.0 | 20.4 | --- |
| Plantacare 810UP | Caprylyl/Capryl Glucoside | | 62 - 65% | 12.0 | --- | --- | --- |
| Jaguar Excel | Guar Hydroxypropyltrimonium Chloride | | 100% | 0.2 | 0.25 | 0.2 | 0.3 |
| Dehyton PK45 | Cocam idopropyl Betaine | | 44 - 46% | | | | 11.0 |
| | | 15-25 | 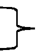 28,5 - 34% | | | | |
| | Coco-Glucoside | 10-20 | | | | | |
| Lamesoft P065 | (and) Glyceryl Oleate water | 62 - 67 | | 2.0 | 2.0 | 2.0 | 3.0 |
| | Lauryl Glucoside | 15 - 25 | 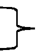 38 - 44% | | | | |
| | (and) Stearyl | 15-25 | | | | | 2.0 |
| Euperlan Green | Citrate water | 55 - 65 | | --- | --- | --- | |
| Gluadin WLM Benz | Hydrolyzed Wheat Protein | | 21 - 24% | --- | 0.5 | 1.0 | --- |
| PLANT ACARE ® PS 10 | Sodium Laureth Sulfate (and) Lauryl Polyglucose | | 33% | 6.0 | 15.0 | 7.5 | 10.5 |
| Copherol 1250 C | Tocopheryl Acetate | | 100% | 0.25 | --- | --- | --- |
| Cetiol J 600 | Oleyl Erucate | | 100% | --- | 0.3 | --- | --- |
| Almond Oil | Prunus Amygdalus Dulcis (Sweet Almond) Oil | | 100% | --- | --- | 0.2 | 0.25 |
| Keltrol CG-SFT | Xanthan Gum | | 100% | 1.0 | 1.0 | | |
| Perfume oil composition 1B | Fragrance | | 100% | 0.25 | 0.25 | 0.25 | 0.25 |
| Na-Benzoate | Sodium Benzoate | | 100% | 0.5 | 0.5 | 0.5 | 0.5 |
| Citric Acid | Citric Acid | | 50% | q.s. | q.s. | q.s. | q.s. |
| NaCl | Sodium Chloride | | 100% | q.s. | q.s. | q.s. | q.s. |
| Floral water | Floral water | | 100% | --- | --- | --- | 10.0 |
| Water | Water | | 100% | 62.8 | 50.2 | 55.15 | 24.2 |
| | | | | | | | |
| pH value | | | | 5.1 | 5.0 | 5.3 | 4.8 |
| Viscosity [mPas] | | | | 6200 | 4600 | 6800 | 5800 |
| Density | g/cm³ | | | | | | 1.028 |

Shampoo 9 corresponds to shampoo 1, where the perfume oil composition 1B1 is replaced by the same amount of the perfume oil composition 2B.
Shampoo 10 corresponds to shampoo 2, where perfume oil composition 1Bis replaced by the same amount of the perfume oil composition 2B.
Shampoo 11 corresponds to shampoo 3, where the perfume oil composition 1B is replaced by the same amount of the perfume oil composition 2B.
Shampoo 12 corresponds to shampoo 4, where the perfume oil composition 1B is replaced by the same amount of the perfume oil composition 2B.
Shampoo 13 corresponds to shampoo 5, where the perfume oil composition 1B is replaced by the same amount of perfume oil composition 2B.
Shampoo 14 corresponds to shampoo 6, where the perfume oil composition 1B is replaced by the same amount of the perfume oil composition 2B.
Shampoo 15 corresponds to shampoo 7, where the perfume oil composition 1B is replaced by the same amount of the perfume oil composition 2B.
Shampoo 16 corresponds to shampoo 8, where the perfume oil composition 1B is replaced by the same amount of the perfume oil composition 2B.
Shampoo 17 corresponds to shampoo 1, where the perfume oil composition 1B is replaced by the same amount of the perfume oil composition 3.
Shampoo 18 corresponds to shampoo 2, where the perfume oil composition 1B is replaced by the same amount of the perfume oil composition 3.
Shampoo 19 corresponds to shampoo 3, where the perfume oil composition 1B is replaced by the same amount of the perfume oil composition 3.
Shampoo 20 corresponds to shampoo 4, where the perfume oil composition 1B is replaced by the same amount of the perfume oil composition 3.
Shampoo 21 corresponds to shampoo 5, where the perfume oil composition 1B is replaced by the same amount of the perfume oil composition 3.
Shampoo 22 corresponds to shampoo 6, where the perfume oil composition 1B is replaced by the same amount of the perfume oil composition 3.
Shampoo 23 corresponds to shampoo 7, where the perfume oil composition 1B is replaced by the same amount of the perfume oil composition 3.
Shampoo 24 corresponds to shampoo 8, where the perfume oil composition 1B is replaced by the same amount of the perfume oil composition 3.
Shampoo 25 corresponds to shampoo 1, where the perfume oil composition 1B is replaced by the same amount of the perfume oil composition 4.
Shampoo 26 corresponds to shampoo 2, where the perfume oil composition 1B is replaced by the same amount of the perfume oil composition 4.
Shampoo 27 corresponds to shampoo 3, where the perfume oil composition 1B is replaced by the same amount of the perfume oil composition 4.
Shampoo 28 corresponds to shampoo 4, where the perfume oil composition 1B is replaced by the same amount of the perfume oil composition 4.
Shampoo 29 corresponds to shampoo 5, where the perfume oil composition 1B is replaced by the same amount of the perfume oil composition 4.
Shampoo 30 corresponds to shampoo 6, where the perfume oil composition 1B is replaced by the same amount of the perfume oil composition 4.
Shampoo 31 corresponds to shampoo 7, where the perfume oil composition 1B is replaced by the same amount of the perfume oil composition 4.
Shampoo 32 corresponds to shampoo 8, where the perfume oil composition 1B is replaced by the same amount of the perfume oil composition 4.
Shampoo 33 corresponds to shampoo 1, where the perfume oil composition 1B is replaced by the same amount of the perfume oil composition 5.
Shampoo 34 corresponds to shampoo 2, where the perfume oil composition 1B is replaced by the same amount of the perfume oil composition 5.
Shampoo 35 corresponds to shampoo 3, where the perfume oil composition 1B is replaced by the same amount of the perfume oil composition 5.
Shampoo 36 corresponds to shampoo 4, where the perfume oil composition 1B is replaced by the same amount of the perfume oil composition 5.
Shampoo 37 corresponds to shampoo 5, where the perfume oil composition 1B is replaced by the same amount of the perfume oil composition 5.
Shampoo 38 corresponds to shampoo 6, where the perfume oil composition 1B is replaced by the same amount of the perfume oil composition 5.
Shampoo 39 corresponds to shampoo 7, where the perfume oil composition 1B is replaced by the same amount of the perfume oil composition 5.
Shampoo 40 corresponds to shampoo 8, where the perfume oil composition 1B is replaced by the same amount of the perfume oil composition 5.
Shampoo 41 corresponds to shampoo 1, where the perfume oil composition 1B is replaced by the same amount of the perfume oil composition 6.
Shampoo 42 corresponds to shampoo 2, where the perfume oil composition 1B is replaced by the same amount of the perfume oil composition 6.
Shampoo 43 corresponds to shampoo 3, where the perfume oil composition 1B is replaced by the same amount of the perfume oil composition 6.
Shampoo 44 corresponds to shampoo 4, where the perfume oil composition 1B is replaced by the same amount of the perfume oil composition 6.
Shampoo 45 corresponds to shampoo 5, where the perfume oil composition 1B is replaced by the same amount of the perfume oil composition 6.
Shampoo 46 corresponds to shampoo 6, where the perfume oil composition 1B is replaced by the same amount of the perfume oil composition 6.
Shampoo 47 corresponds to shampoo 7, where the perfume oil composition 1B is replaced by the same amount of the perfume oil composition 6.
Shampoo 48 corresponds to shampoo 8, where the perfume oil composition 1B is replaced by the same amount of the perfume oil composition 6.
Shampoo 49 corresponds to shampoo 1, where the perfume oil composition 1B is replaced by the same amount of the perfume oil composition 7.
Shampoo 50 corresponds to shampoo 2, where the perfume oil composition 1B is replaced by the same amount of the perfume oil composition 7.
Shampoo 51 corresponds to shampoo 3, where the perfume oil composition 1B is replaced by the same amount of the perfume oil composition 7.
Shampoo 52 corresponds to shampoo 4, where the perfume oil composition 1B is replaced by the same amount of the perfume oil composition 7.
Shampoo 53 corresponds to shampoo 5, where the perfume oil composition 1B is replaced by the same amount of the perfume oil composition 7.
Shampoo 54 corresponds to shampoo 6, where the perfume oil composition 1B is replaced by the same amount of the perfume oil composition 7.
Shampoo 55 corresponds to shampoo 7, where the perfume oil composition 1B is replaced by the same amount of the perfume oil composition 7.
Shampoo 56 corresponds to shampoo 8, where the perfume oil composition 1B is replaced by the same amount of the perfume oil composition 7.
Shampoo 57 corresponds to shampoo 1, where the perfume oil composition 1B is replaced by the same amount of the perfume oil composition 8.
Shampoo 58 corresponds to shampoo 2, where the perfume oil composition 1B is replaced by the same amount of the perfume oil composition 8.
Shampoo 59 corresponds to shampoo 3, where the perfume oil composition 1B is replaced by the same amount of the perfume oil composition 8.
Shampoo 60 corresponds to shampoo 4, where the perfume oil composition 1B is replaced by the same amount of the perfume oil composition 8.
Shampoo 61 corresponds to shampoo 5, where the perfume oil composition 1B is replaced by the same amount of the perfume oil composition 8.
Shampoo 62 corresponds to shampoo 6, where the perfume oil composition 1B is replaced by the same amount of the perfume oil composition 8.
Shampoo 63 corresponds to shampoo 7, where the perfume oil composition 1B is replaced by the same amount of the perfume oil composition 8.
Shampoo 64 corresponds to shampoo 8, where the perfume oil composition 1B is replaced by the same amount of the perfume oil composition 8.
Shampoo 65 corresponds to shampoo 1, where the perfume oil composition 1B is replaced by the same amount of the perfume oil composition 9.
Shampoo 66 corresponds to shampoo 2, where the perfume oil composition 1B is replaced by the same amount of the perfume oil composition 9.
Shampoo 67 corresponds to shampoo 3, where the perfume oil composition 1B is replaced by the same amount of the perfume oil composition 9.
Shampoo 68 corresponds to shampoo 4, where the perfume oil composition 1B is replaced by the same amount of the perfume oil composition 9.
Shampoo 69 corresponds to shampoo 5, where the perfume oil composition 1B is replaced by the same amount of the perfume oil composition 9.
Shampoo 70 corresponds to shampoo 6, where the perfume oil composition 1B is replaced by the same amount of the perfume oil composition 9.
Shampoo 71 corresponds to shampoo 7, where the perfume oil composition 1B is replaced by the same amount of the perfume oil composition 9.
Shampoo 72 corresponds to shampoo 8, where the perfume oil composition 1B is replaced by the same amount of the perfume oil composition 9.
Shampoo 73 corresponds to shampoo 1, where the perfume oil composition 1B is replaced by the same amount of the perfume oil composition 10.
Shampoo 74 corresponds to shampoo 2, where the perfume oil composition 1B is replaced by the same amount of the perfume oil composition 10.
Shampoo 75 corresponds to shampoo 3, where the perfume oil composition 1B is replaced by the same amount of the perfume oil composition 10.
Shampoo 76 corresponds to shampoo 4, where the perfume oil composition 1B is replaced by the same amount of the perfume oil composition 10.
Shampoo 77 corresponds to shampoo 5, where the perfume oil composition 1B is replaced by the same amount of the perfume oil composition 10.
Shampoo 78 corresponds to shampoo 6, where the perfume oil composition 1B is replaced by the same amount of the perfume oil composition 10.
Shampoo 79 corresponds to shampoo 7, where the perfume oil composition 1B is replaced by the same amount of the perfume oil composition 10.
Shampoo 80 corresponds to shampoo 8, where the perfume oil composition 1B is replaced by the same amount of the perfume oil composition 10.
Shampoo 81 corresponds to shampoo 1, where the perfume oil composition 1B is replaced by the same amount of the perfume oil composition 11.
Shampoo 82 corresponds to shampoo 2, where the perfume oil composition 1B is replaced by the same amount of the perfume oil composition 11.
Shampoo 83 corresponds to shampoo 3, where the perfume oil composition 1B is replaced by the same amount of the perfume oil composition 11.
Shampoo 84 corresponds to shampoo 4, where the perfume oil composition 1B is replaced by the same amount of the perfume oil composition 11.
Shampoo 85 corresponds to shampoo 5, where the perfume oil composition 1B is replaced by the same amount of the perfume oil composition 11.
Shampoo 86 corresponds to shampoo 6, where the perfume oil composition 1B is replaced by the same amount of the perfume oil composition 11.
Shampoo 87 corresponds to shampoo 7, where the perfume oil composition 1B is replaced by the same amount of the perfume oil composition 11.
Shampoo 88 corresponds to shampoo 8, where the perfume oil composition 1B is replaced by the same amount of the perfume oil composition 11.
Shampoo 89 corresponds to shampoo 1, where the perfume oil composition 1B is replaced by the same amount of the perfume oil composition 12.
Shampoo 90 corresponds to shampoo 2, where the perfume oil composition 1B is replaced by the same amount of the perfume oil composition 12.
Shampoo 91 corresponds to shampoo 3, where the perfume oil composition 1B is replaced by the same amount of the perfume oil composition 12.
Shampoo 92 corresponds to shampoo 4, where the perfume oil composition 1B is replaced by the same amount of the perfume oil composition 12.
Shampoo 93 corresponds to shampoo 5, where the perfume oil composition 1B is replaced by the same amount of the perfume oil composition 12.
Shampoo 94 corresponds to shampoo 6, where the perfume oil composition 1B is replaced by the same amount of the perfume oil composition 12.
Shampoo 95 corresponds to shampoo 7, where the perfume oil composition 1B is replaced by the same amount of the perfume oil composition 12.
Shampoo 96 corresponds to shampoo 8, where the perfume oil composition 1B is replaced by the same amount of the perfume oil composition 12.
Shampoo 97 corresponds to shampoo 1, where the perfume oil composition 1B is replaced by the same amount of the perfume oil composition 13.
Shampoo 98 corresponds to shampoo 2, where the perfume oil composition 1B is replaced by the same amount of the perfume oil composition 13.
Shampoo 99 corresponds to shampoo 3, where the perfume oil composition 1B is replaced by the same amount of the perfume oil composition 13.
Shampoo 100 corresponds to shampoo 4, where the perfume oil composition 1B is replaced by the same amount of the perfume oil composition 13.
Shampoo 101 corresponds to shampoo 5, where the perfume oil composition 1B is replaced by the same amount of the perfume oil composition 13.
Shampoo 102 corresponds to shampoo 6, where the perfume oil composition 1B is replaced by the same amount of the perfume oil composition 13.
Shampoo 103 corresponds to shampoo 7, where the perfume oil composition 1B is replaced by the same amount of the perfume oil composition 13.
Shampoo 104 corresponds to shampoo 8, where the perfume oil composition 1B is replaced by the same amount of the perfume oil composition 13.
Shampoo 105 corresponds to shampoo 1, where the perfume oil composition 1B is replaced by the same amount of the perfume oil composition 14.
Shampoo 106 corresponds to shampoo 2, where the perfume oil composition 1B is replaced by the same amount of the perfume oil composition 14.
Shampoo 107 corresponds to shampoo 3, where the perfume oil composition 1B is replaced by the same amount of the perfume oil composition 14.
Shampoo 108 corresponds to shampoo 4, where the perfume oil composition 1B is replaced by the same amount of the perfume oil composition 14.
Shampoo 109 corresponds to shampoo 5, where the perfume oil composition 1B is replaced by the same amount of the perfume oil composition 14.
Shampoo 110 corresponds to shampoo 6, where the perfume oil composition 1B is replaced by the same amount of the perfume oil composition 14.
Shampoo 111 corresponds to shampoo 7, where the perfume oil composition 1B is replaced by the same amount of the perfume oil composition 14.
Shampoo 112 corresponds to shampoo 8, where the perfume oil composition 1B is replaced by the same amount of the perfume oil composition 14.

**Table 18: Shower bath 1, 2, 3 and 4**

| **Shower bath** | | | | **1** | **2** | **3** | **4** |
|---|---|---|---|---|---|---|---|
| **Component** | **INCI** | | **Conc.** | **% b.w.** | | | |
| Plantapon SF | Sodium Cocoamphoacetate (and) Glycerin (and) Lauryl Glucoside (and) Sodium Cocoyl Glutamate (and) Sodium Lauryl Glucose Carboxylate water | 10-20 | 42 - 52% | | | | |
| | | 5 - 15 | | | | | |
| | | 5 - 15 | | | | | |
| | | 5 | | | | | |
| | | 5 | | | | | |
| | | 48 58 | | 45.0 | 50.0 | 48.0 | 44.0 |
| Lamesoft PO65 | Coco-Glucoside (and) Glyceryl Oleate water | 15 25 | | | | | |
| | | 10 20 | 28.5 - 34% | | | | |
| | | 62 - 67 | | 2.0 | 2.0 | 2.0 | 3.0 |
| Euperlan Green | Lauryl Glucoside (and) Stearyl Citrate water | 15 - 25 | 38 - 44% | | | | 2.0 |
| | | 15 | | --- | --- | --- | |
| | | 25 | | | | | |
| | | 55 65 | | | | | |
| Gluadin WLM Benz | Hydrolyzed Wheat Protein | | 21 - 24% | - | - | 1.0 | 0.5 |
| Keltrol CG-SFT | Xanthan Gum | | 100% | 1.0 | 0.5 | - | - |
| PLANT ACARE® PS 10 | Sodium Laureth Sulfate (and) Lauryl Polyglucose | | 33% | 4.5 | 6.0 | 10.5 | 7.5 |
| Copherol1250 C | Tocopheryl Acetate | | 100% | 0.1 | - | - | - |
| Cetiol J 600 | Oleyl Erucate | | 100% | --- | 0,2 | --- | --- |
| Almond Oil | Prunus Amygdalus Dulcis (Sweet Almond) Oil | | 100% | --- | --- | 0.15 | 0.1 |
| Perfume oil composition1 B | Fragrance | | 100% | 0.25 | 0.25 | 0.25 | 0.25 |
| Na-Benzoate | Sodium Benzoate | | 100% | 0,5 | 0.5 | 0.5 | 0.5 |
| Citric Acid | Citric Acid | | 50% | q.s. | q.s. | q.s. | q.s. |
| Water | Water | | 100% | 46.65 | 40.55 | 37.6 | 42.15 |
| pH value | | | | 5.1 | 4.7 | 4.9 | 4.9 |
| Viscosity [mPas] | | | | 4200 | 4600 | 2900 | 2800 |
| Density | g/cm³ | | | | | | 1.039 |

**Table 19: Shower bath 5, 6, 7 and 8**

| **Shower bath** | | | **5** | **6** | **7** | **8** |
|---|---|---|---|---|---|---|
| **Component** | **INCI** | **Conc.** | **% b.w.** | | | |
| Texapon N70 | Sodium Laureth Sulfate | 70% | 12.9 | 12.9 | 14.3 | 14.3 |
| Dehyton PK45 | Cocam idopropyl Betaine | 44 - 46% | 5.4 | 5.4 | 5.4 | 5.4 |
| Plantacare 818UP | Coco-Glucoside | 51 - 53% | 2.0 | 2.0 | --- | --- |
| PLANTACARE® PS 10 | Sodium Laureth Sulfate (and) Lauryl Polyglucose | 33% | 9.0 | 9.0 | 10.5 | 10.5 |
| DC 245 | Cyclopentasiloxane | 100% | 1.0 | --- | 1.1 | --- |
| Synfluid PAO2 | Hydrogenated Dide-cene | 100% | --- | 0.8 | --- | 1.0 |
| Jaguar C162 | Hydroxypropyl Guar Hydroxypropyltrimonium Chloride | 100% | 0.2 | 0.2 | --- | --- |
| AquaCAT CG518 | Guar Hydroxypropy-Itrimonium Chloride | 10% | --- | --- | 2.5 | 2.5 |
| Arlypon TT | PEG/PPG-120/10 Trimethylolpropane Trioleate (and) Laureth-2 | 40 - 50% 40 - 50% | 1.0 | 1.5 | 1.0 | 1.5 |
| Perfume oil composition 1B | Fragrance | 100% | 0.3 | 0.3 | 0.3 | 0.3 |
| Na-Benzoate | Sodium Benzoate | 100% | 0.5 | 0.5 | 0.5 | 0.5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 67.7 | 67.4 | 64.4 | 64.0 |
| pH value | | | 5.0 | 4.9 | 5.2 | 5.1 |
| Viscosity [mPas] | | | 6600 | 8100 | 6900 | 9300 |

Shower bath 9 corresponds to shower bath 1, where the perfume oil composition 1B is replaced by the same amount of the perfume oil composition 2B.
Shower bath 10 corresponds to shower bath 2, where the perfume oil composition 1B is replaced by the same amount of the perfume oil composition 2B.
Shower bath 11 corresponds to shower bath 3, where the perfume oil composition 1B is replaced by the same amount of the perfume oil composition 2B.
Shower bath 12 corresponds to shower bath 4, where the perfume oil composition 1B is replaced by the same amount of the perfume oil composition 2B.
Shower bath 13 corresponds to shower bath 5, where the perfume oil composition 1B is replaced by the same amount of the perfume oil composition 2B.
Shower bath 14 corresponds to shower bath 6, where the perfume oil composition 1B is replaced by the same amount of the perfume oil composition 2B.
Shower bath 15 corresponds to shower bath 7, where the perfume oil composition 1B is replaced by the same amount of the perfume oil composition 2B.
Shower bath 16 corresponds to shower bath 8, where the perfume oil composition 1B is replaced by the same amount of the perfume oil composition 2B.
Shower bath 17 corresponds to shower bath 1, where the perfume oil composition 1B is replaced by the same amount of the perfume oil composition 3.
Shower bath 18 corresponds to shower bath 2, where the perfume oil composition 1B is replaced by the same amount of the perfume oil composition 3.
Shower bath 19 corresponds to shower bath 3, where the perfume oil composition 1B is replaced by the same amount of the perfume oil composition 3.
Shower bath 20 corresponds to shower bath 4, where the perfume oil composition 1B is replaced by the same amount of the perfume oil composition 3.
Shower bath 21 corresponds to shower bath 5, where the perfume oil composition 1B is replaced by the same amount of the perfume oil composition 3.
Shower bath 22 corresponds to shower bath 6, where the perfume oil composition 1B is replaced by the same amount of the perfume oil composition 3.
Shower bath 23 corresponds to shower bath 7, where the perfume oil composition 1B is replaced by the same amount of the perfume oil composition 3.
Shower bath 24 corresponds to shower bath 8, where the perfume oil composition 1B is replaced by the same amount of the perfume oil composition 3.
Shower bath 25 corresponds to shower bath 1, where the perfume oil composition 1B is replaced by the same amount of the perfume oil composition 4.
Shower bath 26 corresponds to shower bath 2, where the perfume oil composition 1B is replaced by the same amount of the perfume oil composition 4.
Shower bath 27 corresponds to shower bath 3, where the perfume oil composition 1B is replaced by the same amount of the perfume oil composition 4.
Shower bath 28 corresponds to shower bath 4, where the perfume oil composition 1B is replaced by the same amount of the perfume oil composition 4.
Shower bath 29 corresponds to shower bath 5, where the perfume oil composition 1B is replaced by the same amount of the perfume oil composition 4.
Shower bath 30 corresponds to shower bath 6, where the perfume oil composition 1B is replaced by the same amount of the perfume oil composition 4.
Shower bath 31 corresponds to shower bath 7, where the perfume oil composition 1B is replaced by the same amount of the perfume oil composition 4.
Shower bath 32 corresponds to shower bath 8, where the perfume oil composition 1B is replaced by the same amount of the perfume oil composition 4.
Shower bath 33 corresponds to shower bath 1, where the perfume oil composition 1B is replaced by the same amount of the perfume oil composition 5.
Shower bath 34 corresponds to shower bath 2, where the perfume oil composition 1B is replaced by the same amount of the perfume oil composition 5.
Shower bath 35 corresponds to shower bath 3, where the perfume oil composition 1B is replaced by the same amount of the perfume oil composition 5.
Shower bath 36 corresponds to shower bath 4, where the perfume oil composition 1B is replaced by the same amount of the perfume oil composition 5.
Shower bath 37 corresponds to shower bath 5, where the perfume oil composition 1B is replaced by the same amount of the perfume oil composition 5.
Shower bath 38 corresponds to shower bath 6, where the perfume oil composition 1B is replaced by the same amount of the perfume oil composition 5.
Shower bath 39 corresponds to shower bath 7, where the perfume oil composition 1B is replaced by the same amount of the perfume oil composition 5.
Shower bath 40 corresponds to shower bath 8, where the perfume oil composition 1B is replaced by the same amount of the perfume oil composition 5.
Shower bath 41 corresponds to shower bath 1, where the perfume oil composition 1B is replaced by the same amount of the perfume oil composition 6.
Shower bath 42 corresponds to shower bath 2, where the perfume oil composition 1B is replaced by the same amount of the perfume oil composition 6.
Shower bath 43 corresponds to shower bath 3, where the perfume oil composition 1B is replaced by the same amount of the perfume oil composition 6.
Shower bath 44 corresponds to shower bath 4, where the perfume oil composition 1B is replaced by the same amount of the perfume oil composition 6.
Shower bath 45 corresponds to shower bath 5, where the perfume oil composition 1B is replaced by the same amount of the perfume oil composition 6.
Shower bath 46 corresponds to shower bath 6, where the perfume oil composition 1B is replaced by the same amount of the perfume oil composition 6.
Shower bath 47 corresponds to shower bath 7, where the perfume oil composition 1B is replaced by the same amount of the perfume oil composition 6.
Shower bath 48 corresponds to shower bath 8, where the perfume oil composition 1B is replaced by the same amount of the perfume oil composition 6.
Shower bath 49 corresponds to shower bath 1, where the perfume oil composition 1B is replaced by the same amount of the perfume oil composition 7.
Shower bath 50 corresponds to shower bath 2, where the perfume oil composition 1B is replaced by the same amount of the perfume oil composition 7.
Shower bath 51 corresponds to shower bath 3, where the perfume oil composition 1B is replaced by the same amount of the perfume oil composition 7.
Shower bath 52 corresponds to shower bath 4, where the perfume oil composition 1B is replaced by the same amount of the perfume oil composition 7.
Shower bath 53 corresponds to shower bath 5, where the perfume oil composition 1B is replaced by the same amount of the perfume oil composition 7.
Shower bath 54 corresponds to shower bath 6, where the perfume oil composition 1B is replaced by the same amount of the perfume oil composition 7.
Shower bath 55 corresponds to shower bath 7, where the perfume oil composition 1B is replaced by the same amount of the perfume oil composition 7.
Shower bath 56 corresponds to shower bath 8, where the perfume oil composition 1B is replaced by the same amount of the perfume oil composition 7.
Shower bath 57 corresponds to shower bath 1, where the perfume oil composition 1B is replaced by the same amount of the perfume oil composition 8.
Shower bath 58 corresponds to shower bath 2, where the perfume oil composition 1B is replaced by the same amount of the perfume oil composition 8.
Shower bath 59 corresponds to shower bath 3, where the perfume oil composition 1B is replaced by the same amount of the perfume oil composition 8.
Shower bath 60 corresponds to shower bath 4, where the perfume oil composition 1B is replaced by the same amount of the perfume oil composition 8.
Shower bath 61 corresponds to shower bath 5, where the perfume oil composition 1B is replaced by the same amount of the perfume oil composition 8.
Shower bath 62 corresponds to shower bath 6, where the perfume oil composition 1B is replaced by the same amount of the perfume oil composition 8.
Shower bath 63 corresponds to shower bath 7, where the perfume oil composition 1B is replaced by the same amount of the perfume oil composition 8.
Shower bath 64 corresponds to shower bath 8, where the perfume oil composition 1B is replaced by the same amount of the perfume oil composition 8.
Shower bath 65 corresponds to shower bath 1, where the perfume oil composition 1B is replaced by the same amount of the perfume oil composition 9.
Shower bath 66 corresponds to shower bath 2, where the perfume oil composition 1B is replaced by the same amount of the perfume oil composition 9.
Shower bath 67 corresponds to shower bath 3, where the perfume oil composition 1B is replaced by the same amount of the perfume oil composition 9.
Shower bath 68 corresponds to shower bath 4, where the perfume oil composition 1B is replaced by the same amount of the perfume oil composition 9.
Shower bath 69 corresponds to shower bath 5, where the perfume oil composition 1B is replaced by the same amount of the perfume oil composition 9.
Shower bath 70 corresponds to shower bath 6, where the perfume oil composition 1B is replaced by the same amount of the perfume oil composition 9.
Shower bath 71 corresponds to shower bath 7, where the perfume oil composition 1B is replaced by the same amount of the perfume oil composition 9.
Shower bath 72 corresponds to shower bath 8, where the perfume oil composition 1B is replaced by the same amount of the perfume oil composition 9.
Shower bath 73 corresponds to shower bath 1, where the perfume oil composition 1B is replaced by the same amount of the perfume oil composition 10.
Shower bath 74 corresponds to shower bath 2, where the perfume oil composition 1B is replaced by the same amount of the perfume oil composition 10.
Shower bath 75 corresponds to shower bath 3, where the perfume oil composition 1B is replaced by the same amount of the perfume oil composition 10.
Shower bath 76 corresponds to shower bath 4, where the perfume oil composition 1B is replaced by the same amount of the perfume oil composition 10.
Shower bath 77 corresponds to shower bath 5, where the perfume oil composition 1B is replaced by the same amount of the perfume oil composition 10.
Shower bath 78 corresponds to shower bath 6, where the perfume oil composition 1B is replaced by the same amount of the perfume oil composition 10.
Shower bath 79 corresponds to shower bath 7, where the perfume oil composition 1B is replaced by the same amount of the perfume oil composition 10.
Shower bath 80 corresponds to shower bath 8, where the perfume oil composition 1B is replaced by the same amount of the perfume oil composition 10.
Shower bath 81 corresponds to shower bath 1, where the perfume oil composition 1B is replaced by the same amount of the perfume oil composition 11.
Shower bath 82 corresponds to shower bath 2, where the perfume oil composition 1B is replaced by the same amount of the perfume oil composition 11.
Shower bath 83 corresponds to shower bath 3, where the perfume oil composition 1B is replaced by the same amount of the perfume oil composition 11.
Shower bath 84 corresponds to shower bath 4, where the perfume oil composition 1B is replaced by the same amount of the perfume oil composition 11.
Shower bath 85 corresponds to shower bath 5, where the perfume oil composition 1B is replaced by the same amount of the perfume oil composition 11.
Shower bath 86 corresponds to shower bath 6, where the perfume oil composition 1B is replaced by the same amount of the perfume oil composition 11.
Shower bath 87 corresponds to shower bath 7, where the perfume oil composition 1B is replaced by the same amount of the perfume oil composition 11.
Shower bath 88 corresponds to shower bath 8, where the perfume oil composition 1B is replaced by the same amount of the perfume oil composition 11.
Shower bath 89 corresponds to shower bath 1, where the perfume oil composition 1B is replaced by the same amount of the perfume oil composition 12.
Shower bath 90 corresponds to shower bath 2, where the perfume oil composition 1B is replaced by the same amount of the perfume oil composition 12.
Shower bath 91 corresponds to shower bath 3, where the perfume oil composition 1B is replaced by the same amount of the perfume oil composition 12.
Shower bath 92 corresponds to shower bath 4, where the perfume oil composition 1B is replaced by the same amount of the perfume oil composition 12.
Shower bath 93 corresponds to shower bath 5, where the perfume oil composition 1B is replaced by the same amount of the perfume oil composition 12.
Shower bath 94 corresponds to shower bath 6, where the perfume oil composition 1B is replaced by the same amount of the perfume oil composition 12.
Shower bath 95 corresponds to shower bath 7, where the perfume oil composition 1B is replaced by the same amount of the perfume oil composition 12.
Shower bath 96 corresponds to shower bath 8, where the perfume oil composition 1B is replaced by the same amount of the perfume oil composition 12.
Shower bath 97 corresponds to shower bath 1, where the perfume oil composition 1B is replaced by the same amount of the perfume oil composition 13.
Shower bath 98 corresponds to shower bath 2, where the perfume oil composition 1B is replaced by the same amount of the perfume oil composition 13.
Shower bath 99 corresponds to shower bath 3, where the perfume oil composition 1B is replaced by the same amount of the perfume oil composition 13.
Shower bath 100 corresponds to shower bath 4, where the perfume oil composition 1B is replaced by the same amount of the perfume oil composition 13.
Shower bath 101 corresponds to shower bath 5, where the perfume oil composition 1B is replaced by the same amount of the perfume oil composition 13.
Shower bath 102 corresponds to shower bath 6, where the perfume oil composition 1B is replaced by the same amount of the perfume oil composition 13.
Shower bath 103 corresponds to shower bath 7, where the perfume oil composition 1B is replaced by the same amount of the perfume oil composition 13.
Shower bath 104 corresponds to shower bath 8, where the perfume oil composition 1B is replaced by the same amount of the perfume oil composition 13.
Shower bath 105 corresponds to shower bath 1, where the perfume oil composition 1B is replaced by the same amount of the perfume oil composition 14.
Shower bath 106 corresponds to shower bath 2, where the perfume oil composition 1B is replaced by the same amount of the perfume oil composition 14.
Shower bath 107 corresponds to shower bath 3, where the perfume oil composition 1B is replaced by the same amount of the perfume oil composition 14.
Shower bath 108 corresponds to shower bath 4, where the perfume oil composition 1B is replaced by the same amount of the perfume oil composition 14.
Shower bath 109 corresponds to shower bath 5, where the perfume oil composition 1B is replaced by the same amount of the perfume oil composition 14.
Shower bath 110 corresponds to shower bath 6, where the perfume oil composition 1B is replaced by the same amount of the perfume oil composition 14.
Shower bath 111 corresponds to shower bath 7, where the perfume oil composition 1B is replaced by the same amount of the perfume oil composition 14.
Shower bath 112 corresponds to shower bath 8, where the perfume oil composition 1B is replaced by the same amount of the perfume oil composition 14.

**Table 20: Hydro-alcoholic AP/Deo pump spray**

| **Component** | **INCI** | **amount%** |
|---|---|---|
| Ethanol, cosm. | Alcohol | 40.0 |
| water, demin. | Aqua | 37.5 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1.5 |
| Chlorhydrol (50% solut.) | Aluminium Chlorohydrate | 8.0 |
| Perfume oil composition 1B | | 1.0 |

Deo pump spray according to table 20, where the perfume oil composition 1B is replaced by the same amount of the perfume oil composition 2B.
Deo pump spray according to table 20, where the perfume oil composition 1B is replaced by the same amount of the perfume oil composition 3.
Deo pump spray according to table 20, where the perfume oil composition 1B is replaced by the same amount of the perfume oil composition 4.
Deo pump spray according to table 20, where the perfume oil composition 1B is replaced by the same amount of the perfume oil composition 5.
Deo pump spray according to table 20, where the perfume oil composition 1B is replaced by the same amount of the perfume oil composition 6.
Deo pump spray according to table 20, where the perfume oil composition 1B is replaced by the same amount of the perfume oil composition 7.
Deo pump spray according to table 20, where the perfume oil composition 1B is replaced by the same amount of the perfume oil composition 8.
Deo pump spray according to table 20, where the perfume oil composition 1B is replaced by the same amount of the perfume oil composition 9.
Deo pump spray according to table 20, where the perfume oil composition 1B is replaced by the same amount of the perfume oil composition 10.
Deo pump spray according to table 20, where the perfume oil composition 1B is replaced by the same amount of the perfume oil composition 11.
Deo pump spray according to table 20, where the perfume oil composition 1B is replaced by the same amount of the perfume oil composition 12.
Deo pump spray according to table 20, where the perfume oil composition 1B is replaced by the same amount of the perfume oil composition 13.
Deo pump spray according to table 20, where the perfume oil composition 1B is replaced by the same amount of the perfume oil composition 14.

**Table 21: Aerosol 1**

| **Component** | **INCI** | **amount %** |
|---|---|---|
| Ethanol, cosm. | Alcohol | 80.0 |
| water, demin. | Aqua | 16.7 |
| Propylene glycol | Propylene Glycol | 1.0 |
| Eumulgin® HRE 60 | PEG-60 Hydrogenated Castor Oil | 1.5 |
| alpha-Bisabolol | Bisabolol | 0.05 |
| Triclosan | Triclosan | 0.25 |
| perfume oil composition 1B | | 1.0 |

Aerosol 2 corresponds to aerosol 1, where the perfume oil composition 1B is replaced by the same amount of the perfume oil composition 2B.
Aerosol 3 corresponds to aerosol 1, where the perfume oil composition 1B is replaced by the same amount of the perfume oil composition 3.
Aerosol 4 corresponds to aerosol 1, where the perfume oil composition 1B is replaced by the same amount of the perfume oil composition 4.
Aerosol 5 corresponds to aerosol 1, where the perfume oil composition 1B is replaced by the same amount of the perfume oil composition 5.
Aerosol 6 corresponds to aerosol 1, where the perfume oil composition 1B is replaced by the same amount of the perfume oil composition 6.
Aerosol 7 corresponds to aerosol 1, where the perfume oil composition 1B is replaced by the same amount of the perfume oil composition 7.
Aerosol 8 corresponds to aerosol 1, where the perfume oil composition 1B is replaced by the same amount of the perfume oil composition 8.
Aerosol 9 corresponds to aerosol 1, where the perfume oil composition 1B is replaced by the same amount of the perfume oil composition 9.
Aerosol 10 corresponds to aerosol 1, where the perfume oil composition 1B is replaced by the same amount of the perfume oil composition 10.
Aerosol 11 corresponds to aerosol 1, where the perfume oil composition 1B is replaced by the same amount of the perfume oil composition 11.
Aerosol 12 corresponds to aerosol 1, where the perfume oil composition 1B is replaced by the same amount of the perfume oil composition 12.
Aerosol 13 corresponds to aerosol 1, where the perfume oil composition 1B is replaced by the same amount of the perfume oil composition 13.
Aerosol 14 corresponds to aerosol 1, where the perfume oil composition 1B is replaced by the same amount of the perfume oil composition 14.

**Table 22: Aqueous/alcoholic AP/Deo roll-on**

| **Component** | **INCI** | **amount %** |
|---|---|---|
| Ethanol, cosm. | Alcohol | 40.0 |
| water, demin. | Aqua | 37.0 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1,5 |
| Chlorhydrol (50% solut.) | Aluminium Chlorohydarte | 8.0 |
| Klucel M | Hydroxypropylcellulose | 0.5 |
| Perfume oil composition 1B | | 1.0 |

Aqueous/alcoholic AP/Deo roll-on according to table 22, where the perfume oil composition 1B is replaced by the same amount of the perfume oil composition 2B.
Aqueous/alcoholic AP/Deo roll-on according to table 22, where the perfume oil composition 1B is replaced by the same amount of the perfume oil composition 3.
Aqueous/alcoholic AP/Deo roll-on according to table 22, where the perfume oil composition 1B is replaced by the same amount of the perfume oil composition 4.
Aqueous/alcoholic AP/Deo roll-on according to table 22, where the perfume oil composition 1B is replaced by the same amount of the perfume oil composition 5.
Aqueous/alcoholic AP/Deo roll-on according to table 22, where the perfume oil composition 1B is replaced by the same amount of the perfume oil composition 6.
Aqueous/alcoholic AP/Deo roll-on according to table 22, where the perfume oil composition 1B is replaced by the same amount of the perfume oil composition 7.
Aqueous/alcoholic AP/Deo roll-on according to table 22, where the perfume oil composition 1B is replaced by the same amount of the perfume oil composition 8.
Aqueous/alcoholic AP/Deo roll-on according to table 22, where the perfume oil composition 1 B is replaced by the same amount of the perfume oil composition 9.
Aqueous/alcoholic AP/Deo roll-on according to table 22, where the perfume oil composition 1B is replaced by the same amount of the perfume oil composition 10.
Aqueous/alcoholic AP/Deo roll-on according to table 22, where the perfume oil composition 1B is replaced by the same amount of the perfume oil composition 11.
Aqueous/alcoholic AP/Deo roll-on according to table 22, where the perfume oil composition 1B is replaced by the same amount of the perfume oil composition 12.
Aqueous/alcoholic AP/Deo roll-on according to table 22, where the perfume oil composition 1B is replaced by the same amount of the perfume oil composition 13.
Aqueous/alcoholic AP/Deo roll-on according to table 22, where the perfume oil composition 1B is replaced by the same amount of the perfume oil composition 14.

**Table 23: Styling Gel Type "Out of Bed"**

| **Phase** | **Component** | **INCI** | **%** | **Function** |
|---|---|---|---|---|
| I | Alcool 96% (Prolabo) | Alcohol 96% | 12.00 | Solvent |
| | Luviskol VA 64 (BASF) | PVP/VA | 4.50 | Styling agent |
| | Polyox WSR-301 | PEG-90M | 0.25 | Styling agent |
| | Methocel E4M Premium EP (DOW) | Hydroxypropyl Methylcellulose | 0.60 | Thickener |
| | DEHYQUART® L 80 | Dicocoylethyl Hydroxy-ethylmonium Methosul-fate (and) Propylene Glycol | 0.60 | Active agent |
| II. | Eumulgin® HRE 60 | Hardened castor oil with approx. 60 mol EO | 1.00 | Solubilizer |
| | CETIOL® OE | Dicaprylyl Ether | 1.50 | Emollient |
| III. | Water, de-ionized | Aqua | a.d. | |
| IV. | HISPAGEL® 200 | Glycerin (and) Glyceryl Polyacrylate | 36.70 | Thickener |
| V. | Perfume oil composition 1B | | 0.02 | |
| » | pH-value | | 6.8 | |
| » | Viscosity (mPas) Brookfield RVT 23°C spindle 5, 10rpm | | 220.000 mPas | |

Styling gel according to table 23, where the perfume oil composition 1B is replaced by the same amount of the perfume oil composition 2B.
Styling gel according to table 23, where the perfume oil composition 1B is replaced by the same amount of the perfume oil composition 3.
Styling gel according to table 23, where the perfume oil composition 1B is replaced by the same amount of the perfume oil composition 4.
Styling gel according to table 23, where the perfume oil composition 1B is replaced by the same amount of the perfume oil composition 5.
Styling gel according to table 23, where the perfume oil composition 1B is replaced by the same amount of the perfume oil composition 6.
Styling gel according to table 23, where the perfume oil composition 1B is replaced by the same amount of the perfume oil composition 7.
Styling gel according to table 23, where the perfume oil composition 1B is replaced by the same amount of the perfume oil composition 8.
Styling gel according to table 23, where the perfume oil composition 1B is replaced by the same amount of the perfume oil composition 9.
Styling gel according to table 23, where the perfume oil composition 1B is replaced by the same amount of the perfume oil composition 10.
Styling gel according to table 23, where the perfume oil composition 1B is replaced by the same amount of the perfume oil composition 11.
Styling gel according to table 23, where the perfume oil composition 1B is replaced by the same amount of the perfume oil composition 12.
Styling gel according to table 23, where the perfume oil composition 1B is replaced by the same amount of the perfume oil composition 13.
Styling gel according to table 23, where the perfume oil composition 1B is replaced by the same amount of the perfume oil composition 14.

**Table 24: Shaving Foam**

| **Phase** | **Component** | **INCI** | **%** | **Function** |
|---|---|---|---|---|
| | CUTINA® FS 45 | Stearic Acid (and) Palmitic Acid | 7.6 | Surfactant (foam consistency) |
| | PLANTACARE® 1200 UP | Lauryl Glucoside | 6.0 | Surfactant (inhibitory effect) |
| | MONOMULS® 90-0 18 | Glyceryl Oleate | 1.2 | W/O emulsifier (moisturizing) |
| | EUMULGIN® O20 S | Oleth-20 | 1.2 | O/W-emulsifier (stability) |
| | CETIOL® CC | Dicaprylyl Carbonate | 2.0 | Emollient (low viscosity) |
| | DEHYQUART® SP | Quaternium-52 | 0.5 | Surfactant (inhibitor) |
| | TEA (99%) | | 4.0 | Neutralizer (for Cutina FS 45) |
| | Glycerin | Glycerin | 3.0 | Humectant |
| | Propylene Glycol | Propylene Glycol | 3.0 | Humectant (low viscosiy) |
| | Emulgin® HRE 60 | Hardened castor oil with approx. 60 mol EO | 2.0 | Solubilizer |
| | D-Panthenol | Panthenol | 0.2 | Care additive |
| | Phenonip | Phenoxyethanol (and) Parabens | 0.5 | Preservative |
| | Perfume oil composition 1B | | 0.3 | Fragrance |
| | Distilled or Deionized Water | Aqua | ad 100 | |

Shaving foam according to table 24, where the perfume oil composition 1B is replaced by the same amount of the perfume oil composition 2B.
Shaving foam according to table 24, where the perfume oil composition 1B is replaced by the same amount of the perfume oil composition 3.
Shaving foam according to table 24, where the perfume oil composition 1B is replaced by the same amount of the perfume oil composition 4.
Shaving foam according to table 24, where the perfume oil composition 1B is replaced by the same amount of the perfume oil composition 5.
Shaving foam according to table 24, where the perfume oil composition 1B is replaced by the same amount of the perfume oil composition 6.
Shaving foam according to table 24, where the perfume oil composition 1B is replaced by the same amount of the perfume oil composition 7.
Shaving foam according to table 24, where the perfume oil composition 1B is replaced by the same amount of the perfume oil composition 8.
Shaving foam according to table 24, where the perfume oil composition 1B is replaced by the same amount of the perfume oil composition 9.
Shaving foam according to table 24, where the perfume oil composition 1B is replaced by the same amount of the perfume oil composition 10.
Shaving foam according to table 24, where the perfume oil composition 1B is replaced by the same amount of the perfume oil composition 11.
Shaving foam according to table 24, where the perfume oil composition 1B is replaced by the same amount of the perfume oil composition 12.
Shaving foam according to table 24, where the perfume oil composition 1B is replaced by the same amount of the perfume oil composition 13.
Shaving foam according to table 24, where the perfume oil composition 1B is replaced by the same amount of the perfume oil composition 14.

**Table 25: Sensitive skin Baby shampoo**

| | **Ingredients** | **INCI / Chemistry** | **%** | **Function** |
|---|---|---|---|---|
| I. | PLANTAPON® LGC SORB | Sodium Lauryl Glucose Carboxylate (and) Lauryl Glucoside | 28.00 | Surfactant, cleaning |
| | ARLYPON® TT | PEG/PPG-120/10 Trimethylolpropane Trioleate (and) Laureth-2 | 2.00 | Thickener |
| | DEHYTON® MC | Sodium Cocoamphoacetate | 6.00 | Surfactant |
| | EUMULGIN® SML 20 | Polysorbate 20 | 3.00 | Solubilizer |
| | LAMESOFT® PO 65 | Coco-Glucoside (and) Glyceryl Oleate | 2.20 | Lipid layer enhancer |
| | MELHYDRAN™ LS 4420 | Honey Extract | 1.00 | Active ingredient |
| | Perfume oil composition 1B | Perfume | 0.20 | Perfume |
| | Dermosoft 1388 (Dr. Straetmans) | Water (and) Glycerin (and) Sodium Levulinate (and) Sodium Anisate | 4.00 | Active ingredient |
| | Water, demin. | Aqua | 53.60 | |

Sensitive skin baby shampoo according to table 25, where the perfume oil composition 1 B is replaced by the same amount of the perfume oil composition 2B.
Sensitive skin baby shampoo according to table 25, where the perfume oil composition 1 B is replaced by the same amount of the perfume oil composition 3.
Sensitive skin baby shampoo according to table 25, where the perfume oil composition 1 B is replaced by the same amount of the perfume oil composition 4.
Sensitive skin baby shampoo according to table 25, where the perfume oil composition 1B is replaced by the same amount of the perfume oil composition 5.
Sensitive skin baby shampoo according to table 25, where the perfume oil composition 1B is replaced by the same amount of the perfume oil composition 6.
Sensitive skin baby shampoo according to table 25, where the perfume oil composition 1B is replaced by the same amount of the perfume oil composition 7.
Sensitive skin baby shampoo according to table 25, where the perfume oil composition 1B is replaced by the same amount of the perfume oil composition 8.
Sensitive skin baby shampoo according to table 25, where the perfume oil composition 1B is replaced by the same amount of the perfume oil composition 9.
Sensitive skin baby shampoo according to table 25, where the perfume oil composition 1B is replaced by the same amount of the perfume oil composition 10.
Sensitive skin baby shampoo according to table 25, where the perfume oil composition 1B is replaced by the same amount of the perfume oil composition 11.
Sensitive skin baby shampoo according to table 25, where the perfume oil composition 1B is replaced by the same amount of the perfume oil composition 12.
Sensitive skin baby shampoo according to table 25, where the perfume oil composition 1B is replaced by the same amount of the perfume oil composition 13.
Sensitive skin baby shampoo according to table 25, where the perfume oil composition 1B is replaced by the same amount of the perfume oil composition 14.

**Table 26: Body wash for Sensitive Skin**

| | **Ingredients** | **INCI / Chemistry** | **%** | **Function** |
|---|---|---|---|---|
| I. | Water, demin. | Aqua | 70.90 | |
| | TEXAPON® N 70 | Sodium Laureth Sulfate | 13.00 | Surfactant |
| | PLANTACARE® 818 UP | Coco-Glucoside | 3.00 | Surfactant |
| | LAMESOFT® PO 65 | Coco-Glucoside (and) Glyceryl Oleate | 5.00 | Active, skin conditioning |
| | Perfume oil composition 1B | Fragrance | 0.15 | Perfume |
| | Sodium Benzoate | Sodium Benzoate | 0.55 | Preservative |
| II. | DEHYQUART® CC7 BZ | Polyquaternium-7 | 2.00 | Active, hair conditioning |
| III. | MELHYDRAN™ LS 4420 | Honey Extract | 1.00 | Active, moisturizing |
| | ARLYPON® TT | PEG/PPG-120/10 Trimethylolpropane Trioleate | 0.50 | Thickener |
| | | (and) Laureth-2 | | |
| | DEHYTON® PK 45 | Cocamidopropyl Betaine | 3.80 | Surfactant |
| IV. | Sodium Chloride | Sodium Chloride | 0.10 | Agent, thickening |

Body wash for sensitive skin according to table 26, where the perfume oil composition 1B is replaced by the same amount of the perfume oil composition 2B.
Body wash for sensitive skin according to table 26, where the perfume oil composition 1B is replaced by the same amount of the perfume oil composition 3.
Body wash for sensitive skin according to table 26, where the perfume oil composition 1B is replaced by the same amount of the perfume oil composition 4.
Body wash for sensitive skin according to table 26, where the perfume oil composition 1B is replaced by the same amount of the perfume oil composition 5.
Body wash for sensitive skin according to table 26, where the perfume oil composition 1B is replaced by the same amount of the perfume oil composition 6.
Body wash for sensitive skin according to table 26, where the perfume oil composition 1B is replaced by the same amount of the perfume oil composition 7.
Body wash for sensitive skin according to table 26, where the perfume oil composition 1B is replaced by the same amount of the perfume oil composition 8.
Body wash for sensitive skin according to table 26, where the perfume oil composition 1B is replaced by the same amount of the perfume oil composition 9.
Body wash for sensitive skin according to table 26, where the perfume oil composition 1B is replaced by the same amount of the perfume oil composition 10.
Body wash for sensitive skin according to table 26, where the perfume oil composition 1B is replaced by the same amount of the perfume oil composition 11.
Body wash for sensitive skin according to table 26, where the perfume oil composition 1B is replaced by the same amount of the perfume oil composition 12.
Body wash for sensitive skin according to table 26, where the perfume oil composition 1B is replaced by the same amount of the perfume oil composition 13.
Body wash for sensitive skin according to table 26, where the perfume oil composition 1B is replaced by the same amount of the perfume oil composition 14.

**Table 27: Gloss Enhancing Shampoo for Sensitive Scalp**

| **Ingredients** | | **INCI / Chemistry** | **%** | **Function** |
|---|---|---|---|---|
| I. | Water, demin. | Aqua | 71.60 | |

| | **Ingredients** | **INCI / Chemistry** | **%** | **Function** |
|---|---|---|---|---|
| | Luviquat Ultra Care (BASF) | Polyquaternium-44 | 1.50 | Active, hair conditioning |
| II. | Sodium Benzoate | Sodium Benzoate | 0.55 | Preservative |
| III. | TEXAPON® N 70 | Sodium Laureth Sulfate | 14.30 | Surfactant |
| | DEHYTON® PK 45 | Cocamidopropyl Betaine | 5.40 | Surfactant |
| | MELHYDRAN™ LS 4420 | Honey Extract | 1.00 | Active, moisturizing |
| | Perfume oil composition 1B | Fragrance | 0.15 | Perfume |
| IV. | DEHYDOL® LS 2 DEO N | Laureth-2 | 1.00 | Thickener |
| V. | LAMESOFT® CARE | PEG-4 Distearyl Ether | 3.00 | Active, hair conditioning |
| | | (and) Sodium Laureth sulfate (and) Distearyl Ether | | |
| | | (and) Dicaprylyl Ether | | |
| VI. | Sodium Chloride | Sodium Chloride | 1.50 | Agent, thickening |

Gloss enhancing shampoo for sensitive scalp according to table 27, where the perfume oil composition 1B is replaced by the same amount of the perfume oil composition 2B.
Gloss enhancing shampoo for sensitive scalp according to table 27, where the perfume oil composition 1B is replaced by the same amount of the perfume oil composition 3.
Gloss enhancing shampoo for sensitive scalp according to table 27, where the perfume oil composition 1B is replaced by the same amount of the perfume oil composition 4.
Gloss enhancing shampoo for sensitive scalp according to table 27, where the perfume oil composition 1B is replaced by the same amount of the perfume oil composition 5.
Gloss enhancing shampoo for sensitive scalp according to table 27, where the perfume oil composition 1B is replaced by the same amount of the perfume oil composition 6.
Gloss enhancing shampoo for sensitive scalp according to table 27, where the perfume oil composition 1B is replaced by the same amount of the perfume oil composition 7.
Gloss enhancing shampoo for sensitive scalp according to table 27, where the perfume oil composition 1B is replaced by the same amount of the perfume oil composition 8.
Gloss enhancing shampoo for sensitive scalp according to table 27, where the perfume oil composition 1B is replaced by the same amount of the perfume oil composition 9.
Gloss enhancing shampoo for sensitive scalp according to table 27, where the perfume oil composition 1B is replaced by the same amount of the perfume oil composition 10.
Gloss enhancing shampoo for sensitive scalp according to table 27, where the perfume oil composition 1B is replaced by the same amount of the perfume oil composition 11.
Gloss enhancing shampoo for sensitive scalp according to table 27, where the perfume oil composition 1B is replaced by the same amount of the perfume oil composition 12.
Gloss enhancing shampoo for sensitive scalp according to table 27, where the perfume oil composition 1B is replaced by the same amount of the perfume oil composition 13.
Gloss enhancing shampoo for sensitive scalp according to table 27, where the perfume oil composition 1B is replaced by the same amount of the perfume oil composition 14.

**Table 28: Deo Stick**

| | **Ingredients** | **INCI / Chemistry** | **%** | **Function** |
|---|---|---|---|---|
| I. | LANETTE® 18 DEO | Stearyl Alcohol | 18.50 | Consistency factor |
| | CUTINA® HR POWDER | Hydrogenated Castor Oil | 4.50 | Consistency factor |
| | CUTINA® CP | Cetyl Palmitate | 25.00 | Emollient |
| | CETIOL® MM | Myristyl Myristate | 10.00 | Emollient |
| | CETIOL® RLF | Caprylyl Caprylate/Caprate | 5.00 | Emollient |
| | MYRITOL® 312 | Caprylic/Capric Triglyceride | 29.30 | Emollient |
| | HYDAGEN® C.A.T. | Triethyl Citrate | 5.00 | Active, antiperspirant |
| II. | COSMEDIA® SILC | Silica | 2.00 | Skin feel modifier |
| | PHYTOSOOTHE™ LS 9766 | Brassica Campestris (Rapeseed) Sterols (and) Cetearyl Alcohol | 0.50 | Active ingredient |
| III. | Perfume oil composition 1B | Fragrance | 0.20 | Perfume |

Deo stick according to table 28, where the perfume oil composition 1B is replaced by the same amount of the perfume oil composition 2B.
Deo stick according to table 28, where the perfume oil composition 1B is replaced by the same amount of the perfume oil composition 3.
Deo stick according to table 28, where the perfume oil composition 1B is replaced by the same amount of the perfume oil composition 4.
Deo stick according to table 28, where the perfume oil composition 1B is replaced by the same amount of the perfume oil composition 5.
Deo stick according to table 28, where the perfume oil composition 1B is replaced by the same amount of the perfume oil composition 6.
Deo stick according to table 28, where the perfume oil composition 1B is replaced by the same amount of the perfume oil composition 7.
Deo stick according to table 28, where the perfume oil composition 1B is replaced by the same amount of the perfume oil composition 8.
Deo stick according to table 28, where the perfume oil composition 1B is replaced by the same amount of the perfume oil composition 9.
Deo stick according to table 28, where the perfume oil composition 1B is replaced by the same amount of the perfume oil composition 10.
Deo stick according to table 28, where the perfume oil composition 1B is replaced by the same amount of the perfume oil composition 11.
Deo stick according to table 28, where the perfume oil composition 1B is replaced by the same amount of the perfume oil composition 12.
Deo stick according to table 28, where the perfume oil composition 1B is replaced by the same amount of the perfume oil composition 13.
Deo stick according to table 28, where the perfume oil composition 1B is replaced by the same amount of the perfume oil composition 14.

**Table 29: Baby Wipe**

| | **Ingredients** | **INCI / Chemistry** | **%** | **Function** |
|---|---|---|---|---|
| I. | Water, demin. | Water | 95.25 | |
| | Sodium Benzoate | Sodium Benzoate | 0.50 | Preservative |
| II. | EMULGADE® CPE | Vegetable Oil (and) Glycerin (and) Lauryl Glucoside (and) | | Emulsion base (O/W) |
| | | Polyglyceryl-2-Dipolyhydroxystearate (and) Glyceryl Oleate | 4.00 | |
| | LIPOFRUCTYL™ ARGAN LS 9779 | Argania Spinosa Kernel Oil | 0.10 | Active ingredient |
| | Perfume oil composition 1B | Fragrance | 0.10 | Perfume |
| III. | Citric Acid (50 %) | Citric Acid | 0.15 | Agent, pH adjusting |

Baby wipe according to table 29, where the perfume oil composition 1B is replaced by the same amount of the perfume oil composition 2B.
Baby wipe according to table 29, where the perfume oil composition 1B is replaced by the same amount of the perfume oil composition 3.
Baby wipe according to table 29, where the perfume oil composition 1B is replaced by the same amount of the perfume oil composition 4.
Baby wipe according to table 29, where the perfume oil composition 1B is replaced by the same amount of the perfume oil composition 5.
Baby wipe according to table 29, where the perfume oil composition 1B is replaced by the same amount of the perfume oil composition 6.
Baby wipe according to table 29, where the perfume oil composition 1B is replaced by the same amount of the perfume oil composition 7.
Baby wipe according to table 29, where the perfume oil composition 1B is replaced by the same amount of the perfume oil composition 8.
Baby wipe according to table 29, where the perfume oil composition 1B is replaced by the same amount of the perfume oil composition 9.
Baby wipe according to table 29, where the perfume oil composition 1B is replaced by the same amount of the perfume oil composition 10.
Baby wipe according to table 29, where the perfume oil composition 1B is replaced by the same amount of the perfume oil composition 11.
Baby wipe according to table 29, where the perfume oil composition 1B is replaced by the same amount of the perfume oil composition 12.
Baby wipe according to table 29, where the perfume oil composition 1B is replaced by the same amount of the perfume oil composition 13.
Baby wipe according to table 29, where the perfume oil composition 1B is replaced by the same amount of the perfume oil composition 14.

**Table 30: After shave balm**

| **Ingredients** | | **INCI** | **%** | **Function** |
|---|---|---|---|---|
| I | Myritol® 331 | Cocoglycerides | 2.50 | Emollient |
| | Cetiol® Sensoft | Propylheptyl Caprylate | 5.00 | Emollient |
| | Cetiol® C5 | Coco-Caprylate | 2.50 | Emollient |
| | Cosmedia® Triple C | Polyquaternium-37 (and) Dicaprylyl Carbonate (and) Lauryl Glucoside | 1.50 | Cationic Polymer |
| II | Deionized Water | Aqua | 84.80 | |
| | Glycerine | Glycerin | 1.00 | Humectant |
| III | Anasensyl™ LS 9322 | Mannitol (and) Ammonium Glycyrrhizate (and) Caffeine (and) Zinc Gluconate (and) Aesculus Hippocastanum Extract | 1.00 | Soothing Active |
| IV | Perfume oil composition 1B | Fragrance | 0.10 | Perfume |
| V | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| VI | NaOH (Aq. Sol. 25%) | Sodium Hydroxide | 0.10 | Neutralizing Agent |

After shave balm according to table 30, where the perfume oil composition 1B is replaced by the same amount of the perfume oil composition 2B.
After shave balm according to table 30, where the perfume oil composition 1B is replaced by the same amount of the perfume oil composition 3.
After shave balm according to table 30, where the perfume oil composition 1B is replaced by the same amount of the perfume oil composition 4.
After shave balm according to table 30, where the perfume oil composition 1B is replaced by the same amount of the perfume oil composition 5.
After shave balm according to table 30, where the perfume oil composition 1B is replaced by the same amount of the perfume oil composition 6.
After shave balm according to table 30, where the perfume oil composition 1B is replaced by the same amount of the perfume oil composition 7.
After shave balm according to table 30, where the perfume oil composition 1B is replaced by the same amount of the perfume oil composition 8.
After shave balm according to table 30, where the perfume oil composition 1B is replaced by the same amount of the perfume oil composition 9.
After shave balm according to table 30, where the perfume oil composition 1B is replaced by the same amount of the perfume oil composition 10.
After shave balm according to table 30, where the perfume oil composition 1B is replaced by the same amount of the perfume oil composition 11.
After shave balm according to table 30, where the perfume oil composition 1B is replaced by the same amount of the perfume oil composition 12.
After shave balm according to table 30, where the perfume oil composition 1B is replaced by the same amount of the perfume oil composition 13.
After shave balm according to table 30, where the perfume oil composition 1B is replaced by the same amount of the perfume oil composition 14.

**Table 31: Face Gel**

| **Ingredients** | | **INCI** | **%** | **Function** |
|---|---|---|---|---|
| I | Deionized Water | Aqua | 71.65 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 1.20 | Emulsifying Polymer |
| III | Cegesoft® PFO | Passiflora Incarnata Oil | 2.00 | Emollient |
| | Uvinul MC 80 (BASF) | Ethylhexyl Methoxycinnamate | 2.00 | UVB Filter |
| | KF 96, 100 cs (Shin Etsu) | Dimethicone | 2.00 | Emollient |
| IV | Cetiol® CC | Dicaprylyl Carbonate | 5.00 | Emollient |
| | Cetiol® C5 | Coco-Caprylate | 4.00 | Emollient |
| | Uvinul A Plus Granular (BASF) | Diethylamino Hydroxybenzoyl Hexyl Benzoate | 0.50 | UVA Filter |
| | Cegesoft® SBE | Butyrospermum Parkii | 1.50 | Emollient |
| | Eumulgin® SG | Sodium Stearoyl Glutamate | 0.10 | O/W Emulsifier |
| V | Deionized Water | Aqua | 5.00 | |
| | Skinasensyl™ PW LS 9852 | Mannitol (and) Sodium Citrate (and) Acetyl Tetrapeptide-15 | 0.30 | Soothing Active |
| VI | Covi-ox® T70C | Tocopherol | 0.10 | Antioxidant |
| | Perfume oil composition 1B | Fragrance | 0.10 | Perfume |
| VII | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face gel according to table 31, where the perfume oil composition 1B is replaced by the same amount of the perfume oil composition 2B.
Face gel according to table 31, where the perfume oil composition 1B is replaced by the same amount of the perfume oil composition 3.
Face gel according to table 31, where the perfume oil composition 1B is replaced by the same amount of the perfume oil composition 4.
Face gel according to table 31, where the perfume oil composition 1B is replaced by the same amount of the perfume oil composition 5.
Face gel according to table 31, where the perfume oil composition 1B is replaced by the same amount of the perfume oil composition 6.
Face gel according to table 31, where the perfume oil composition 1B is replaced by the same amount of the perfume oil composition 7.
Face gel according to table 31, where the perfume oil composition 1B is replaced by the same amount of the perfume oil composition 8.
Face gel according to table 31, where the perfume oil composition 1B is replaced by the same amount of the perfume oil composition 9.
Face gel according to table 31, where the perfume oil composition 1B is replaced by the same amount of the perfume oil composition 10.
Face gel according to table 31, where the perfume oil composition 1B is replaced by the same amount of the perfume oil composition 11.
Face gel according to table 31, where the perfume oil composition 1B is replaced by the same amount of the perfume oil composition 12.
Face gel according to table 31, where the perfume oil composition 1B is replaced by the same amount of the perfume oil composition 13.
Face gel according to table 31, where the perfume oil composition 1B is replaced by the same amount of the perfume oil composition 14.

**Table 32: Face Day Care Cream**

| **Ingredients** | | **INCI** | **%** | **Function** |
|---|---|---|---|---|
| I | Deionized Water | Aqua | 69.55 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 0.80 | Emulsifying Polymer |
| III | Emulgade® PL 68/50 | Cetearyl Glucoside (and) Cetearyl Alcohol | 2.00 | Self-Emulsifying Base |
| | Eumulgin® SG | Sodium Stearoyl Glutamate | 0.50 | O/W Emulsifier |
| | Monomuls® 90-0-18 | Glyceryl Oleate | 2.00 | W/O Emulsifier |
| | Cutina® GMS-V | Glyceryl Stearate | 2.00 | Consistency Giving Factor |
| | Cetiol® Sensoft | Propylheptyl Caprylate | 5.00 | Emollient |
| | Myritol® 331 | Cocoglycerides | 3.00 | Emollient |
| | Cegesoft® PFO | Passiflora Incarnata Oil | 3.00 | Emollient |
| | Lipodermol™ LS 8656 | Octyldodecanol (and) Lecithin (and) Arachidyl Propionate (and) Tocopheryl Acetate (and) Retinyl Palmitate (and) Ethyl Linoleate (and) | 1.00 | Anti-Ageing Active |
| | | Ethyl Linolenate (and) Ethyl Oleate | | |
| | Generol® R | Brassica Campestris (Rapeseed) Sterols | 1.00 | Anti-inflammatory Active |
| IV | Sphingoceryl™ WS LS 9859 | Aqua (and) Glycerin (and) Helianthus Annuus Seed Extract (and) Decyl Glucoside | 1.00 | Moisturizing Active |
| | Deionized Water | Aqua | 4.00 | |
| | Perfume oil composition 1B | Fragrance | 0.10 | Perfume |
| V | Covi-ox® T70C | Tocopherol | 0.50 | Antioxidant |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face day care cream according to table 32, where the perfume oil composition 1B is replaced by the same amount of the perfume oil composition 2B.
Face day care cream according to table 32, where the perfume oil composition 1B is replaced by the same amount of the perfume oil composition 3.
Face day care cream according to table 32, where the perfume oil composition 1B is replaced by the same amount of the perfume oil composition 4.
Face day care cream according to table 32, where the perfume oil composition 1B is replaced by the same amount of the perfume oil composition 5.
Face day care cream according to table 32, where the perfume oil composition 1B is replaced by the same amount of the perfume oil composition 6.
Face day care cream according to table 32, where the perfume oil composition 1B is replaced by the same amount of the perfume oil composition 7.
Face day care cream according to table 32, where the perfume oil composition 1B is replaced by the same amount of the perfume oil composition 8.
Face day care cream according to table 32, where the perfume oil composition 1B is replaced by the same amount of the perfume oil composition 9.
Face day care cream according to table 32, where the perfume oil composition 1B is replaced by the same amount of the perfume oil composition 10.
Face day care cream according to table 32, where the perfume oil composition 1B is replaced by the same amount of the perfume oil composition 11.
Face day care cream according to table 32, where the perfume oil composition 1B is replaced by the same amount of the perfume oil composition 12.
Face day care cream according to table 32, where the perfume oil composition 1B is replaced by the same amount of the perfume oil composition 13.
Face day care cream according to table 32, where the perfume oil composition 1B is replaced by the same amount of the perfume oil composition 14.

**Table 33: Face Cleanser**

| **Ingredients** | | **INCI** | **%** | **Function** |
|---|---|---|---|---|
| I | Deionized Water | Aqua | 80.15 | |
| | EDETA B Powder (BASF) | Tetrasodium EDTA | 0.05 | Complexing Agent |
| | Eumulgin® SG | Sodium Stearoyl Glutamate | 0.30 | O/W Emulsifier |
| II | Cosmedia® SP | Sodium Polyacrylate | 1.20 | Emulsifier |
| III | Isopropylpalmitate | Isopropyl palmitate | 5.00 | Emollient |
| | Cetiol® CC | Dicaprylyl Carbonate | 3.00 | Emollient |
| | Cetiol® C5 | Coco-Caprylate | 7.00 | Emollient |
| IV | Plantacare® 818 UP | Coco Glucoside | 0.75 | Non Ionic Surfactant |
| V | Indinyl® CA LS 8998 | Water (and) Cassia Angustifolia Seed Polysaccharide | 0.50 | Smoothing and Moisturizing Active |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| | VII NaOH (Aq. Sol. 25%) | Sodium Hydroxide | 0.45 | Neutralizing Agent |

Face cleanser according to table 33, where the perfume oil composition 1B is replaced by the same amount of the perfume oil composition 2B.
Face cleanser according to table 33, where the perfume oil composition 1B is replaced by the same amount of the perfume oil composition 3.
Face cleanser according to table 33, where the perfume oil composition 1B is replaced by the same amount of the perfume oil composition 4.
Face cleanser according to table 33, where the perfume oil composition 1B is replaced by the same amount of the perfume oil composition 5.
Face cleanser according to table 33, where the perfume oil composition 1B is replaced by the same amount of the perfume oil composition 6.
Face cleanser according to table 33, where the perfume oil composition 1B is replaced by the same amount of the perfume oil composition 7.
Face cleanser according to table 33, where the perfume oil composition 1B is replaced by the same amount of the perfume oil composition 8.
Face cleanser according to table 33, where the perfume oil composition 1B is replaced by the same amount of the perfume oil composition 9.
Face cleanser according to table 33, where the perfume oil composition 1B is replaced by the same amount of the perfume oil composition 10.
Face cleanser according to table 33, where the perfume oil composition 1B is replaced by the same amount of the perfume oil composition 11.
Face cleanser according to table 33, where the perfume oil composition 1B is replaced by the same amount of the perfume oil composition 12.
Face cleanser according to table 33, where the perfume oil composition 1B is replaced by the same amount of the perfume oil composition 13.
Face cleanser according to table 33, where the perfume oil composition 1B is replaced by the same amount of the perfume oil composition 14.

**Table 34: Sun Care SPF50+, Sprayable Lotion**

| | **Ingredients** | **INCI / Chemistry** | **%** | **Function** |
|---|---|---|---|---|
| I. | DEHYMULS® PGPH | Polyglyceryl-2 Dipolyhydroxystearate | 4.00 | Emulsifier (W/O) |
| | CETIOL® B | Dibutyl Adipate | 8.00 | Emollient |
| | MYRITOL® 331 | Cocoglycerides | 5.00 | Emollient |
| | Tinosorb S (BASF) | Bis-Ethylhexyloxyphenol Methoxyphenyl Triazine | 3.00 | UV filter, UV-A + UV-B |
| | Uvinul T 150 ( BASF) | Ethylhexyl Triazone | 2.50 | UV filter, UV-B |
| | Uvinul A Plus (BASF) | Diethylamino Hydroxybenzoyl Hexyl Benzoate | 10.00 | UV filter, UV-A |
| | Uvinul MC 80 (BASF) | Ethylhexyl Methoxycinnamate | 10.00 | UV filter, UV-B |
| | Sensiva SC 50 (Schülke) | Ethylhexylglycerin | 0.50 | Preservative |
| II. | Water, demin. | Water | 45.55 | |
| | Glycerin | Glycerin | 3.00 | Agent, humectant |
| | EDETA BD (BASF) | Disodium EDTA | 0.05 | Chelating agent |
| | Euxyl PE 9010 (Schülke) | Phenoxyethanol, Ethylhexylglycerin | 1.00 | Preservative |
| | Keltrol CG-T (CP Kel-co) | Xanthan Gum | 0.10 | Agent, thickening |
| | Veegum Ultra (RT Vanderbilt, Inc.) | Magnesium Aluminum Silicate | 2.00 | Stabilizer |
| III. | PLANTAPON® LGC SORB | Sodium Lauryl Glucose Carboxylate (and) Lauryl Glucoside | 1.50 | Surfactant |
| IV. | Eusolex T 2000 (Merck) | Titanium Dioxide and Alumina and Simethicone | 3.5 | UV filter, UV-A + UV-B |
| V. | DN-AGE™ PW LS 9827 | Cassia Alata Leaf Extract (and) Maltodextrin | 0.10 | Active ingredient |
| | Perfume oil composition 1B | Fragrance | 0.20 | Perfume |

Sun Care SPF50+, sprayable lotion according to table 34, where the perfume oil composition 1B is replaced by the same amount of the perfume oil composition 2B.
Sun Care SPF50+, sprayable lotion according to table 34, where the perfume oil composition 1B is replaced by the same amount of the perfume oil composition 3.
Sun Care SPF50+, sprayable lotion according to table 34, where the perfume oil composition 1B is replaced by the same amount of the perfume oil composition 4.
Sun Care SPF50+, sprayable lotion according to table 34, where the perfume oil composition 1B is replaced by the same amount of the perfume oil composition 5.
Sun Care SPF50+, sprayable lotion according to table 34, where the perfume oil composition 1B is replaced by the same amount of the perfume oil composition 6.
Sun Care SPF50+, sprayable lotion according to table 34, where the perfume oil composition 1B is replaced by the same amount of the perfume oil composition 7.
Sun Care SPF50+, sprayable lotion according to table 34, where the perfume oil composition 1B is replaced by the same amount of the perfume oil composition 8.
Sun Care SPF50+, sprayable lotion according to table 34, where the perfume oil composition 1B is replaced by the same amount of the perfume oil composition 9.
Sun Care SPF50+, sprayable lotion according to table 34, where the perfume oil composition 1B is replaced by the same amount of the perfume oil composition 10.
Sun Care SPF50+, sprayable lotion according to table 34, where the perfume oil composition 1B is replaced by the same amount of the perfume oil composition 11.
Sun Care SPF50+, sprayable lotion according to table 34, where the perfume oil composition 1 B is replaced by the same amount of the perfume oil composition 12.
Sun Care SPF50+, sprayable lotion according to table 34, where the perfume oil composition 1B is replaced by the same amount of the perfume oil composition 13.
Sun Care SPF50+, sprayable lotion according to table 34, where the perfume oil composition 1B is replaced by the same amount of the perfume oil composition 14.

**Table 35: Hand dish cleaner, regular**

| **Hand dish cleaner, regular** | | |
|---|---|---|
| **Component** | **INCI** | **amount %** |
| Lutensit ® A-LBN 50 | Benzenesulfonic acid, C10-13-alkyl derivs., sodium salts | 25 |
| Lutensol ® GD 70 | Alkyl polyglucoside | 3 |
| Lutensol ® A 7 N | Fatty alcohol ethoxylate | 2 |
| Perfume oil composition 1B | Fragrance | 0.7 |
| Dyes | Dye | 0.05 |
| Water, de ionized | Aqua | ad 100 |

Hand dish cleaner according to table 35, where the perfume oil composition 1B is replaced by the same amount of the perfume oil composition 2B.
Hand dish cleaner according to table 35, where the perfume oil composition 1B is replaced by the same amount of the perfume oil composition 3.
Hand dish cleaner according to table 35, where the perfume oil composition 1B is replaced by the same amount of the perfume oil composition 4.
Hand dish cleaner according to table 35, where the perfume oil composition 1B is replaced by the same amount of the perfume oil composition 5.
Hand dish cleaner according to table 35, where the perfume oil composition 1B is replaced by the same amount of the perfume oil composition 6.
Hand dish cleaner according to table 35, where the perfume oil composition 1B is replaced by the same amount of the perfume oil composition 7.
Hand dish cleaner according to table 35, where the perfume oil composition 1B is replaced by the same amount of the perfume oil composition 8.
Hand dish cleaner according to table 35, where the perfume oil composition 1B is replaced by the same amount of the perfume oil composition 9.
Hand dish cleaner according to table 35, where the perfume oil composition 1B is replaced by the same amount of the perfume oil composition 10.
Hand dish cleaner according to table 35, where the perfume oil composition 1B is replaced by the same amount of the perfume oil composition 11.
Hand dish cleaner according to table 35, where the perfume oil composition 1B is replaced by the same amount of the perfume oil composition 12.
Hand dish cleaner according to table 35, where the perfume oil composition 1B is replaced by the same amount of the perfume oil composition 13.
Hand dish cleaner according to table 35, where the perfume oil composition 1B is replaced by the same amount of the perfume oil composition 14.

**Table 36: Body lotion**

| Ingredients | | INCI | % | Function |
|---|---|---|---|---|
| I | Eumulgin® SG | Sodium Stearoyl Glutamate | 0.40 | O/W Emulsifier |
| | Cutina® GMS-V | Glyceryl Stearate | 1.50 | Consistency Giving Factor |
| | Cetiol® SB 45 | Butyrospermum Parkii (Shea) Butter | 3.00 | Emollient |
| | Cetiol® C5 | Coco-Caprylate | 2.00 | Emollient |
| | lrwinol™ LS 9890 | Octyldodecanol (and) Irvingia Gabonensis (and) Hydrogenated CocoGlycerides | 1.00 | Moisturizing Active |
| II | Water, demin. | Aqua | 86.77 | |
| | Glycerine | Glycerin | 3.00 | Humectant |
| | EDETA B Powder (BASF) | Tetrasodium EDTA | 0.05 | Complexing Agent |
| III | Rheocare™ C Plus | Carbomer | 0.35 | Thickener |
| IV | NaOH (Aq sol. 25%) | Sodium Hydroxide | 0.10 | Neutralizing Agent |
| V | Covi-ox® T70C | Tocopherol | 0.05 | Antioxidant |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC50 (Schülke) | Ethylhexylglycerin | 0.50 | Preservative |
| | VII NaOH (Aq sol. 25%) | Sodium Hydroxide | 0.18 | Neutralizing Agent |

Body lotion according to table 36, where the perfume oil composition 1B is replaced by the same amount of the perfume oil composition 2B.
Body lotion according to table 36, where the perfume oil composition 1B is replaced by the same amount of the perfume oil composition 3.
Body lotion according to table 36, where the perfume oil composition 1B is replaced by the same amount of the perfume oil composition 4.
Body lotion according to table 36, where the perfume oil composition 1B is replaced by the same amount of the perfume oil composition 5.
Body lotion according to table 36, where the perfume oil composition 1B is replaced by the same amount of the perfume oil composition 6.
Body lotion according to table 36, where the perfume oil composition 1B is replaced by the same amount of the perfume oil composition 7.
Body lotion according to table 36, where the perfume oil composition 1B is replaced by the same amount of the perfume oil composition 8.
Body lotion according to table 36, where the perfume oil composition 1B is replaced by the same amount of the perfume oil composition 9.
Body lotion according to table 36, where the perfume oil composition 1B is replaced by the same amount of the perfume oil composition 10.
Body lotion according to table 36, where the perfume oil composition 1B is replaced by the same amount of the perfume oil composition 11.
Body lotion according to table 36, where the perfume oil composition 1B is replaced by the same amount of the perfume oil composition 12.
Body lotion according to table 36, where the perfume oil composition 1B is replaced by the same amount of the perfume oil composition 13.
Body lotion according to table 36, where the perfume oil composition 1B is replaced by the same amount of the perfume oil composition 14.

**Table 37: Hand dish cleaner, concentrate:**

| **Hand dish cleaner, concentrate** | | |
|---|---|---|
| **Component** | **INCI** | **amount %** |
| Lutensit ® A-LBN 50 | Benzenesulfonic acid, C10-13-alkyl derivs., sodium salts | 40 |
| Lutensit ® AS 2230 | Alkylether sulphate | 25 |
| Lutensol ® GD 70 | Alkyl polyglucoside | 7 |
| Lutensol ® A 7 N | Fatty alcohol ethoxylate | 4 |
| Perfume oil composition 1B | Perfume | 1 |
| Dyes | Dye | 0.05 |
| Water, de ionized | Aqua | ad 100 |

Hand dish cleaner according to table 37, where the perfume oil composition 1B is replaced by the same amount of the perfume oil composition 2B.
Hand dish cleaner according to table 37, where the perfume oil composition 1B is replaced by the same amount of the perfume oil composition 3.
Hand dish cleaner according to table 37, where the perfume oil composition 1B is replaced by the same amount of the perfume oil composition 4.
Hand dish cleaner according to table 37, where the perfume oil composition 1B is replaced by the same amount of the perfume oil composition 5.
Hand dish cleaner according to table 37, where the perfume oil composition 1B is replaced by the same amount of the perfume oil composition 6.
Hand dish cleaner according to table 37, where the perfume oil composition 1 is replaced by the same amount of the perfume oil composition 7.
Hand dish cleaner according to table 37, where the perfume oil composition 1B is replaced by the same amount of the perfume oil composition 8.
Hand dish cleaner according to table 37, where the perfume oil composition 1B is replaced by the same amount of the perfume oil composition 9.
Hand dish cleaner according to table 37, where the perfume oil composition 1B is replaced by the same amount of the perfume oil composition 10.
Hand dish cleaner according to table 37, where the perfume oil composition 1B is replaced by the same amount of the perfume oil composition 11.
Hand dish cleaner according to table 37, where the perfume oil composition 1B is replaced by the same amount of the perfume oil composition 12.
Hand dish cleaner according to table 37, where the perfume oil composition 1B is replaced by the same amount of the perfume oil composition 13.
Hand dish cleaner according to table 37, where the perfume oil composition 1B is replaced by the same amount of the perfume oil composition 14.

**Table 38: Sanitary cleaner, concentrate**

| **Sanitary cleaner, concentrate** | | |
|---|---|---|
| **Component** | **INCI** | **amount %** |
| Lutensol ® TO 8 | isotridecanolethoxylate | 5 |
| Phosphoric acid | Phosphoric acid | 20 |
| Korantin ® PM | 2-propyn-1-ol, ethoxylated | 3 |
| Citric acid | Citric acid | 3 |
| Perfume oil composition 1B | Fragrance | 1 |
| Water, deionized | Aqua | ad 100 |

Sanitary cleaner according to table 38, where the perfume oil composition 1B is replaced by the same amount of the perfume oil composition 2B.
Sanitary cleaner according to table 38, where the perfume oil composition 1B is replaced by the same amount of the perfume oil composition 3.
Sanitary cleaner according to table 38, where the perfume oil composition 1B is replaced by the same amount of the perfume oil composition 4.
Sanitary cleaner according to table 38, where the perfume oil composition 1B is replaced by the same amount of the perfume oil composition 5.
Sanitary cleaner according to table 38, where the perfume oil composition 1B is replaced by the same amount of the perfume oil composition 6.
Sanitary cleaner according to table 38, where the perfume oil composition 1B is replaced by the same amount of the perfume oil composition 7.
Sanitary cleaner according to table 38, where the perfume oil composition 1B is replaced by the same amount of the perfume oil composition 8.
Sanitary cleaner according to table 38, where the perfume oil composition 1B is replaced by the same amount of the perfume oil composition 9.
Sanitary cleaner according to table 38, where the perfume oil composition 1B is replaced by the same amount of the perfume oil composition 10.
Sanitary cleaner according to table 38, where the perfume oil composition 1B is replaced by the same amount of the perfume oil composition 11.
Sanitary cleaner according to table 38, where the perfume oil composition 1B is replaced by the same amount of the perfume oil composition 12.
Sanitary cleaner according to table 38, where the perfume oil composition 1B is replaced by the same amount of the perfume oil composition 13.
Sanitary cleaner according to table 38, where the perfume oil composition 1B is replaced by the same amount of the perfume oil composition 14.

**Table 39: All purpose cleaner**

| **All purpose cleaner** | | |
|---|---|---|
| **Component** | **INCI** | **amount %** |
| Lutensit ® A LBA | Benzenesulfonic acid, 4-C10-13-sec-alkyl derivs | 1 |
| Ammonia | Ammonia | 0,2 |
| Lutensol ® CS 6250 | Hexan-1-ol, ethoxylated | 3 |
| Trilon ® A | trisodium nitrilotriacetate | 1 |
| Citric acid | Citric acid | 0,65 |
| Ethanol | Ethanol | 5 |
| Perfume oil composition 1B | Perfume | 0,5 |
| Water, de ionized | Aqua | ad 100 |

All purpose cleaner according to table 39, where the perfume oil composition 1B is replaced by the same amount of the perfume oil composition 2B.
All purpose cleaner according to table 39, where the perfume oil composition 1B is replaced by the same amount of the perfume oil composition 3.
All purpose cleaner according to table 39, where the perfume oil composition 1B is replaced by the same amount of the perfume oil composition 4.
All purpose cleaner according to table 39, where the perfume oil composition 1B is replaced by the same amount of the perfume oil composition 5.
All purpose cleaner according to table 39, where the perfume oil composition 1B is replaced by the same amount of the perfume oil composition 6.
All purpose cleaner according to table 39, where the perfume oil composition 1B is replaced by the same amount of the perfume oil composition 7.
All purpose cleaner according to table 39, where the perfume oil composition 1B is replaced by the same amount of the perfume oil composition 8.
All purpose cleaner according to table 39, where the perfume oil composition 1B is replaced by the same amount of the perfume oil composition 9.
All purpose cleaner according to table 39, where the perfume oil composition 1B is replaced by the same amount of the perfume oil composition 10.
All purpose cleaner according to table 39, where the perfume oil composition 1B is replaced by the same amount of the perfume oil composition 11.
All purpose cleaner according to table 39, where the perfume oil composition 1B is replaced by the same amount of the perfume oil composition 12.
All purpose cleaner according to table 39, where the perfume oil composition 1B is replaced by the same amount of the perfume oil composition 13.
All purpose cleaner according to table 39, where the perfume oil composition 1B is replaced by the same amount of the perfume oil composition 14.

**Table 40: Anti bacterial fabric softener**

| **Anti-bacterial fabric softener** | | |
|---|---|---|
| **Component** | **INCI** | **amount %** |
| Tinosan ® HP 100 | Hydroxydichlordiphenyl ether | 0.3 |
| Dehyquat AU-46 | Esterquat | 4 |
| Lutensol ® AO 7 | Alcohols, C13-15, ethoxylated | 0.5 |
| Perfume oil composition 1B | Fragrance | 0.5 |
| Water, deionized | Aqua | ad 100 |

Anti bacterial fabric softener according to table 40, where the perfume oil composition 1 B is replaced by the same amount of the perfume oil composition 2B.
Anti bacterial fabric softener according to table 40, where the perfume oil composition 1 B is replaced by the same amount of the perfume oil composition 3.
Anti bacterial fabric softener according to table 40, where the perfume oil composition 1 B is replaced by the same amount of the perfume oil composition 4.
Anti bacterial fabric softener according to table 40, where the perfume oil composition 1 B is replaced by the same amount of the perfume oil composition 5.
Anti bacterial fabric softener according to table 40, where the perfume oil composition 1B is replaced by the same amount of the perfume oil composition 6.
Anti bacterial fabric softener according to table 40, where the perfume oil composition 1B is replaced by the same amount of the perfume oil composition 7.
Anti bacterial fabric softener according to table 40, where the perfume oil composition 1B is replaced by the same amount of the perfume oil composition 8.
Anti bacterial fabric softener according to table 40, where the perfume oil composition 1B is replaced by the same amount of the perfume oil composition 9.
Anti bacterial fabric softener according to table 40, where the perfume oil composition 1B is replaced by the same amount of the perfume oil composition 10.
Anti bacterial fabric softener according to table 40, where the perfume oil composition 1B is replaced by the same amount of the perfume oil composition 11.
Anti bacterial fabric softener according to table 40, where the perfume oil composition 1B is replaced by the same amount of the perfume oil composition 12.
Anti bacterial fabric softener according to table 40, where the perfume oil composition 1B is replaced by the same amount of the perfume oil composition 13.
Anti bacterial fabric softener according to table 40, where the perfume oil composition 1B is replaced by the same amount of the perfume oil composition 14.

## Claims

1. A use of a compound of formula (I) 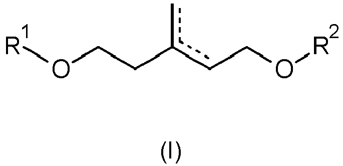 wherein
one of the symbols -̅ -̅ is a single bond and the other is a double bond or a single bond,
R¹ is hydrogen, C₁-C₄-alkyl or -(C=O)-R³,
R² is hydrogen, C₁-C₄-alkyl or -(C=O)-R⁴,
R³ is hydrogen or C₁-C₄-alkyl,
R⁴ is hydrogen or C₁-C₄-alkyl,
a stereoisomer thereof or a mixture of stereoisomers thereof,
as an aroma chemical,
with the proviso that if R¹ is hydrogen, R² is different from hydrogen.

2. The use of a compound according to claim 1, where in formula (I)
R¹ is C₁-C₄-alkyl,
R² is hydrogen or C₁-C₄-alkyl.

3. The use of a compound according to claim 1 or 2, where the compound of formula (I) has at least one of the following features a), b) and/or c)
a) R¹ and R² are identical,
b) R¹ and R² are C₁-C₃-alkyl,
c) R¹ and R² are methyl or ethyl, preferably methyl.

4. The use of a compound according to claim 1, where in formula (I)
R¹ is -(C=O)-R³,
R² is hydrogen, -(C=O)-R⁴ or C₁-C₄-alkyl.

5. The use according to claim 4, where the compound of formula (I) has at least one of the following features d), e), f) and/or g)
d) R² is -(C=O)-R⁴,
e) R³ and R⁴ are identical,
f) R³ and R⁴ are hydrogen or C₁-C₄-alkyl,
g) R³ and R⁴ are hydrogen or C₁-C₃-alkyl, preferably hydrogen, methyl or ethyl.

6. The use according to any of the preceding claims, in a composition selected from perfume compositions, cosmetic compositions, body care compositions, products for oral and dental hygiene, hygiene articles, cleaning compositions, textile detergent compositions, dishwashing compositions, scent dispensers, foods, food supplements, pharmaceutical compositions and crop protection compositions.

7. Aroma chemical composition comprising at least one compound of formula (I), a stereoisomer thereof or a mixture of stereoisomers thereof, according to any of claims 1 to 5, and at least one further compound selected from the group consisting of further aroma chemicals different from compounds (I) and non-aroma chemical carriers.

8. The composition according to claim 7, selected from perfume compositions, cosmetic compositions, body care compositions, products for oral and dental hygiene, hygiene articles, cleaning compositions, textile detergent compositions, dishwashing compositions, scent dispensers, foods, food supplements, pharmaceutical compositions and crop protection compositions.

9. A method of preparing a fragranced ready-to-use composition, comprising incorporating at least one compound of formula (I), a stereoisomer thereof or a mixture of stereoisomers thereof, according to any of claims 1 to 5, into a ready-to-use composition.

10. The use of a compound of formula (I), a stereoisomer thereof or a mixture of stereoisomers thereof for modifying the scent character of a fragranced ready-to-use composition.

11. A compound of the general formula (I) 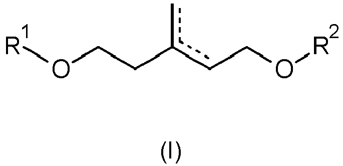 wherein
one of the symbols -̅ -̅ is a single bond and the other is a double bond or a single bond,
R¹ is hydrogen, C₁-C₄-alkyl or -(C=O)-R³,
R² is hydrogen, C₁-C₄-alkyl or -(C=O)-R⁴,
R³ is hydrogen or C₁-C₄-alkyl,
R⁵ is hydrogen or C₁-C₄-alkyl,
a stereoisomer thereof or a mixture of stereoisomers thereof,
with the proviso that if R¹ is hydrogen R² is different from hydrogen,
except for the following compounds
- (5-acetoxy-3-methylene-pentyl) acetate,
- [(E)-5-acetoxy-3-methyl-pent-3-enyl] acetate,
- [(Z)-5-acetoxy-3-methyl-pent-3-enyl] acetate,
- (5-acetoxy-3-methyl-pentyl) acetate,
- [(E)-5-formyloxy-3-methyl-pent-3-enyl] formate,
- [(Z)-5-formyloxy-3-methyl-pent-3-enyl] formate,
- (3-methyl-5-propanoyloxy-pentyl) propanoate,
- [5-(2,2-dimethylpropanoyloxy)-3-methyl-pentyl] 2,2-dimethylpropanoate,
- 1,5-dimethoxy-3-methylene-pentane,
- (E)-1,5-dimethoxy-3-methyl-pent-2-ene,
- (Z)-1,5-dimethoxy-3-methyl-pent-2-ene,
- 1,5-dimethoxy-3-methyl-pentane,
- (Z)-1,5-diethoxy-3-methyl-pent-2-ene,
- 1,5-diethoxy-3-methyl-pentane,
- (E)-1,5-diisopropoxy-3-methyl-pent-2-ene,
- (Z)-1,5-diisopropoxy-3-methyl-pent-2-ene,
- 1,5-dibutoxy-3-methylene-pentane,
- (E)-1,5-dibutoxy-3-methyl-pent-2-ene,
- (Z)-1,5-dibutoxy-3-methyl-pent-2-ene
- mixture of [(E)-5-acetoxy-3-methyl-pent-3-enyl] acetate and [(Z)-5-acetoxy-3-methyl-pent-3-enyl] acetate,
- mixture of [(E)-3-methyl-5-propanoyloxy-pent-3-enyl] propanoate and [(Z)-3-methyl-5-propanoyloxy-pent-3-enyl] propanoate,
- mixture of (E)-1,5-dimethoxy-3-methyl-pent-2-ene and (Z)-1,5-dimethoxy-3-methyl-pent-2-ene,
- mixture of (Z)-1,5-diethoxy-3-methyl-pent-2-ene and (E)-1,5-diethoxy-3-methyl-pent-2-ene,
- mixture of (E)-3-methyl-1,5-dipropoxy-pent-2-ene and (Z)-3-methyl-1,5-dipropoxy-pent-2-ene and
- mixture of (E)-1,5-dibutoxy-3-methyl-pent-2-ene and (Z)-1,5-dibutoxy-3-methyl-pent-2-en.

12. A composition comprising a mixture of at least two compounds selected from the compounds of formulae (1.1), (1.2) and (1.3), 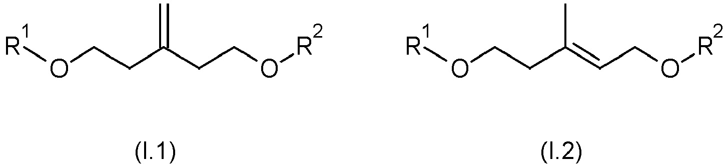 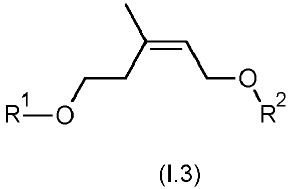 wherein R¹ and R² has one of the meanings as defined in claims 1 to 5, comprising each of the compounds in an amount of 1 - 99 % by weight, with the proviso that weight of the compounds of formulae (1.1), (1.2) and (1.3) in said mixture adds up to 100%.

13. The composition according to claim 12, wherein the ratio of (I.1):(I.2):(I.3) is in the range of 90:5:5 to 10:45:45, preferably 80:10:10 to 20:40:40.

14. The composition according to any of claims 12 or 13, wherein
R¹ and R² are C₁-C₄-alkyl, or
R¹ is -(C=O)-R³, R² -(C=O)-R⁴, R³ and R⁴ are C₁-C₄-alkyl.

15. The use of a composition according to any of claims 12 to 14 as aroma chemical.
